# EUROPEAN PATENT SPECIFICATION

(11) **EP 0 359 454 B1**
(45) Date of publication and mention of the grant of the patent: **27.12.2000**
(21) Application number: 89308920.1
(22) Date of filing: 04.09.1989
(51) Int. Cl.: C07D 519/00, A61K 31/40, D06L 3/12, C08K 5/3417

(54) **CC-1065 analogs having two CPI subunits**
CC-1065-Analoge mit zwei CPI-Subeinheiten
Analogues du CC-1065 avec deux sous-unités CPI

(30) Priority: 12.09.1988 US 243350; 07.08.1989 WO PCT/US89/03329
(43) Date of publication of application: 21.03.1990
(73) Proprietor: PHARMACIA & UPJOHN COMPANY, Kalamazoo, Michigan 49001 (US)
(72) Inventor: Kelly, Robert C., Augusta Michigan (US); Aristoff, Paul A., Portage Michigan (US)
(74) Representative: Perry, Robert Edward

(56) References cited:
- EP-A- 0 154 445
- WO-A-88/45659
- US-A- 4 301 248
- US-A- 4 912 227
- Cancer Research, 1979; vol. 39; 4816-4822
- Investigational New Drugs 1991; vol. 9; 137-148; Kluwer Academic Publishers
- Tetrahedron Lett. 1986, vol. 27, 4103-4106
- J. Med. Chem. 1988, vol. 31, 590-603

## Description

### BACKGROUND OF THE INVENTION

Antibiotic CC-1065, (7bR,8aS)-7-[[1,6-dihydro-4-hydroxy-5-methoxy-7-[(4,5,8,8a-tetrahydro-7-methyl-4-oxocyclopropa[c]pyrrolo[3,2-e]indol-2(1H)carbonyl]benzo[1,2-b:4,3-b′]dipyrrol-3(2H)-yl]carbonyl]1,6-dihydro-4-hydroxy-5-methoxy-benzo[1,2-b:4,3-b′]dipyrole-3(2H)-carboxamide, is disclosed and claimed in L.J. Hanka et al. U.S. Patent No. 4,169,888 together with a process for preparing antibiotic CC-1065 by aerobic fermentation procedures, and recovering antibiotic CC-1065 therefrom.

In The Journal of Antibiotics, 1985, 38, 746, D.G. Martin et al reported that acetic acid adds across the spirocyclopropylcyclohexadienyl (SCPCH) system of CC-1065 to produce the phenolic, acetic acid product (AAP), 7-[[7-[[1-[(acetyloxy)methyl]-1,6-dihydro-5-hydroxy-8-methylbenzo[1,2-b:4,3-b′]dipyrrol-3(2H)-yl]carbonyl]-1,6-dihydro-4-hydroxy-5-methoxybenzo[1,2:4,3-b′]-dipyrrol-3(2H)-yl]carbonyl]-1,6-dihydro-4-hydroxy-5-methoxy-benzo[1,2-b:4,3-b′]dipyrrole-3(2H)-carboxamide. AAP was tested in vitro and in vivo and found to be less potent than CC-1065 by a factor of 10³ to 10⁴ depending upon the particular test system and therefore tended to divert attention from adducts of the SCPCH system as useful antitumor agents or as prodrugs to CC-1065 analogs.

In J. Am. Chem. Soc., 103, No. 18, 1981, W. Wierenga published a "Synthesis of the Left-Hand Segment of the Antitumor Agent CC-1065".

EP Application 0 154 445 (published 11.09.85) discloses various analogs of antibiotic CC-1065, including compounds of formula EP-I and EP-II (see General Formula chart of EP 0154 445), wherein R₁ in formula EP-II is CH₃-, -CH₂Ph, CH=CHCH₂-, -CH₂SCH₃, -CH₂OCH₃, -CH₂OCH₂CH₂OCH₃, -CH₂CCl₃, -CH₂CH₂Si(R₂)₃, or H, where Ph is phenyl; R is alkyl(C₁-C₅) phenyl, or H; R₂′ is C₁ to C₅-alkyl, phenyl or hydrogen, and is not necessarily the same as R in one compound; R₃ is alkyl(C₁-C₅), phenyl, or H; and X is Cl, Br, I or OSO₂R₄₀, where R₄₀ is C₁ to C₅-alkyl, phenyl, tolyl, bromophenyl, nitrophenyl, or trifluromethyl. The O-protected compounds of the formula EP-II are chemically stable and only removable under specific chemical conditions. However, when the compounds of formula EP-II are 0-deprotected, they can be cyclized to yield the compounds of EP-I.

EP Application 0 154 445 also discloses CPI dimers joined by -CO-(CH₂)ₙ₁-CO- where n₁ is 2-12 and CPI dimers joined by the tether -C(O)-(-R₁₁-)-C(O)-X₇-(-CH₂CH₂-X₇)n4-C-(O)-(-R₁₁)-C(O)- where R₁₁ is CH₂CH₂ or CH=CH, X₇ is O or NH, and n4 is 1-4, and the HCl and MeI salts when X₇ is NH. Further such dimers are disclosed in WO-A-8804659.

### SUMMARY OF THE INVENTION

Novel compounds according to this invention are useful as UV light absorbers and as chemical intermediates. Representative compounds of the invention have also been shown to possess anti-tumour activity. These compounds are of formula I and Compound 16 (see the General Formula Chart):
wherein CPI₁ and CPI₂ are independently selected from formulae A and B (see General Formula Chart);
W and Z are independently selected from hydrogen, C₁-C₅ alkyl and phenyl;
X is azido, a halogen atom, cyanate, thiocyanate, isocyanate, thioisocyanate, -PO(OR)₂, -O-PO₂R, -O-PSOR, -O-SOR or -O-SO₂R; and
Y is hydrogen, -C(O)R, -C(S)R, -C(O)OR₁, -S(O)₂R₁, -C(O)NR₂R₃, -C(S)NR₂R₃, or -C(O)NHSO₂R₃;
wherein R is C₁-C₂₀ alkyl; C₂-C₆ alkenyl; C₂-C₆ alkynyl; phenyl optionally substituted with one, 2 or 3 substituents selected from C₁-C₄ alkyl, C₁-C₃ alkoxy, halo, C₁-C₃ alkylthio, CF₃, C₂-C₆ dialkylamino and nitro; or naphthyl optionally substituted with one or 2 substituents as defined above;
R₁ is C₁-C₂₀ alkyl or optionally substituted phenyl as defined above;
R₂ is hydrogen or C₁-C₂₀ alkyl;
R₃ is hydrogen, C₁-C₂₀ or optionally-substituted phenyl as defined above; and
R₄ is C₁-C₁₀ alkyl, optionally-substituted phenyl as defined above or optionally-substituted naphthyl as defined above;
R₅ and R'₅ are independently selected from a direct bond and a divalent group of any of the formulae shown in Chart C,
wherein X₁ is H, CH₃, OH, OCH₃, NO₂, NH, NHNHC(O)CH3, NHC(O)C₆H₅, or halogen; X₃ is H, OH or OCH₃; R₈ is H, CH₃ or C₂H₅; R' is H or CH₃S-; X₈ is -O-, -S- or NH; X₉ and X₁₀ are each -CH= or -N=; and Y₁ is H, halo, C₁-C₄ alkyl, C₁-C₃ alkoxy, C₂-C₆ dialkylamino, nitro, aminocarbonylalkyl(C₁-C₁₀), hydroxy, -NH₂, -NHCONH₂, -NHCOCH₃ or -NHC(O)-C₆H₅; and
wherein T is a tether linkage selected from:
   a) -NHC(O)-;
   b) -C(O)NH-;
   c) -C(O)O-;
   d) -OC(O)-;
   e) -NR₁₃-T'-NR₁₄- where either R₁₃ and R₁₄ are independently hydrogen or C₁-C₈ alkyl, or R₁₃ and R₁₄ are taken together and are -(CH₂)ₙ- where n is 2 or 3; and T' is -CO-, -COCO-, -CO(CH₂)₁₋₅CO-, -C(O)PhC(O)-where Ph is 1,3- or 1,4-phenylene, or -C(O)-het-C(O)-; and
   f) -C(O)-het-C(O)-, when R₅ and R'₅ are both a direct bond;
wherein -het- is a fused mono-, di- or tricyclic heteroaryl of 5 to 12 members, containing one, 2 or 3 heteroatoms selected from O, N and S, optionally-substituted with one or 2 substituents as defined above.

### DESCRIPTION OF THE INVENTION

Preferably, CPI₁ and CPI₂ are the same or different and are each either 1-(chloromethyl)-1,6-dihydro-8-methyl-5-hydroxybenzo[1,2:4,3-6']dipyrrol-3(2H)-yl or 4,5,8,8a-tetrahydro-7-methyl-4-oxocyclopropa[c]pyrrolo(3,2-e)indol-2(1H)-yl.

W is preferably methyl.

X is preferably halogen, more preferably chloro or bromo.

Y is preferably -COR, wherein R is selected from C₁-C₁₀ alkyl; phenyl optionally substituted with one, 2 or 3 C₁-C₄ alkyl, C₁-C₃ alkoxy, halo, C₁-C₃ alkylthio, trifluoromethyl, C₂-C₆ dialkylamino, or nitro; -C(O)NHSO₂R₄; and -C(O)NR₂R₃. Most preferably, Y is H or (C₁-C₁₀ alkyl)carbonyl.

Z is preferably hydrogen.

T is preferably -NH-CO-NH- or -NH-CO-het-CO-NH- and -het- is selected from pyrrole-2,5-diyl, furan-2,5-diyl, indole-2,5-diyl, benzofuran-2,5-diyl and 3,6-dihydrobenzo[1,2-b:4,3-b']dipyrrole-2,7-diyl.

R₅ and R'₅ are preferably selected from 2-carbonylindol-5-yl, 2-carbonyl-6-hydroxy-7-methoxyindol-5-yl, 2-carbonyl-1,2,3,6-tetrahydrobenzo[1,2-b:4,3-b']dipyrrol-7-yl and 2-carbonyl-4-hydroxy-5-methoxy-1,2,3,6-tetrahydrobenzo[1,2-b:4,3-b']dipyrrol-7-yl.

Halogen atom (halo) refers to a bromo, chloro, iodo or fluoro atom.

Examples of C₁-C₂₀ alkyl are methyl, ethyl, propyl, butyl and the like, including isomeric forms thereof. Examples of C₁-C₃ alkoxy are methoxy, ethoxy, propoxy and isomeric forms thereof. Examples of C₂-C₆ dialkylamino are dimethylamino, diethylamino, methylethylamino, dipropylamino and ethylpropylamino. Examples of aminocarbonylalkyl-(C₁-C₁₀) are aminocarbonylpentyl (-NHCOC₅H₁₁) and aminocarbonylmethyl (-NHCOCH₃).

Examples of het include furan-2,4-diyl, furan-2,5-diyl, pyrrol-2,4-diyl, pyrrol-2,5-diyl, thiophen-2,4-diyl, thiophen-2,5-diyl, indol-2,5-diyl, benzofuran-2,5-diyl, benzothiophen-2,5-diyl, pyridin-2,6-diyl, pyrazin-2,6-diyl, pyrimidin-2,6-diyl, quinolin-2,6-diyl, quinoxalin-2,6-diyl, quinazolin-2,6-diyl, benzo[1,2-b:4,3-b′]dipyrrol-2,7-diyl, benzo[1,2-b:4,3-b′[difuran-2,7-diyl, benzoxazol-2,5-diyl.

Examples of optionally substituted het are 3,4-dichlorofuran-2,5-diyl 3,4-dimethylfuran-2,5-diyl, 3-chloropyrrol-2,5-diyl, 3,4-dichloropyrrol-2,5-diyl, 3-methoxypyrol-2,5-diyl, 3-methyl-4-ethylpyrrol-2,5-diyl, 3,4-difluoropyrrol-2,5-diyl, 3-bromo-thiophen-2,5-diyl, 4-chloropyridin-2,6-diyl, 4,5-dimethoxybenzo[1,2-b:4,3-b′]dipyrrol-2,7-diyl, 3,5-dichloropyridin-2,6-diyl.

The compounds of formula B on the GENERAL FORMULA sheet can be named as derivatives of the numbering system (B′) shown on the GENERAL FORMULA sheet. Such compounds will contain the 1,2,3,6-tetrahydro-8-W-5-Y-benzo[1,2-b:4,3-b′]dipyrrol-1-[Z-CH(X)]-structure.

The compounds of Formula I are drawn as the racemic mixture and include the natural isomer of Formula I′a which can be resolved from the racemic mixture and/or prepared from starting materials of the natural, i.e. 1(S)-configuration. Examples of Formula I compounds of this invention include:
[S-(R*,R*)]-6,6′-[carbonylbis(imino-1H-indole-5,2-dicarbonyl)]bis[8-chloromethyl)-3,6,7,8-tetrahydro-1-methylbenzo[1,2-b:4,3-b′]dipyrrol-4-ol (Compound 1);
[7bR-[2(7′bR*,8′aS*), 7bR*,8aS*]]-2,2′-[carbonylbis(imino-1H-indole-5,2-dicarbonyl)]bis[1,2,8,8a-tetrahydro-7-methyl-cyclopropa(c)pyrrolo[3,2-e]indol-4(5H)-one (Compound 2);
(R*,S*)-N,N′-bis[2-[[1-(chloromethyl)-1,6-dihydro-5-hydroxy-8-methylbenzo[1,2-b:4,3-b′]dipyrrol-3(2H)-yl]carbonyl]-1H-indol-5-yl]-ethanediamide (Compound 3);
[S-(R*,R*)]-6,6′-(1H-pyrrole-2,5-diyldicarbonyl)bis[8-(chloromethyl)-3,6,7,8-tertrahydro-1-methyl-benzo[1,2-b:4,3-b′-]dipyrrol-4-ol (Compound 4);
[S-(R*,R*)]-6,6′-(2,5-furandiyldicarbonyl)bis[8-(chloromethyl)-3,6,7,8-tertrahydro-1-methyl-benzo[1,2-b:4,3′b′]dipyrrol-4-ol (Compound 5);
[S-(R*,R*]-N,N′-bis[2-[[1-(chloromethyl)-1,6-dihydro-5-hydroxy-8-methylbenzo[1,2-b:4,3-b′]dipyrrol-3(2H)-yl]carbonyl]-1H-indol-5-yl]-2,5-furandicarboxamide (Compound 6);
[S-(R*,R*)]-N,N′-bis[2[[1-(chloromethyl)-1,6-dihydro-5-hydroxy-8-methylbenzo[1,2-b:4,3-b′]dipyrrol-3(2H)-yl]carbonyl]-1H-indol-5-yl]-1H-pyrrole-2,5-dicarboxamide (Compound 7);
[R-(R*,S*)]-N,N′-bis[2[[1-(chloromethyl)-1,6-dihydro-5-hydroxy-8-methylbenzo[1,2-b:4,3-b′]dipyrrol-3(2H)-yl]carbonyl]-1H-indol-5-yl]-propanediamide (Compound 8);
[S-(R*,R*)]-6,6′-[carbonylbis(imino-1H-indole-5,2-diylcarbonyl)bis[8-(chloromethyl)-3,6,7,8-tetrahydro-1-methyl-benzo[1,2-b:4,3-b′]dipyrrol-4-ol diacetate (Compound 9);
[S-(R*,R*)]-6,6′-(1H-indole-2,5-diyldicarbonyl)bis[8-(chloromethyl)-3,6,7,8-tetrahydro-1-methyl-benzo[1,2-b:4,3-b′]dipyrrol-4-ol (Compound 10);
[S-(R*,R*)]-N,N′-bis[2-[[1-(chloromethyl)-1,6-dihydro-5-hydroxy-8-methylbenzo[1,2-b:4,3-b′]dipyrrol-3(2H)-yl]carbonyl]-1H-indol-5-yl]-1H-indole-2,5-dicarboxamide (Compound 11);
[S-(R*,R*)]-2-[[1-(chloromethyl)-1,6-dihydro-5-hydroxy-8-methylbenzo[1,2-b:4,3-b′]dipyrrol-3(2H)-yl]carbonyl]-N-[3-[[1-(chloromethyl)-1,6-dihydro-5-hydroxy-8-methylbenzo[1,2-b:4,3-b′]dipyrrol-3(2H)-yl]carbonyl]-1H-indol-6-yl]-1H-indole-5-carboxamide (Compound 12);
[S-(R*,R*)]-5-[[1-(chloromethyl)-1,6-dihydro-5-hydroxy-8-methylbenzo[1,2-b:4,3-b′]dipyrrol-3(2H)-yl]carbonyl)-N-[2-[[1-(chloromethyl)-1,6-dihydro-5-hydroxy-8-methylbenzo[1,2-b:4,3-b′]dipyrrol-3(2H)-yl]carbonyl)-1H-indol-5-yl]-1H-indole-5-carboxamide (Compound 13);
[S-(R*,R*)]-carbonylbis[imino-1H-indole-5,2-diylcarbonyl[1-(chloromethyl)-1,6-dihydro-8-methylbenzo[1,2-b:4,3-b′]dipyrrole-3,5(2H)-diyl]]ester, 2,2-dimethylpropanoic acid (Compound 14);
[S-(R*,R*)]-carbonylbis[imino-1H-indole-5,2-diylcarbonyl[1-(chloromethyl)-1,6-dihydro-8-methylbenzo[1,2-b:4,3-b′]dipyrrole-3,5(2H)-diyl]]ester, decanoic acid (Compound 15);
[S-(R*,R*)]-6,6′-[carbonylbis[(7,8-dihydrobenzo[1,2-b:4,3-b′]dipyrrole-6,2(3H)-diyl)carbonyl]]bis[8-chloromethyl)-3,6,7,8- tetrahydro-1-methyl-benzo[1,2-b:4,3b′]dipyrrol-4-ol (Compound 16).

The compounds of Formula I are readily prepared by reacting the appropriate 4,5,8,8a-tetrahydro-4-oxocyclopropan(c)pyrrolo(3,2-e)-indole analog (Formula B) with the biscarboxylic acid, bisisocyanate, bis-amino carboxylate or oxycarboxylate reagent and then with the other appropriate 4,5,8,8a-tetrahydro-4-oxocyclopropan(c)pyrrolo(3,2-e)indole analog (Formula B) as illustrated in Scheme 1, 2, 2a, 3, 4, 5 and 6 using the following reaction conditions:
Steps 1 and 4: Run in solution of 1 mg to 100 mg/ml in solvents such as ethyl acetate, methylene chloride or 1,2-dichloroethane with a gaseous acid at 1 to 5 M such as HCl, or HBr or a strong organic acid such as CF₃CO₃H. The reaction run at 0° to 50°C. The product as an amine salt isolated by evaporation of solvent and acid. The reaction may be run from 10 min to 48 hr.
Step 2: Reaction run in polar solvent such as DMF, DMA, or THF at -10° to 50°C. Ratio of CPI moiety: biscarboxylic acid:dehydrating agent preferably 2:1:2. Dehydrating or coupling reagent can be a carbodiimide such as dicyclohexylcarbodiimide (DCC) or ethyldimethylaminopropylcarbodiimide (EDC) or other amide dehydrating agents known in the art. The reaction may be run from 10 min to 72 hrs.
Steps 5 and 7: Same as Step 2 but ratio of CPI moiety:acid:dehydrating reagent is preferably 1:1:1.
Steps 3, 8, 9, 11, 14, 15 and 17: The reaction is run in aqueous organic base. The preferred conditions are acetonitrile:-water: triethylamine in a ratio of 1:1:1. The ratio of these reagents may change to 1:2:2 to 5:1:1. Also the solvents may change so that in place of acetonitrile may be used DMF, DMA, N-methylpyrrolidone, THF, etc. Also the organic base can be varied to other tertiary organic amines such as 1,5 diazo-bicyclo[3.3.3)octane (DABCO), or ethyl diisopropylamine. The temperature is usually 0°C to 50°C and the reaction is usually run for 5 min to 10 hr.
Step 6: The deprotection depends on the protecting group R. If it is t-butyl then the group may be removed by acid treatment similar to Step 1. If R = benzyl it may be removed by hydrogenolysis with Pd catalysis in ethyl acetate or THF, for example, or by ammonium formate, methanol, water and Pd/C. If R = SiR₃ then it may be removed by acid or fluoride ions. These methods are all well known in the art.
Steps 5a and 7a: The reactions may be run in aprotic polar solvents. Preferable are pyridine, DMF, DMA, THF, or methylene chloride. When the solvent is other than pyridine a base such as triethylamine may be added. The reaction is run in a ratio of CPI moiety to activated carbonyl compound of about 1:1. The reaction may be run at -50° to 100°C and for from 5 min to 72 hr.
Steps 10 and 16: The reagents are combined in a ratio of CPI moiety to active carbonyl of about 2:1. The reaction may be run in polar aprotic solvents such as THF, DMF, DMA, pyridine, acetonitrile, etc. The reaction may be run at -50°C to 100°C and for 10 min to several weeks.
Steps 12, 13, 18 and 19: Ratio of CPI moiety to active carbonyl moiety of about 1:1. Otherwise run as Step 10.

In the following examples NMR spectral data were recorded in DMSO-d6 unless otherwise noted. IR spectra were recorded from Nujol mulls. Solution for UV spectra were prepared by dissolving the solid in a few drops of DMF or dimethylacetamide (DMA) and diluting with 95% ethanol or methanol unless stated otherwise. TLC data is given for Analtech Uniplates of Silica gel GF.

### Example 1 Preparation of [S-(R*,R*)]-6,6′-[carbonylbis(imino-1H-indole-5,2-dicarbonyl)]bis[8-chloromethyl)-3,6,7,8-tetrahydro-1-methyl-benzo[1,2-b:4,3-b′]dipyrrol-4-ol (Compound 1).

### Part A - Preparation of 5,5′-(carbonyldiimino)bis-1H-indole-2-carboxylic acid diethyl ester.

A solution of 5-aminoindole-2-carboxylic acid ethyl ester (6.82g, 33.4 mM) and 4-dimethylaminopyridine (19 mg, 0.15 mM) dissolved in THF (80 mL) are cooled to -98°C in a frozen methanol bath. To this is added diisopropylethylamine (7 mL, 40.2 mM) followed by 1.93 M phosgene in toluene (8.5 mL, 16.4 mM). The reaction is stirred at -98°C for 1 hour, then at -78°C for 1 hour. It is then stored at -13°C overnight. The reaction is then quenched with water, and diluted with water and ethyl acetate. The aqueous layer is separated and re-extracted twice with ethyl acetate. The combined ethyl acetate layers are washed with N HCl, giving a mixture which is separated by filtration. The filtered solid is found to be pure product 5,5′-(carbonyldiimino)bis-1H-indole-2-carboxylic acid diethyl ester (6.38 g, 88%). The filtrate is dried and concentrated leaving 1.49 g of dark solid. This material is chromatographed over silica gel (100 g) eluted with 10% DMF in toluene. Twenty ml fractions are collected. Product is found by TLC in fractions 23-50. Concentration of these fractions under high vacuum leaves a residue which is crystallized from DMF-methanol. There is thus obtained an additional 0.80 g of product.
NMR: δ 1.36(t, 6H); 4.35(q, 4H); 7.10(s, 2H); 7.26(d, 2H), J=9Hz); 7.39(d, 2H, J=9Hz); 7.88(s, 2H); 8.52(s, 2H); 11.77(bs, 2H).
IR: 3330, 3260, 1705, 1695, 1545, 1250 cm⁻¹
UV: ^{λ}max (THF)=338 nm (ε=7000); 301 nm (ε=28000); 292 nm (ε-26000); 250 nm (ε=31000).
MS(FAB) m/z 435(M+H), 406, 362, 253, 204, 159, 132.
TLC: R_{f}=0.30 in (10-90) DMF-toluene.

### Part B - Preparation of 5,5′-(carbonyldiimino)bis-1H-indole-2-carboxylic acid.

The indole dimer diester, 5,5′-(carbonyldiimino)bis-1H-indole-2-carboxylic acid diethyl ester, (7.16 g, 16.5 mM) is treated with pyridine (300 ml) and N NaOH (60 mL). The resultant mixture is stirred 24 hrs at 25°C, 7 hrs at 50-60°C, and additional 48 hrs at 25°C at which point TLC indicates the reaction to be done. The reaction is concentrated at 25°C under high vacuum to near dryness. The residue is acidified with 3 N HCl and the resulting solid collected by filtration. The filtered solid is dissolved in DMF (100 ml) at about 100°C. The solution is then diluted with methanol (400 ml) and water (10 ml). The solution is cooled and the crystalline product 5,5′-(carbonyldiimino)bis-1H-indole-2-carboxylic acid is collected by filtration (5.11 g, 82%).
NMR: δ 7.07(d, 2H, J=2Hz); 7.28(dd, 2H, J=1.8Hz, J=9Hz); 7.40(d, 2H, J=9Hz); 7.89(d, 2H, J=1.8Hz); 8.59(bs, 2HO); 11.65(d, 2H, J=1.5Hz).
IR: 3400-2300, 1680, 1530, 1225 cm⁻¹.
UV: ^{λ}max=294 nm (ε=23000).
MS(FAB): m/z 379(M+H), 362, 331, 253, 176, 158, 132.
TLC: R_{f}=0.33 in (40-60-2) DMF-toluene-acetic acid.

### Part C - Reaction of (S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] with hydrogen chloride followed by condensation with 5,5′-(carbonyldiimino)bis-1H-indole-2-carboxylic acid.

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (128 mg, 0.38 mM) is dissolved in ethyl acetate (6 ml) and the solution treated with a freshly prepared solution of saturated CHl in ethyl acetate (6 ml). The reaction is stirred one hr at which time TLC shows the complete disappearance of starting material. The reaction solution is concentrated under vacuum and returned to atmospheric pressure under nitrogen. The resulting residue is twice re-evaporated from methylene chloride to give (S)-1-(chloromethyl)-5-hydroxy-8-methyl-1,2,3,6-tetrahydro-benzo[1,2-b:4,3-b′]dipyrrole hydrochloride (CPI phenol chloride hydrochloride salt). This material is treated with a solution of the diacid, 5,5′-(carbonyldiimino)bis-1H-indole-2-carboxylic acid, (78 mg, 0.21 mM) in of dry dimethylacetamide (4 ml). 1-Ethyl-3-(3-dimethylaminopropyl) carbodiimide hydrochloride (EDC, 68 mg, 0.08 mM) is then added as a solid. After 45 min, a second quantity of EDC is added (68 mg). Stirring is continued 50 min. The reaction is precipitated with water and the solid centrifuged. It is washed with 0.1 NHCl, 0.5% NaHCO₃ and water, centrifuging and withdrawing the aqueous each time. The residue is evaporated in vacuo at less than 20 torr over two days. The crude product is adsorbed from DMF onto Celite and chromatographed on a silica gel column (15 g) eluting with 30% DMF in toluene. Fractions of 10 ml are collected. [S-(R*,R*)]-6,6′-[carbonylbis(imino-1H-indole-5,2-dicarbonyl)]bis[8-chloromethyl)-3,6,7,8-tetrahydro-1-methyl-benzo[1,2-b:4,3-b′]dipyrrol-4-ol is found in fractions 4-16 (115.1 mg of solid, 74% yield based on BocCPI). NMR, IR, UV, FAB.
NMR: δ 2.356(S, 6H); 3.582(m, 2H); 3.92(m, 2H); 4.03 (m, 2H); 4.548(m, 2H); 4.67(m, 2H); 7.044(s, 4H); 7.26(d, 2H); 7.42(d, 2H); 7.64(bs, 2H); 7.849(s, 2H); 8.466(bs, 2H); 9.767(bs, 2H); 10.704(bs, 2H); 11.531(bs, 2H).
IR: 3260, 1580, 1195, cm⁻¹.
UV: ^{λ}max=349 nm(ε=25000); 295 nm(ε=31000).
MS(FAB): m/z 815(M+H), 779, 579, 236, 201, 187, 159.
DNA BINDING: 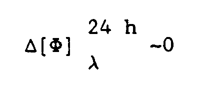
TLC: R_{f}=0.44 in (30-70)DMF-toluene.

### Example 2 Preparation of [7bR-[2(7′bR*,8′aS*),7bR*,8aS*]]-2,2′-[carbonylbis(imino-1H-indole-5,2-dicarbonyl)]bis[1,2,8,8a-tetrahydro-7-methyl-cyclopropa(c)pyrrolo[3,2-e]indol-4(5H)-one (Compound 2).

[S-(R*,R*)]-6,6′-[carbonylbis(imino-1H-indole-5,2-dicarbonyl)]bis[8-chloromethyl)-3,6,7,8-tetrahydro-1-methyl-benzo[1,2-b:4,3-b′]dipyrrol-4-ol (80 mg, 0.1 mM) is treated with acetonitrile (10mL), water (3 mL) and triethylamine (3 mL). The reaction is stirred under nitrogen at room temperature. After 40 minutes the product which has precipitated is collected by filtration. The filtrate is diluted with water and ethyl acetate and a second quantity of product collected by filtration. The solids are combined giving [7bR-[2(7′bR*,-8′aS*),7bR*,*aS*]]-2,2′-[carbonylbis(imino-1H-indole-5,2-dicarbonyl)]bis[1,2,8,8a-tetrahydro-7-methyl-cyclopropan(c)pyrrolo[3,2-e]indol-4(5H)-one (38 mg, 52%).
NMR: δ 1.394(m, 2H); 2.008(s+m, 8H); 3.169(m, 2H); 4.44(m, 2H); 4.522(m, 2H); 6.693(s, 2H); 6.881(s, 2H); 7.120(s, 2H); 7.266(d, 2H); 7.402(d, 2H); 7.872(s, 2H); 8.512(bs, 2H); 11.536(bs, 2H); 11.675(bs, 2H).
IR: 3250, 1560, 1375, 1255 cm⁻¹.
UV: λmax=365nm (ε-36000); 316 nm(ε=31000).
MS(FAB): Calc'd. for C₄₃H₃₅N₈O₅:743.2730; found 743.2737. Ions at m/z 543, 368, 201.
DNA BINDING: 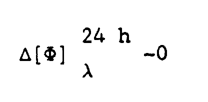
TLC: R_{f}=0.43 in (30-70) DMF-toluene.

### Example 3 Preparation of (R*,S*)-N,N′-bis[2-[[1-(chloromethyl)-1,6-dihydro-5-hydroxy-8-methylbenzo[1,2-b:4,3-b′]dipyrrol-3(2H)-yl]carbonyl]-1H-indol-5-yl]-ethanediamide (Compound 3).

### Part A - Preparation of 5,5′-[(1,2-dioxo, 1,2-ethanediyl)diimino]bis-1H-indole-2-carboxylic acid diethyl ester.

5-Aminoindole-2-carboxylic acid ethyl ester (190.4 mg, 0.93 mM) is dissolved in distilled THF (15 mL) under a nitrogen atmosphere, and the solution cooled at -78°C. Diisopropylethylamine (175 µl, 1.00 mM) is syringed in, followed by oxalyl chloride (41 µL, 0.47 mM). The reaction is stirred at -78°C for one hr. The reaction is evaporated to half volume in vacuo, then treated with water. The product, 5,5′-[(1,2-dioxo-1,2-ethanediyl)diimino]bis-1H-indole-2-carboxylic acid diethyl ester, precipitates as a white solid which is collected by centrifugation and dried 72 hrs at less than 20 torr (232 mg).
NMR: δ 1.348(t, 6H); 4.35(q, 4H); 7.16(s, 2H); 7.45(d, 2H); 7.68(d, 2H); 8.224(s, 2H); 10.732(bs, 2H); 11.92(bs, 2H).
IR: 3360, 3320, 1690, 1670, 1525, 1255, 1215 cm⁻¹.
UV: ^{λ}max=330 nm (ε=5000); 299 nm (ε-10000);
MS(E1): m/z 462(M+), 417, 230, 204, 158.

### Part B - Preparation of 5,5′-[(1,2-dioxo-1,2-ethanediyl)diimino]bis-1H-indole-2-carboxylic acid.

5,5′-[(1,2-dioxo-1,2-ethanediyl)diimino]bis-1H-indole-2-carboxylic acid diethyl ester (220 mg, 0.48 mM) is suspended in pyridine (9 mL) and treated with 1N NaOH (1.5 mL). The milky mixture is heated to 60°C under nitrogen. After 24 hrs N NaOH again added (1 mL) and the reaction heated another 3 hrs. The reaction is then neutralized by the addition of 1N HCl (2.5 mL) and concentrated to one-half volume as less than 20 torr. The residue is then treated with 1N HCl (50 mL) and centrifuged giving a residual slightly pink solid. This is is washed with water, centrifuged, and dried in the vacuum oven leaving the product 5,5′-[(1,2-dioxo-1,2-ethanediyl)di-imino]bis-1H-indole-2-carboxylic acid (148 mg).
NMR: δ 7.098(s, 2H); 7.42(d, 2H, J=9Hz); 7.66(d, 2H, J=9Hz); 8.201(s, 2H); 10.709(bs, 2H); 11.80(bs, 2H).
IR: 3420, 3310, 1660, 1510, 1220 cm⁻¹.
MS(FAB): m/z 407(M+H), 385, 331, 253, 177.
TLC: R_{f} = 0.31 in (40-60-2) DMF-Toluene-acetic acid.

### Part C - Reaction of 5,5′-[(1,2-dioxo-1,2-ethanediyl)diimino]bis-1H-indole-2-carboxylic acid with (S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride].

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (42.5 mg, 0.13 mM) is dissolved in ethyl acetate (1 mL) under a nitrogen atmosphere in the dark. The reaction is treated with ethyl acetate (3 mL) saturated with HCl. The reaction is stirred 1 hr and evaporated in vacuo. Nitrogen is let in when the vacuum is released. (S)-1-(chloromethyl)-5-hydroxy-8-methyl-1,2,3,6-tetrahydro-benzo[1,2-b:4,3-b′]dipyrrole hydrochloride (CPI phenol chloride hydrochloride) as a solid is obtained on re-evaporation 2x with CH₂Cl₂. A suspension of 5,5′-[(1,2-dioxo-1,2-ethanediyl)diimino]bis-1H-indole-2-carboxylic acid (28.4 mg, 0.06 mM) in dimethylacetamide (2 mL) is added to the reaction. EDC is added as a solid (43.4 mg, 0.23 mM). The reaction is stirred in the dark under nitrogen at room temperature for 100 min. Water is added to precipitate the product. The solid, isolated by centrifugation, is washed twice with 5% NaHCO₃, then with 0.1N HCl and water. The solid is dried under vacuum. The solid is adsorbed from DMF onto silica gel, and chromatographed on a silica gel column (3 g) in 20% to 30% DMF in toluene. Fractions of 1-2 mL are collected. (R*,S*)-N,N′-bis[2-[[1-(chloromethyl)-1,6-dihydro-5-hydroxy-8-methylbenzo[1,2-[b:4,3-b′]dipyrrol-3(2H)-yl]carbonyl]-1H-indol-5-yl]-ethanediamide is found in fractions 15-22 (24.6 mg, 45% yield).
NMR: (DMF-d7) δ 2.216(s, 6H); 3.47(m, 2H); 3.77(m, 2H); 3.918(m, 2H); 4.51(m, 4H); 6.95(s, 2H); 7.03(s, 2H); 7.40(d, 2H); 7.62(d, 2H); 8.22(s, 2H).
UV: ^{λ}max=341 nm (ε=38000); 293 nm (ε=52000).
TLC: R_{f} = 0.58 in (30-70) DMF-toluene.

### Example 4 Preparation of [S-(R*,R*)]-6,6′-(1H-pyrrole-2,5-diyldicarbonyl)bis[8-(chloromethyl)-3,6,7,8-benzo[1,2-b:4,3-b′]dipyrrol-4-ol (Compound 4).

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (40 mg, 0.12 nM) is dissolved in ethyl acetate (1 mL) under a nitrogen atmosphere. The reaction is treated with ethyl acetate freshly saturated with HCl (3 mL) and the resultant solution stirred at room temperature for 1 hr. The solvent is removed in vacuo, and the (S)-1-(chloromethyl)-5-hydroxy-8-methyl-1,2,3,6-tetrahydro-benzo[1,2-b:4,3-b′]dipyrrole hydrochloride (CPI phenol chloride hydrochloride) as a purplish residual solid is re-evaporated 2x with CH₂Cl₂, flushing with nitrogen between evaporations. This is treated with pyrrole-2,5-dicarboxylic acid (8.9 mg, 0.057 mM) in dry dimethylacetamide (1 mL) and EDC (24 mg, 0.125 mM) and the resultant solution stirred at 25 C. After 50 min. and additional quantity of EDC is added (24 mg, 0.125 mM). After an additional 55 min the reaction is diluted with water (5 mL) and saturated aqueous sodium chloride solution (5 mL) and extracted with ethyl acetate 4 times. The combined organic layers are dried (Na₂SO₄) and evaporated. The crude residue is adsorbed from ethyl acetate-acetone onto 0.5 g silica gel and chromatographed on a 6 g silica gel column in 10% DMF-toluene followed by 15% DMF-toluene. Fractions of 3-4 ml are collected. [S-(R*,R*)]-6,6′-(1H-pyrrole-2,5-diyldicarbonyl)bis[8-(chloromethyl)-3,6,7,8-benzo[1,2-b:4,3-b′-]dipyrrol-4-ol is found in Fr. 23-37 (25 mg).
NMR: δ 2.349(s, 6H); 3.59(m, 2H); 3.90(m, 2H); 4.01(m, 2H); 4.40(m, 2H); 4.55(m, 2H); 6.850(s, 2H); 7.039(m, 2H); 7.60(m, 2H); 9.77(s, 2H); 10.71(bs, 2H);11.36(bs, 1H).
UV: ^{λ}max = 356 nm (ε=16000); 290 nm (ε=21000).
TLC: R_{f} = 0.47 in (20-80) DMF-toluene.

### Example 5 Preparation of [S-(R*,R*)]-6,6′-(2,5-furandiyldicarbonyl)bis[8-(chloromethyl)-3,6,7,8-tetrahydro-1-methyl-benzo[1,2-b 4,3-b′]dipyrrol-4-ol (Compound 5).

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (40.6 mg, 0.12 mM) is dissolved in ethyl acetate (1 mL), put under nitrogen, and treated with HCl-saturated ethyl acetate (3 mL). After 50 minutes, the reaction is evaporated in vacuo, returning to atmospheric pressure under nitrogen. (S)-1-(chloromethyl)-5-hydroxy-8-methyl-1,2,3,6-tetrahydro-benzo[1,2-b:4,3-b′]dipyrrole hydrochloride (CPI phenol chloride hydrochloride) as a solid is obtained on re-evaporation 2x from CH₂Cl₂. The solid is treated with a solution of furan-2,5-dicarboxylic acid (9.7 mg, 0.062 mM) in dry dimethyl acetamide (1 mL). EDC (43.1 mg 0.22 mM) is added as a solid in 2 equal portions, 45 min apart. The reaction is stopped 170 min after the second addition, by adding ethyl acetate and water. The water is re-extracted with ethyl acetate. The combined organic layers are washed with 0.5N HCl, 1% NaHCO₃, and water. Drying (Na₂SO₄) and concentrated gives a yellow solid (55.2 mg). The solid is adsorbed from DMF-acetone onto silica gel (0.7 g). This is placed on a silica gel column (7 g), eluting with 10% to 15% DMF in toluene. Fractions of 3-4 mL are collected. [S-(R*,R*)]-6,6′-(2,5-furandiyldicarbonyl)bis[8(chloromethyl)-3,6,7,8-tetrahydro-1-methyl-benzo[1,2-b 4,3-b′]dipyrrol-4-ol, as a bright yellow solid, is isolated by concentrating fractions 23-31 (22.3 mg, 63% yield).
NMR: δ 2.344(s, 6H); 3.63(m, 2H); 3.89(m, 2H); 4.029(m, 2H); 4.51(m, 2H); 4.60(m, 2H); 7.048(s, 2H); 7.37(s, 2H); 7.64(m, 2H); 9.82(s, 2H); 10.75(bs, 2H).
UV: ^{λ}max = 360 nm (ε=20000); 295 nm (ε=24000).
TLC: R_{f} = 0.47 in (20-80) DMF-toluene.

### Example 6 Preparation of [S-(R*,R*]-N,N′-bis[2-[[1-(chloromethyl)-1,6-dihydro-5-hydroxy-8-methylbenzo[1,2-b:4,3-b′]dipyrrol-3(2H)-yl]carbonyl]-1H-indol-5-yl]-2,5-furandicarboxamide (Compound 6).

### Part A - Preparation of 5,5′-[2,5-furandiylbis(carbonylimino)]bis-1H-indole-2-carboxylic acid diethyl ester.

Furan-2,5-dicarboxylic acid (50 mg, 0.32 mM, A Sohst and B. Tollens, Justig Liebigs Ann. Chem., 245, 1 (1888)) and 5-aminoindole-2-carboxylic acid ethyl ester (130 mg, 0.64 mM) are dissolved in dimethylacetamide (2 mL) and the solution treated with EDC (132 mg, 0.69 mM). After 20 hrs the reaction is diluted with water (20 mL) and extracted twice with ethyl acetate. The combined ethyl acetate solutions are washed with N HCl and N NaOH, and dried (M_{g}SO₄). Concentration of the ethyl acetate in vacuo leaves a residue (154 mg). This is chromatographed over 10 g of silica gel eluting with 50% to 60% ethyl acetate in hexane followed by 50% ethyl acetate in toluene. Fractions of 4 mL are collected. 5,5′-[2,5-furandiylbis(carbonylimino)]bis-1H-indole-2-carboxylic acid diethyl ester (107 mg) is obtained on evaporation of fr 22-50.
NMR: δ 1.413(t, 6H); 4.38(g, 4H); 7.137(s, 2H); 7.51(d, 2H); 7.58(d, 2H); 8.021(s, 2H); 8.097(bs, 2H); 10.146(bs, 2H); 11.742(bs, 2H).
TLC: R_{f} = 0.38 in (60-40) ethyl acetate-hexane.

### Part B - Preparation of 5,5′-[2,5-furandiylbis(carbonylimino)]bis-1H-indole-2-carboxylic acid.

5,5′-[2,5-furandiylbis(carbonylimino)]bis-1H-indole-2-carboxylic acid diethyl ester (60 mg, 0.11 mM) is suspended in methanol (0.4 mL) and treated with N NaOH (0.25 mL). After 5 days the reaction is evaporated in vacuo and the residue dissovlved in water and the solution acidified to pH 2 with N HCl. This solution is freeze dried leaving 5,5′-[2,5-furandiylbis(carbonylimino)]bis-1H-indole-2-carboxylic acid (22 mg).

### Part C - Reaction of 5,5′-[2,5-furandiylbis(carbonylimino)]bis-1H-indole-2-carboxylic acid with (Boc)CPI phenol chloride.

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (33 mg, 0.098 mM) is dissolved in ethyl acetate (1 mL) and the resultant solution stirred under an Ar atmosphere and treated with a freshly prepared saturated HCl solution in ethyl acetate (3 mL). After 45 min the mixture is evaporated in vacuo and the resultant (S)-1-(chloromethyl)-5-hydroxy-8-methyl-1,2,3,6-tetrahydro-benzo[1,2-b:4,3-b′]dipyrrole hydrochloride (CPI phenol chloride hydrochloride) twice treated with CH₂Cl₂ and re-evaporated. This is treated with dimethylacetamide (1 mL) containing 5,5′-[2,5-furandiylbis(carbonylimino]bis-1H-indole-2-carboxylic acid (22 mg, 0.047 mg). The resultant solution is treated in 2 portions about 50 min apart with EDC (40 mg, 0.21 mM). After 50 min the reaction is diluted with water and mixture extracted with THF-ethyl acetate. The combined organic layers are dried over MgSO₄ and concentrated in vacuo leaving a solid (15 mg). This material is chromatographed over silica gel (5 g) eluted with 20% to 30 % DMF in toluene. Fractions of 3 mL are collected. [S-(R*,R*]-N,N′-bis[2-[[1-(chloromethyl)-1,6-dihydro-5-hydroxy-8-methylbenzo[1,2-b:4,3-b′]dipyrrol-3(2H)-yl]-carbonyl]-1H-indol-5-yl]-2,5-furandicarboxamide (5 mg) is obtained on evaporation of fr 16-28.
NMR: δ 2.363(s, 6H); 3.60(m, 2H); 3.90(m, 2H); 4.03(m, 2H); 4.56(m, 2H); 4.68(m, 2H); 6,616(s, 1H); 7.05(s, 2H); 7.16(m, 2H); 7.41(s, 2H); 7.53(s, 4H); 7.64(m, 1H); 8.16(s, 2H); 9.79(s, 2H); 10.29(bs, 2H); 10.73(bs, 2H); 11.73(bs, 2H).
UV: ^{λ}max (MeOH) = 350 nm (ε=41000); 283 nm (ε=50500).
MS(FAB): m/z 911 (M + H), 909 (M + H), 412. 395, 335, 300, 253, 210.
TLC: R_{f} = 0.217 in (20-80) DMF-toluene.

### Example 7 Preparation of [S-(R*,R*)]-N,N′-bis[2[[1-(chloromethyl)-1,6-dihydro-5-hydroxy-8-methylbenzo [1,2-b 4,3-b′]dipyrrol-3(2H)-yl]carbonyl]-1H-indol-5-yl]-1H-pyrrole-2,5-dicarboxamide (Compound 7).

### Part A - Coupling of pyrrole-2,5-dicarboxylic acid to 5-aminoindole-2-carboxylic acid ethyl ester.

Pyrrole-2,5-dicarboxylic acid (50 mg, 0.32 mM, Kuhn, R. and Dury, K., Justus Liebigs Ann. Chem. 571, 44 (1951)) and 5-aminoindole-2-carboxylic acid ethyl ester (130 mg, 0.64 mM) are dissolved in dimethylacetamide (2 mL) and the solution treated with EDC (129 mg, 0.67 mM). After 22 hrs the mixture is treated with ice water (20 mL) and the precipitated solid filtered. This material is chromatographed over silica gel (10 g), eluted with 10% to 15% DMF in toluene. Fractions of 4 mL are collected. A residue containing the product (72 mg) is obtained on evaporation of fr. 22-42. This residue is triturated with ethyl acetate and dried leaving 5,5′-[1H-pyrrole-2,5-diylbis(carbonylimino)-bis-1H-indole-2-carboxylic acid diethyl ester (42 mg).
NMR: δ1.413(t, 6H); 4.38(q, 4H); 7.137(s, 2H); 7.51(d, 2H); 7.58(d, 2H); 8.021 (s, 2H); 8.097(bs, 2H); 10.146(bs, 2H); 11,742(bs, 2H).
TLC: R_{f} = 0.38 in (60-40) ethyl acetate-hexane.

### Part B - Preparation of 5,5′-[1H-pyrrole-2,5-diylbis(carbonylimino)-bis-1H-indole-2-carboxylic acid

5,5′-[1H-pyrrole-2,5-diylbis(carbonylimino)-bis-1H-indole-2-carboxylic acid diethyl ester (42 mg, 08 mM) is dissolved in pyridine (1 mL) and the solution treated with N NaOH (0.2 mL). After 24 hrs the reaction is treated with additional N NaOH (0.4 mL). After an additional 96 hrs the reaction is evaporated in vacuo and the residue dissolved in water (5 mL). The aqueous solution is acidified with N HCl to pH 2 and then freeze-dried, leaving 5,5′-[1H-pyrrole-2,5-diylbis(carbonylimino)-bis-1H-indole-2-carboxylic acid.
TLC: R_{f} = 0.18 in (2-15-85) acetic acid-DMF-toluene.

### Part C - Coupling of 5,5′-[1H-pyrrole-2,5-diylbis(carbonylimino)-bis-1H-indole-2-carboxylic acid with (S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride]

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (40 mg, 0.12 nM) is dissolved in ethyl acetate (1 mL) under a nitrogen atmosphere. The reaction is treated with ethyl acetate freshly saturated with HCl (3 mL) and the resultant solution stirred at room temperature for 45 min. The solvent is removed in vacuo, and the residue returned to atmospheric pressure with nitrogen. The residue is twice re-evaporated with CH₂Cl₂ leaving (S)-1-(chloromethyl)-5-hydroxy-8-methyl-1,2,3,6-tetrahydro-benzo[1,2-b:4,3-b′]dipyrrole hydrochloride (CPI phenol chloride hydrochloride). This material is dissolved in dimethylacetamide (1 mL) and the solution treated with 5,5′-[1H-pyrrole-2,5-diylbis(carbonylimino)-bis-1H-indole-2-carboxylic acid (28 mg) and with EDC (25 mg, 0.13 mM). After 50 min the reaction is treated with additional EDC (25 mg). After another 50 min the reaction is diluted with water and the precipitated solids partitioned into THF-ethyl acetate. The combined organic layers are dried (MgSO₄) and concentrated in vacuo leaving a solid (80 mg). This material is chromatographed over a silica gel (10 g) eluted with 20% DMF in toluene. Fractions of 3 mL are collected. [S-(R*,R*)]-N,N′-bis[2[[1-(chloromethyl)-1,6-dihydro-5-hydroxy-8-methylbenzo [1,2-b 4,3-b′]dipyrrol-3(2H)-yl]carbonyl]-1H-indol-5-yl]-1H-pyrrole-2,5-dicarboxamide (11 mg) is obtained on evaporation of fr 26-45.
NMR: δ2.363(s, 6H); 3.60(m, 2H); 3.91(m, 2H); 4.032(m, 2H); 4.54(m, 2H); 4.68(m, 2H); 6.625(s, 1H); 7.05(s, 2H); 7.09(s, 2H); 7.13(s, 2H); 7.49(m, 4H); 7.64(m, 1H); 8.18(s, 2H); 9.78(s, 2H); 10.09 (bs, 2H); 10.72(bs, 2H); 11.67(bs, 2H); 12.22(bs, 1H).
UV: ^{λ}max(methanol) = 344 nm (ε=58600); 296 nm (ε=68000).
MS(FAB): Calc'd for C₄₈H₃₉Cl₂N₉O₆; 908.2478;
Found: 908.2496. ions at m/z 543, 368, 201.
TLC: R_{f} = 0.21 in (20-80)DMF-toluene.

### Example 8 Preparation of [R-(R*,R*)]-N,N′-bis[2-[[1-(chloromethyl)-1,6-dihydro-5-hydroxy-8-methylbenzo[1,2-b 4,3-b′]dipyrrol-3(2H)-yl]carbonyl]-1H-indol-5-yl]-propanediamide (Compound 8).

### Part A - Reaction of 5-aminoindole-2-carboxylic acid ethyl ester with malonyl dichloride.

A solution of 5-aminoindole-2-carboxylic acid ethyl ester (196 mg, 0.96 mM) in dry distilled THF (15 ml) is cooled in an ice bath under nitrogen. N,N-Diisopropylethylamine (350 µL, 2.01 mM) and malonyl dichloride (80 µL, 0.82 mM) are added by syringe. After one hour the solvent is partially removed and 5,5′-[(1,3-dioxo-1,3-propanediyl)-diimino]bis-1H-indole-2-carboxylic acid diethyl ester precipitated with water. The mixture is centrifuged, and the liquid layer withdrawn. The solid is dried under vacuum. The residue is chromatographed over silica gel (2 g) eluted with 25% acetone in toluene, 40% acetone in toluene, 60% acetone in toluene, 80% acetone in toluene and acetone. Fractions of 15 mL are collected. The product 5,5′-[(1,3-dioxo-1,3-propanediyl)-diimino]bis-1H-indole-2-carboxylic acid diethyl ester is found in fr 9-13 (77 mg).
NMR: (Acetone-d6) δ1.37(t, 6H); 3.571(s, 2H); 4.36(q, 4H); 7.16(s, 2H); 7.48(m, 4H); 8.17(s, 2H); 9.71(bs, 2H); 10.92(bs, 2H).
IR: 3300, 1650, 1200 cm⁻¹.
UV: ^{λ}max = 340 nm (ε=5400); 297 (ε=28000).
MS(El): m/z476(M+), 340, 272, 226, 204, 158.
TLC: R_{f} = 0.21 in (40-60) Acetone-hexane.

### Part B - Preparation of 5,5′-[(1,3-dioxo-1,3-propanediyl)-diimino]bis-1H-indole-2-carboxylic acid.

The diester of Part A (0.16 mM) is dissolved in pyridine (3 ml) and 1 N sodium Hydroxide (0.53 ml). The reaction mixture is stirred under nitrogen at 50°C for about 2 hours, then at room temperature for 4 hours. The reaction mixture is treated with 1 N HCl and evaporated to near dryness. The product is precipitated with water and isolated by centrifugation, and washed with water, leaving 5,5′-[(1,3-dioxo-1,3-propanediyl)-diimino]bis-1H-indole-2-carboxylic acid.
NMR: δ 3.47(s,2H); 7.04(s,2H); 7.36(m,4H); 8.03(s,2H); 10.09(bs,2H); 11.68(bs,2H).
UV: ^{λ}max = 294 nm (ε = 13000).

### Part C - Coupling of 5,5′-[(1,3-dioxo-1,3-propanediyl)diimino]-bis-1H-indole-2-carboxylic acid and (S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride].

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (37.8 mg, 0.11 mM) is dissolved in ethyl acetate (0.5 mL) under a nitrogen atmosphere. The solution is treated with HCl-saturated ethyl acetate (3 mL) for one hr. The reaction is evaporated, releasing the vacuum with nitrogen. The resultant (S)-1-(chloromethyl)-5-hydroxy-8-methyl-1,2,3,6-tetrahydro-benzo[1,2-b:4,3- b′]dipyrrole hydrochloride (CPI phenol chloride hydrochloride) is re-evaporated 2x with CH₂Cl₂. This material is treated with 5,5′-[(1,3-dioxo-1,3-propanediyl)diimino]bis-1H-indole-2-carboxylic acid, (24.3 mg, 0.058 mM) in dry dimethylacetamide (1 mL). EDC (49 mg, 0.156 mM) is weighed out and about one half is added to the reaction. The mixture is stirred at room temperature 45 min. Then the rest of the EDC is added, and stirred is continued 40 min. The reaction is diluted with ethyl acetate and water. The insoluble materials are removed by filtration and saved. The filtrate is separated into layers and the aqueous re-extracted with ethyl acetate. The combined organic layers are dried (Na₂SO₄) and concentrated in vacuum. The residue is combined with the filtered solids from above and are absorbed onto silica gel (0.4 g) and added to the top of a silica gel column (5 g). The column is eluted with 22% to 28% DMF in toluene, collecting fractions of 2 mL. The product 5,5′-[(1,3-dioxo-1,3-propanediyl)diimino]bis-1H-indole-2-carboxylic acid is isolated in fractions 14-28 (36 mg).
NMR: δ 2.354(s, 6H); 3.51(m, 2H); 3.60(m, 2H); 3.91(m, 2H); 4.03(m, 2H); 4.52(m, 2H); 4.67(m, 2H); 7.05(s, 2H); 7.10(s, 2H); 7.38(d, 2H); 7.46(d, 2H); 7.64(m, 2H); 8.10(s, 2H); 9.78(s, 2H); 10.13(bs, 2H); 10.72(bs, 2H); 11.64(bs, 2H).
UV: ^{λ}max = 336 nm(ε=35000); 294 nm (ε=44000).
TLC: R_{f} = 0.46 in (30-70) DMF-toluene.

### Example 9 Preparation of [S-(R*,R*)]-6,6′-[carbonylbis(imino-1H-indole-5,2-diylcarbonyl)]bis[8-(chloromethyl)-3,6,7,8-tetrahydro-1-methyl-benzo[1,2-b:4,3-b′]dipyrrol-4-ol diacetate (Compound 9).

[7bR-[2(7′bR*,8′aS*),7bR*,8aS*]]-2,2′-[carbonylbis(imino-1H-indole-5,2-dicarbonyl)]bis[1,2,8,8a-tetrahydro-7-methyl-cyclopropa(c)pyrrolo[3,2-e]indol-4(5H)-one (5 mg, 0.007 mM) is dissolved in DMF (200 µl) and acetone (0.5 ml) and the solution treated with pyridine hydrochloride (8.3 mg, 0.07 mM). The reaction is stirred at room temperature in the dark for 110 minutes and then concentrated to a small volume, and treated with water. The mixture is centrifuged and the liquids removed leaving [S-(R*,R*)]-6,6′-[carbonylbis(imino-1H-indole-5,2-dicarbonyl)]bis[8-chloromethyl)-3,6,7,8-tetrahydro-1-methyl-benzo[1,2-b:4,3-b′]dipyrrol-4-ol as a solid. The solid is dissolved in pyridine (100 µL) and treated with acetic anhydride (6 µL, 0.06 mM). The reaction is stirred in the dark at room temperature for 85 minutes, quenched with water and concentrated to dryness. The residue is treated with water and centrifuged. The liquid is removed and the solid dried under vacuum. This solid is adsorbed from DMF onto silica gel (0.07 g). Silica with absorbed compound is placed on a silica gel column (1 g), eluting with 10% to 40% DMF in toluene. Fractions of 1/2 mL are taken. [S-(R*,R*)]-6,6′-[carbonylbis(imino-1H-indole-5,2-diylcarbonyl)]bis[8-(chloromethyl)-3,6,7,8-tetrahydro-1-methyl-benzo[1,2-b 4,3-b′]dipyrrol-4-ol diacetate elutes in fractions 38-47 (3.4 mg).
NMR(DMSO-d6, TMS): δ 2.37 (s, 6H); 2.40(s, 6H); 3.72(m, 2H); 3.99 (m, 2H); 4.19 (m, 2H); 4.61(m, 2H); 4.74(m, 2H); 7.09 (s, 2H); 7.24 (m, 4H); 7.43 (m, 2H); 7.83 (bs, 2H); 7.87 (m, 2H); 8.50 (bs, 2H); 11.12 (bs, 2H); 11.56 (bs, 2H).
UV(DMA+MeOH): λmax = 325nm (ε=43,800); 294nm (ε = 43,800).

### Example 10 Preparation of [S-(R*,R*)]-6,6′-(1H-indole-2,5-diyldicarbonyl)bis[8-(chloromethyl)-3,6,7,8-tetrahydro-1-methyl-benzo[1,2-b:4,3-b′]dipyrrol-4-ol (Compound 10).

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (49 mg, 0.146 mM) is stirred at room temperature under nitrogen in ethyl acetate (0.6 mL). Ethyl acetate saturated with gaseous HCl (3 mL) is added and the reaction followed by TLC and is complete in about 45 minutes. The reaction is evaporated, releasing the vacuum with nitrogen. The resultant (S)-1-(chloromethyl)-5-hydroxy-8-methyl-1,2,3,6-tetrahydro-benzo[1,2-b:4,3-b′]dipyrrole hydrochloride (CPI phenol chloride hydrochloride) is re-evaporated 2x with CH₂Cl₂. The residual CPI phenol chloride hydrochloride salt is dissolved in dry dimethylacetamide (1 mL) and stirred at room temperature under nitrogen. Indole-2,5-dicarboxylic acid (15 mg, 0.073 mM) is added, followed by EDC (40 mg, 0.21 mM). After 45 minutes, additional EDC (23 mg, 0.12 mM) is added to the reaction mixture and which is then left to react for 1 hour. The reaction mixture is transferred to a centrifuge tube, rinsing in reaction with DMF (0.5 mL) and diluted with water to precipitate the product which is centrifuged and decanted. The crude product is transferred to a round bottom flask with acetone and the resulting solution evaporated under vacuum. The crude product is coated on silica gel (0.5 g) and chromatographed over a silica gel column (6 g) made up in 20% DMF in toluene and eluted with the same solvent collected 1 ml fractions. [S-(R*,R*)]-6,6′-(1H-indole-2,5-diyldicarbonyl)bis[ 8-(chloromethyl)-3,6,7,8-tetrahydro-1-methyl-benzo[1,2-b:4,3-b′]dipyrrol-4-ol is collected in fractions 22-47 and obtained on evaporation to dryness (31 mg).
NMR(d₆-DMSO,TMS): δ 2.33(s, 3H); 2.36(s, 3H); 3.54-3.68(t, 2H); 3.78-3.96(m, 3H); 3.98-4.11(t, 2H); 4.23-4.44(bs, 1H); 4.50-4.60(d, 1H); 4.62-4.75(t, 1H); 7.02(s, 1H); 7.05(s, 1H); 7.22(s, 1H); 7.44-7.53(d, 1H); 7.53-7.62(d, 1H); 7.62-7.73(bs, 1H); 7.95(s, 1H); 7.98(s, 1H); 9.60-9.75(bs, 1H); 9.80(s, 1H); 10.66(s, 1H); 10.73(s, 1H); 11.93(s, 1H).

### Example 11 Preparation of [S-(R*,R*)]-N,N′-bis[2-[[1-(chloromethyl)-1,6-dihydro-5-hydroxy-8-methylbenzo[1,2-b:4,3-b′]dipyrrol-3(2H)-yl]carbonyl]-1H-indol-5-yl]-1H-indole-2,5-dicarboxamide (Compound 11).

### Part A - Preparation of 5,5′-[1H-indole-2,5-diylbis(carbonylimino)bis-1H-indole-2-carboxylic acid diethyl ester.

Indole-2,5-dicarboxylic acid (200 mg, 0.49 mM) is stirred at room temperature under nitrogen in dry DMF (4 mL). To this is added 5-amino-indole-2-carboxylic acid ethyl ester (400 mg, 1.96 mM) and EDC (200 mg, 1.05mM). After 3 days, the reaction mixture is transferred to a centrifuge tube and diluted with water. The precipitated solid is centrifuged and the supernatant liquid decanted. The residue is washed with acetone leaving 5,5′-(1H-indole-2,5-diylbis(carbonylimino)bis-1H-indole-2-carboxylic acid diethyl ester (78 mg). Additional 5,5′-[1H-indole-2,5-diylbis(carbonylimino)bis-1H-indole-2-carboxylic acid diethyl ester is obtained from the above acetone washings by concentration to dryness and chromatography over silica gel (40 g) eluting with 10% - 20% DMF in toluene. Factions of 15 mL are collected and the 5,5′-[1H-indole-2,5-diylbis(carbonylimino)bis-1H-indole-2-carboxylic acid diethyl ester (83 mg) is obtained on evaporation to dryness of fractions 35-40.

### Part B - Preparation of 5,5′-[1H-indole-2,5-diylbis(carbonylimino)]bis-1H-indole-2-carboxylic acid.

5,5′-[1H-indole-2,5-diylbis(carbonylimino)]bis-1H-indole-2-carboxylic acid diethyl ester (161 mg, 0.28 mM) is dissolved with stirring under nitrogen at room temperature in pyridine (5 mL) absolute ethanol (5 mL). To the resultant solution is added N NaOH (1 mL) and which is allowed to stand for 5 hr. The solvent is then mostly evaporated under vacuum, water is added, and the reaction mixture freeze-dried. The resultant crude product is coated on Celite (1.5 g) and added to the top of a C-18 reverse phase silica gel column (1.5 g). The column is eluted with the following: 50% DMF in water (200 mL); 60% DMF in water (100 mL); 70% DMF - 30% water (100 ml); 80% DMF-20% water (300 ml). Fractions of 5 ml are collected, analyzing them by TLC. 5,5′-[1H-indole-2,5-diylbis(carbonylimino)]bis-1H-indole-2-carboxylic acid (46 mg) is found in fractions 7-21.

### Part C - Reaction of 5,5′-[1H-indole-2,5-diylbis(carbonylimino)]bis-1H-indole-2-carboxylic acid with (Boc)CPI phenol chloride.

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (59 mg, 0.176 mM) is stirred at room temperature under nitrogen in dark for 45 min in ethyl acetate (1 mL) and ethyl acetate saturated with gaseous HCl (4 mL). TLC after 30 min shows reaction to be complete. The reaction mixture is evaporated under vacuum, and re-evaporated with CH₂Cl₂ 2x, letting in nitrogen each time giving (S)-1-(chloromethyl)-5-hydroxy-8-methyl-1,2,3,6-tetrahydro-benzo[1,2-b:4,3-b′]dipyrrole hydrochloride (CPI phenol chloride hydrochloride). The CPI phenol chloride hydrochloride salt is dissolved with stirring under nitrogen in dark in dry DMA (1 mL). The solution is treated with 5,5′-[1H-indole-2,5-diylbis(carbonylimino)]bis-1H-indole-2-carboxylic acid (46 mg, 0.088 mM) and EDC (50 mg, 0.26 mM). After 30 min additional EDC is added (26 mg, 0.13 mM). The reaction is allowed to stand an additional 1 hr then transferred to a centrifuge tube and washed in with DMF (1 mL). Water is added to precipitate product which is then collected by centrifugation. The solid is washed into a RB flask with acetone and evaporated under vacuum. The residue is coated on silica gel (1 g) and chromatographed over a silica gel column (9 g). The column is eluted with 25% DMF in toluene followed by 30% and 40% DMF in toluene. Fractions of 2 ml are collected, and analyzed by TLC. [S(R*,R*)]-N,N′-bis[2-[[1-(chloromethyl)-1,6-dihydro-5-hydroxy-8-methylbenzo-[1,2-b 4,3-b′]dipyrrol-3(2H)-yl]carbonyl]-1H-indol-5-yl]-1H-indole-2,5-dicarboxamide is found in fractions 31-80. Impure product is found in fractions 8-30. These are rechromatographed over a silica gel column (4.5 g) eluted with 20% DMf in toluene to 30% DMF in toluene. Fractions of 1 mL are collected. [S-(R*,R*)]-N,N′-bis[2-[[1-(chloromethyl)-1,6-dihydro-5-hydroxy-8-methylbenzo[1,2-b 4,3-b′]dipyrrol-3(2H)-yl]carbonyl]-1H-indol-5-yl]-1H-indole-2,5-dicarboxamide is found in fractions 25-75. Combination of the material from the two chromatographies gives [S-(R*,R*)]-N,N′-bis[2-[[1-(chloromethyl)-1,6-dihydro-5-hydroxy-8-methylbenzo[1,2-b 4,3-b′]dipyrrol-3(2H)-yl]carbonyl]-1H-indol-5-yl]-1H-indole-2,5-dicarboxamide (47 mg).
NMR(d₆-DMSO,TMS): δ 2.37(s, 6H); 3.55-3.68(t, 2H); 3.87-3.97(d, 2H); 3.97-4.10(t, 2H); 4.51-4.61(d, 2H); 4.61-4.77(t, 2H); 7.07(s, 2H) 7.14(s, 1H); 7.17(s, 1H); 7.45-7.75(m, 8H); 7.96(s, 1H); 8.23(s, 2H); 8.26(s, 1H); 8.46(s, 1H); 9.82(s, 2H); 10.18(s, 1H); 10.33(s, 1H); 10.75(s, 2H); 11.66(s, 1H); 11.71(s, 1H); 12.09(s, 1H).
UV(DMA+MeOH): λmax = 328nm (ε = 33,560); 281nm (ε = 38,350)

### Example 12 Preparation of [S-R*,R*)]-2-[[1-(chloromethyl)-1,6-dihydro-5-hydroxy-8-methyl-benzo[1,2-b:4,3-b′]dipyrrol-3(2H)-yl]-carbonyl]-N-[3-[[1-(chloromethyl)-1,6-dihydro-5-hydroxy-8-methylbenzo[1,2-b:4,3-b′]dipyrrol3(2H)-yl]carbonyl]-1H-indol-6-yl]-1H-indole-5-carboxamide (Compound 12);

### Part A - Preparation of 5-[[(2-carboxy-1H-indol-5-yl)amino]carbonyl]-1H-indole-2-carboxylic acid

Indole-2,5-dicarboxylic acid (98 mg, 0.42 mmoles) and 5-aminoindole-2-carboxylic acid ethyl ester (86 mg, 0.42 mM) are stirred at room temperature under nitrogen in dry DMF (2 mL). EDC (90 mg, 0.47 mM) is added and left to react for 25 hr. The reaction mixture is diluted with water and centrifuged and the supernatant is decanted. The residual solid is dissolved in acetone and re-evaporated and then is chromatographed over silica gel (20 g), and eluted with 50% ethyl acetate in hexane followed by 30% acetone in hexane. Fractions of 15 ml are collected, and analyzed by TLC. 5-[[(2-carboxy-1H-indol-5-yl)amino]carbonyl]-1H-indole-2-carboxylic acid diethyl ester is found in fractions 8-86 (137 mg, 78% yield).

### Part B - Preparation of 5-[[(2-carboxy-1H-indol-5-yl)amino]carbonyl]-1H-indole-2-carboxylic acid

5-[[(2-carboxy-1H-indol-5-yl)amino]carbonyl]-1H-indole-2-carboxylic acid diethyl ester (137 mg, 0.33mM) is dissolved with stirring at room temperature under nitrogen in pyridine (3 mL) and absolute ethanol (3 mL). 1N NaOH (1 mL) is added and the reaction left to react for 29 hr during which time a precipitate forms. 1N HCl (1 mL) is added and the reaction mixture concentrated on a rotary evaporator. Water is added to the residue, forming a cloudy solution. The solution is freeze-dried. The residue is coated on celite (1 g) which is then added to the top of a C-18 reversed phase silica gel column (10 g). The column is eluted with 40% DMF water to 80% DMF water in 10 % increments (100 mL each). Fractions of 5 mL are collected. 5-[[(2-carboxy-1H-indol-5-yl)amino]carbonyl]-1H-indole-2-carboxylic acid is found in fractions 30-70 (40 mg).

### Part C - Reaction of 5-[[(2-carboxy-1H-indol-5-yl)amino]carbonyl]-1H-indole-2-carboxylic acid with (Boc)CPI phenol chloride

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (74 mg, 0.22 mM) is stirred at room temperature under nitrogen in dark for 45 min in ethyl acetate (1 mL) and the ethyl acetate saturated with gaseous HCl (5 mL). The reaction mixture is evaporated under vacuum, and re-evaporated with CH₂Cl₂ 2x, letting in nitrogen each time leaving (S)-1-(chloromethyl)-5-hydroxy-8-methyl-1,2,3,6-tetrahydro-benzo[1,2-b:4,3-b′]dipyrrole hydrochloride (CPI phenol chloride hydrochloride). The CPI phenol chloride hydrochloride salt is dissolved with stirring under nitrogen in dark in dry DMA (1 mL). 5-[[(2-carboxy-1H-indol-5-yl)amino]carbonyl]-1H-indole-2-carboxylic acid (40 mg, 0.11 mM) and EDC (75 mg, 0.39 mM) are added to the reaction and left to react for 30 min, when addittional EDC (20 mg, 0.10 mM) is added and left to react for 1 hr more. The solution is transferred to a centrifugal tube and washed in with DMF (1 mL), diluted with water to precipitate product, and centrifuged. The supernatant is decanted. The solid is washed into RB flask with acetone and re-evaporated under vacuum.

The crude product is coated on silica gel (1 g) and placed on top of a silica gel column (9 g) made up in 20% DMF-80% toluene. The column is eluted with 20% DMF in toluene, followed by 30% DMF in toluene. Fractions of 2 ml are collected. Fractions 21-78 are collected and evaporated and the residue rechromatographed over a silica gel column (5 g) eluted with 20% DMF in toluene. Fractions of 1 mL are collected. [S-(R*,R*)]-2-[[1-(chloromethyl)-1,6-dihydro-5-hydroxy-8-methyl-benzo[1,2-b:4,3-b′]dipyrrol-3(2H)-yl]-carbonyl]-N- [3-[[1-(chloromethyl)-1,6-dihydro-5-hydroxy-8-methylbenzo[1,2-b:4,3-b′]dipyrrol-3(2H)-yl]carbonyl]-1H-indole-6-yl]-1H-indole-5-carboxamide is found in fractions 20-45 (49 mg, 56% yield).
NMR(d₆-DMSO,TMS(: δ 2.37(s, 6H); 3.54-3.68(t, 2H); 3.87-3.97(d, 2H); 3.98-4.10((t, 2H); 4.50-4.60(d, 2H); 4.61-4.77(q, 2H); 7.07(s, 2H); 7.13(s, 1H); 7.29(s, 1H); 7.45-7.52(d, 1H); 7.55-7.73(m, 4H); 7.91(s, 1H); 8.23(s, 1H); 8.45(s, 1H); 9.82(s, 1H); 9.84(s, 1H); 10.18(s, 1H); 10.75(s, 1H); 10.76(s, 1H); 11.65(s, 1H); 12.00(s, 1H).
UV(DMA+MeOH): λmax = 328nm (ε = 36,830); 292nm sh (ε = 43,800)

### Example 13 Preparation of [S-(R*,R*)]-5-[[1-(chloromethyl)-1,6-dihydro-5-hydroxy-8-methylbenzo[1,2-b:4,3-b′]dipyrrol-3(2H)-yl]-carbonyl)-N-[2-[[1-(chloromethyl)-1,6-dihydro-5-hydroxy-8-methylbenzo[1,2-b:4,3-b′]dipyrrol-3(2H)-yl]carbonyl)-1H-indol-5-yl]-1H-indole-2-carboxamide (Compound 13).

### Part A - Preparation of 5-[[(5-carboxy-1H-indol-2-yl)carbonyl]amino]-1H-indole-2-carboxylic acid diethyl ester

Indole-2,5-dicarboxylic acid 5-ethyl ester (65 mg, 0.28 mM) is stirred at room temperature under nitrogen in dry DMF(1 mL). 5-Amino-indole-2-carboxylic acid (57 mg, 0.28 mM) and EDC (60 mg, 31 mM) are added. After 24 hr the reaction is diluted with water and the solids collected by filtration. The residual solid is dissolved in acetone and evaporated onto silica gel (2 g). This material is added to the top of a silica gel column (18 g) and eluted with 10% DMF in toluene. 5-[[(5-carboxy-1H-indol-2-yl)carbonyl]amino]-1H-indole-2-carboxylic acid diethyl ester (75 mg, 64% yield) is found in fractions.

### Part B - Preparation of 5-[[(5-carboxy-1H-indol-2-yl)carbonyl]amino]-1H-indole-2-carboxylic acid

5-[[(5-carboxy-1H-indol-2-yl)carbonyl]amino-1H-indole-2-carboxylic acid diethyl ester (75 mg, 0.18 mM) is stirred at room temperature under nitrogen in pyridine (2 mL), absolute ethanol (2 mL) and 1N NaOH (600 µL) for 120 hr at 25°C. Additional 1N NaOH (1 mL) is then added and the reaction heated for 20 hr at 50°C. 1N HCl (1.6 mL) is then added and the reaction evaporated under vacuum. The residue is coated on Celite (1 g) and placed in an C-18 silica gel column (10 g). The column is eluted with 50% DMF in water to 80% DMF in water (100 ml each in 10% increments). Fractions of 5 ml are collected and analyzed by TLC. 5-[[(5-carboxy-1H-indol-2-yl)carbonyl]amino]-1H-indole-2-carboxylic acid is found in fractions 5-77.

### Part C - Reaction of 5-[[(5-carboxy-1H-indol-2-yl)carbonyl]-amino]-1H-indole-2-carboxylic acid with (Boc)CPI phenol chloride

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (121 mg, 0.36 mM) is stirred at room temperature under nitrogen in dark for 30 min in ethyl acetate (2 mL) and ethyl acetate saturated with gaseous HCl (8 mL). TLC after 30 min shows the reaction to be complete. The reaction is evaporated under vacuum and re-evaporated with CH₂Cl₂ 2x, letting in nitrogen each time giving (S)-1-(chloromethyl)-5-hydroxy-8-methyl-1,2,3,6-tetrahydrobenzo[1,2-b:4,3-b′]dipyrrole hydrochloride (CPI phenol chloride hydrochloride). The CPI phenol chloride hydrochloride salt is dissolved with stirring under nitrogen in the dark in dry DMA (1.5 mL). 5-[[(5-carboxy-1H-indol-2-yl)carbonyl]amino]-1H-indole-2-carboxylic acid (65 mg, 0.18 mM) and EDC (110 mg, 0.57 mM) are added to the resultant solution. After 30 min reaction is treated with additional EDC (45 mg, 0.23 mM) and left to react 1.5 hr more. The raction solution is transferred to a centrifuge tube and washed in with DMF (1 mL), diluted with water to precipitate product, and centrifuged. The supernatant is decanted. The solid is washed into RB flask with acetone and re-evaporated under vacuum. The crude product is coated on silica gel (1 g) and placed on top of the silica gel column (10 g) made up in 20% DMF-80% toluene. The column is eluted with 20% DMF in toluene, followed by 35% DMF in touene. Fractions of 2 ml are collected. [S-(R*,R*)]-5-[[1-(chloromethyl)-1,6-dihydro-5-hydroxy-8-methylbenzo[1,2-b:4,3-b′]dipyrrol-3(2H)-yl]carbonyl)-N-[2-[[1-(chloromethyl)-1,6-dihydro-5-hydroxy-8-methyl-benzo[1,2-b:4,3-b′]dipyrrol-3(2H)-yl]carbonyl)-1H-indol-5-yl]-1H-indole-2-carboxamide (43 mg) is isolated from fractions 25-47.
NMR(d₆-DMSO,TMS): δ 2.33(s, 3H); 2.37(s, 3H); 3.50-3.70(t, 2H); 3.78-3.97(m, 3H); 3.97-4.14(t, 2H); 4.24-4.43(bs, 1H); 4.48-4.62(d, 1H); 4.62-4.75(t, 1H); 7.04(s, 1H); 7.06(s, 1H); 7.16(s, 1H); 7.40-7.72(m, 7H); 8.00(s, 1H); 8.25(s, 1H); 9.64-9.80(bs, 1H); 9.81(s, 1H); 10.30(s, 1H); 10.68(s, 1H); 10.75(s, 1H); 11.70(s, 1H); 12.03(s, 1H).
UV(DMA+MeOH): λmax = 316nm sh (ε = 36,830); 295nm (ε = 40,030)

### Example 14 Preparation of [S-(R*,R*)]-carbonylbis[imino-1H-indole-5,2-diylcarbonyl[1-(chloromethyl)-1,6-dihydro-8-methylbenzo[1,2-b:4,3-b′]dipyrrole-3,5(2H)-diyl]]ester, 2,2-dimethylpropanoic acid (Compound 14).

[7bR-[2(7′bR*,8′aS*), 7bR*,8aS*]]-2,2′-[carbonylbis(imino-1H-indole-5,2-dicarbonyl)]bis[1,2,8,8a-tetrahydro-7-methyl-cyclopropa(c)pyrrolo[3,2-e]indol-4(5H)-one (5.4 mg, 7.3 µM) is dissolve in pyridine (200 µL) and the the solution treated with 2,2-dimethylpropionic acid chloride (24 µL, 192 µM) at 0 C. After 10 minutes the reaction is warmed to 25 C and is stirred 20 min at that temperature. The reaction is then quenched with water (0.1 mL) and concentrated to dryness. The residue is dissolved in DMF and evaporated under vacuum onto silica gel (0.1 g). This material is added to the top of a silica gel column (1 g) and eluted wiht 10% to 30% DMF in toluene. Fractions of 0.2 mL and collected. Fractions 17-50 are evapoarated to dryness and the residue washed with acetone. The residue from the acetone wash is then chromatographed over a silica gel column (0.3 g) eluted with 10%-12% DMF in toluene. Fractions of 0.2 mL are collected. [S-(R*,R*)]-carbonylbis[imino-1H-indole-5,2-diylcarbonyl[1-(chloromethyl)-1,6-dihydro-8-methylbenzo[1,2-b:4,3-b′]dipyrrole-3,5(2H)-diyl]]ester, 2,2-dimethylpropanoic acid (1.1 mg) is obtained on evaporation of fr 9-23.
NMR(d₆-DMSO,TMS): δ 1.39(s, 18H); 2.41 (s, 6H); 3.73 (m, 2H); 4.00 (m, 2H); 4.21 (m, 2H); 4.60 (m, 2H); 4.73(m, 2H); 7.09 (s, 2H); 7.25 (m, 4H); 7.42 (m, 2H); 7.80 (bs, 2H); 7.88 (s, 2H); 8.55 (bs, 2H); 10.87 (bs, 2H); 11.54 (bs, 2H).
UV(DMA + MeOH): λmax = 324 nm (ε = 40,000); 294 nm (ε = 40,000).

### Example 15 Preparation of [S-(R*,R*)]-carbonylbis[imino-1H-indole-5,2-diylcarbonyl[1-(chloromethyl)-1,6-dihydro-8-methylbenzo[1,2-b:4,3-b′]dipyrrole-3,5(2H)-diyl]]ester, decanoic acid (Compound 15).

[S-(R*, R*)]-6,6′-[carbonylbis(imino-1H-indole-5,2-dicarbonyl)]bis[8-chloromethyl)-3,6,7,8-tetrahydro-1-methyl-benzo[1,2-b:4,3-b′]dipyrrol-4-ol (10 mg, 0.012 mM) is dissolved in pyridine (0.2 mL) and DMF (0.01 mL). The solution is treated with decanoic anydride (75 mg, 0.23 mM) and the reaction stirred in the dark for 24 hr. The reaction is then quenched with water (0.05 mL) and evaporated to dryness. The residue is washed with water and with toluene each the insolubles are collected by centrifugation. The resultant residue is chromatographed over a silica gel column (1.2 g) eluted with 5%, 6%, 7%, and 10% DMF in toluene. Fractions of 0.3 mL are collected. [S-(R*,R*)]-carbonylbis[imino-1H-indole-5,2-diylcarbonyl[1-(chloromethyl)-1,6-dihydro-8-methylbenzo[1,2-b:4,3-b′]dipyrrole-3,5(2H)-diyl]]ester, decanoic acid (1.3 mg) is obtained on evaporation of fractions 18-60.
NMR(d₆-DMSO,TMS); δ 0.86 (t); 1.24 (m); 1.45 (m); 2.18 (t); 2.40(s); 3.70 (m); 3.98(m); 4.15 (m); 4.59 (m); 4.71 (m); 7.08 (s); 7.27 (m); 7.42 (m); 7.80 (m); 7.88 (s); 8.59 (bs); 11.05 (bs); 11.53 (bs).
UV(DMA +MeOH): λmax = 324 nm (ε = 40,000); 294 nm (ε = 40,000).

### Example 16 Preparation of [S-(R*,R*)]-6,6′-[carbonylbis[(7,8-dihydrobenzo[1,2-b:4,3-b′]dipyrrole-6,2(3H)-diyl)carbonyl]]bis[8-(chloromethyl)-3,6,7,8-tetrahydro-1-methyl-benzo[1,2-b:4,3b′]dipyrrol-4-ol (Compound 16).

### Part A - Preparation of 6,6′-2-carbonylbis [3,6,7,8-tetrahydro-benzo[1,2-b:4,3-b′]dipyrrole-2-carboxylic acid ethyl ester is described in M. A. Warpehoski, V. S. Bradford, Tetrahedron Lett, 1986, 27, 2735.

NMR: δ 1.35(t, 6H) 3.285(t, 4H); 4.123(t, 4H); 4.34(q, 4H); 7.06(s, 2H); 7.22(d, 2H); 7.32(d, 2H); 11.845(s, 2H).
MS(El): m/z 586(M+), 257, 230, 184, 156.
TLC: R_{f} = 0.14 in (25-75) Acetone-hexane.

### Part B - Preparation of 6,6′-carbonylbis[3,6,7,8-tetrahydro-benzo[1,2-b:4,3-b′]dipyrrole-2-carboxylic acid diethyl ester.

6,6′-carbonylbis[3,6,7,8-tetrahydro-benzo[1,2-b:4,3-b′]dipyrrole-2-carboxylic acid ethyl ester (30 mg, 0.13 mM) is dissolve in dry THF (0.5 mL) and the solution treated with 4-dimethylaminopyridine (1 mg). This solution is cooled to -98°C in a liquid nitrogen-frozen methanol bath under nitrogen. Diisopropylethylamine is added (25 µl, 0.14 mM) followed by 1.93 M phosgene in toluene (35 µl, 0.067 mM). The reaction is stirred at about -98°C for 3 hr. It is then stored in the -65°C freezer overnight. The reaction is allowed to stir at room temperature for 90 min. It is diluted with water and ethyl acetate. The ethyl acetate layer is washed with brine, dried and evaporated. The crude residue (47.9 mg) is adsorbed onto silica gel (0.5 g) from distilled THF. The resultant solid is added to the top of a silica column (5 g) and eluted with 5%-15% acetone in toluene. Fractions are of 3 mL are collected. 6,6′-carbonylbis[3,6,7,8-tetrahydrobenzo[1,2-b:4,3-b′-dipyrrole-2-carboxylic acid diethyl ester (5.6 mg) is found in fraction 5-9.

### Part C -

6,6′-carbonylbis[3,6,7,8-tetrahydrobenzo[1,2-b:4,3-b′]dipyrrole-2-carboxylic acid diethyl ester (7 mg, 0.014 mM) is dissolved in pyridine and the solution treated with 1 N NaOH (0.05 mL). The reaction is then heated to 50 C for 6 hr. The reaction is acidified with 1 N HCl (0.2 mL) and 6,6′-carbonylbis[3,6,7,8-tetrahydrobenzo[1,2-b:4,3-b′]dipyrrole-2-carboxylic acid diethyl ester precipitated as a solid is collected by centrifugation and dried under vacuum.

### Part D - Preparation of [S-(R*,R*)]-6,6′-[carbonylbis[(7,8-dihydrobenzo[1,2-b:4,3-b′]dipyrrole-6,2(3H)-diyl)carbonyl]]bis[8-(chloromethyl)-3,6,7,8-tetrahydro-1-methyl-benzo[1,2-b:4,3b′]dipyrrol-4-ol (Compound 16).

(S)-1-(chloromethyl)-1,6-dihydro-5-hydroxy-8-methyl-benzo[1,2-b:4,3-b′]dipyrrole-3(2H)-carboxylic acid 1,1-dimethyl ester [(Boc)CPI phenol chloride] (11 mg, 0.033 mM) is dissolved in ethyl acetate (0.2) and HCl-saturated ethyl acetate (0.7 mL). The mixture is stirred at room temperature under nitrogen for 1 hr. It is evaporated under a nitrogen stream. CH₂Cl₂ is added, and the residual (S)-1-(chloromethyl)-5-hydroxy-8-methyl-1,2,3,6-tetrahydrobenzo[1,2-b:4,3-b′]dipyrrole hydrochloride (CPI phenol chloride hydrochloride salt) is re-evaporated twice under a nitrogen stream. 6,6′-carbonylbis[3,6,7,8-tetrahydrobenzo[1,2-b:4,3-b′]dipyrrole-2-carboxylic acid diethyl ester (6 mg, 0.014mM) is dissolved in DMA (0.3 mL) and added to the CPI phenol chloride hydrochloride salt. Then the mixture is treated with EDC (6.4 mg, 0.034 mM). The reaction is stirred under nitrogen at room temperature 50 min. Additional EDC (6.4 mg, 0.034 mM) is added, and stirring is continued 65 min. The product is precipitated out by the gradual addition of water. The solids (28 mg) are collected by centrifugation and dried under vacuum. The crude product is adsorbed from DMF onto silica gel (0.3 g). It is put on a silica gel column (3 g), eluting with 10, 12, 14, 16 and 20% DMF-toluene. Fractions of 1 mL are collected. [S-(R*,R*)]-6,6′-[carbonylbis[(7,8-dihydrobenzo[1,2-b:4,3-b′]dipyrrole-6,2(3H)-diyl)carbonyl]]bis[8-(chloromethyl)-3,6,7,8-tetrahydro-1-methyl-benzo[1,2-b:4,3-b′]dipyrrol-4-ol (6 mg) is obtained on concentration of fractions 40-62.
NMR: δ 2.36(s, 6H); 3.3(t, 4H); 3.61(m, 2H); 3.92(m, 2H); 4.03(m, 2H); 4.16(t, 4H); 4.55(m, 2H); 4.69(m, 2H); 7.01(s, 2H); 7.05(s, 2H); 7.27(m, 4H); 7.66(m, 2H); 9.79(s, 2H); 10.74(bs, 2H); 11.62(bs, 2H).
UV: λmax - 353 nm (ε=28000); 286 nm(ε=45000).
TLC: R_{f} = 0.29 in (20-80) DMF-toluene.

The starting compounds are known or can be readily prepared by known methods. See M.A. Warpehoski, Tet. Lett., 27, 4103 (1986); W. W. Wierenga, J. Am. Chem. Soc., 103, No. 18, 1981; D.G. Martin, J. Antibiotics 1985, 38, 746; and M.A. Warpehoski, I. Gebhart, R.C. Kelly, W.C. Krueger, L.H. Li, J.P. McGovren, M.D. Prairie, N. Wicnienski and W. Wierenga, J. Med. Chem. 1988, 31, pp. 590-603.

The preparation of CPI(Boc) HCl is described in R. C. Kelly, I. Gebhard, N. Wicnienski, P. A. Aristoff, P. D. Johnson, D. G. Martin, J. Am. Chem. Soc. 1987, 109 6837.

The spirocyclopropylcyclohexadienyl compounds of Formula A and 1-(halomethyl)-1,6-hydro-5-hydroxy-8-methyl-benzo[1,2-b:4,3-b′]dipyrrole-3(2H)-yl 5-ester or urethanes (Formula B) can also be prepared by the procedures and methods disclosed in US-A-4912227 and WO-8804659. See also EP Application 0 154 445 (published 9 November 1985).

All compounds of the subject invention have UV absorption in the range of 200 nm to 380 nm. Thus, novel compounds of the subject invention (Formula I) are useful as UV adsorbents in technical and industrial areas, as follows:
(a) textile materials, for example, wool, silk, cotton, hemp, flax, linen and the like;
(b) natural or synthetic resins.

Depending on the nature of the material to be treated, the requirements with regard to the degree of activity and durability, and other factors, the proportion of the light screening agent to be incorporated in the material may vary within fairly wide limits, for example, from about 0.01% to about 10%, and, advantageously, 0.1% to 2% of the weight of the material which is to be directly protected against the action of UV rays.

The compounds of Formula I are particularly useful as antitumor agents. Examples of compounds of Formula I demonstrate antitumor activity in P388 leukemic mice, and also show significant activity in the L1210 leukemia and B16 melanoma murine test systems. These murine test systems are predictive for clinically useful human antitumor agents (see, for examle, A. Geldin et al, European J. Cancer, Vol. 17, pp 129-142, 1981; J.M. Vendetti, Cancer Treatment Reports, Vol. 67, pp. 767-772, 1983; and J.M. Vendetti et al, Advances in Pharmacology and Chemotherapy, Vol. 20, pp. 1-20, 1984), and, therefore, the compounds of the subject invention (Formula I) will be useful in the control and treatment of susceptible neoplastic (cancer) diseases, including susceptible leukemics, in humans when given, for example, intravenously in doses of 0.001 µg/kg to about 10 mg/kg of body weight per day, the exact dose depending on the age, weight, and condition of the patient, and on the frequency of administration.

The compounds of Formula I are effective when administered intravenously (IV) in fluid solutions by bolus injection or by infusion. The preferred doses are 0.01 microgram/kg to 1000 microgram/kg by bolus injection and 0.0002 to 20 microgram/kg/min by infusion. The exact dose will vary depending on the particular compound as well as the age, weight, route of administration, and physical condition of the patient, and on the frequency of administration.

Illustrative in vivo and in vitro L1210 testing data on the compounds of Formula I are presented in Tables 1 and 2. Table 3 presents data comparing [S-(R*,R*)]-6,6′-[carbonylbis(imino-1H-indole-5,2-dicarbonyl)]bis[8-chloromethyl)-3,6,7,8-tetrahydro-1-methyl-benzo[1,2-b:4,3-b′]dipyrrol-4-ol (Compound 1) with (7bR)-N-[2-[(4,5,8,8a-tetrahydro-7-methyl-4-oxocyclopropa-[C]pyrrolo[3,2-e]indol-2(1H)0yl)carbonyl]-1H-indol-5-yl]-2-benzofurancarboxamide (U-73,975) in the other murine systems where the compounds have been run jointly. Compound 1 is as least as active as the compound U-73,975 in every system and in some such as the subcutaneous L1210 assay it shows superior activity over several dose ranges. Further, Compound 1, like U-73,975, has been found not to cause delayed death.

In vivo L1210 biological data shows [S-(R*,R*)]-6,6′-[carbonylbis(imino-1H-indole-5,2-dicarbonyl)]bis[8-chloromethyl)-3,6,7,8-tetrahydro-1-methyl-benzo[1,2-b:4,3-b′]dipyrrol-4-ol (Compound 1) to be the most active analog being highly curative at 15 µg/kg. The results set forth in Tables 1 and 4 were obtained using standard well known procedures (In Vivo Cancer, Models, NIH Publication No. 84-2635, 1984).

T/C refers to median life span of treated mice divided by median life span of control mice times 100.

The compounds of formula I are useful as antibacterial agents. These compounds are useful to control the proliferation of susceptible microbes in various environments using standard microbiological techniques. Such environments include dental utensils contaminated with S. aureus, and the like, and laboratory benches in a microbiological laboratory which can be cleansed with a formulation containing about 1-10% (w/v) of a compound of formula I.

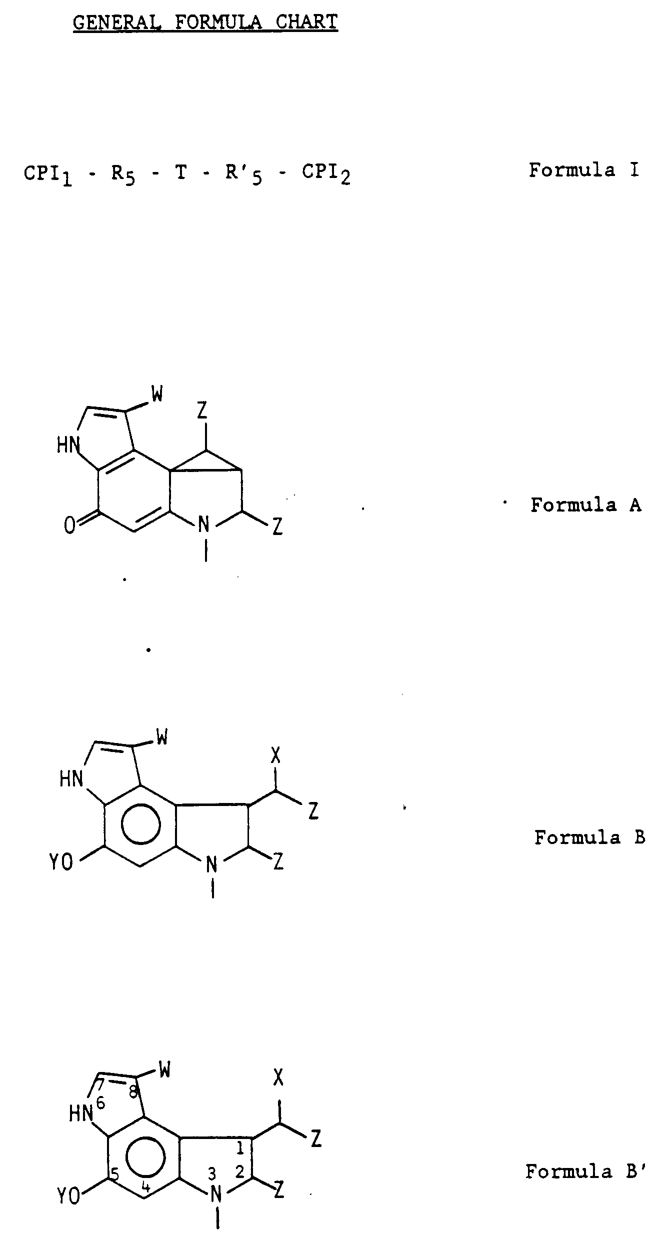

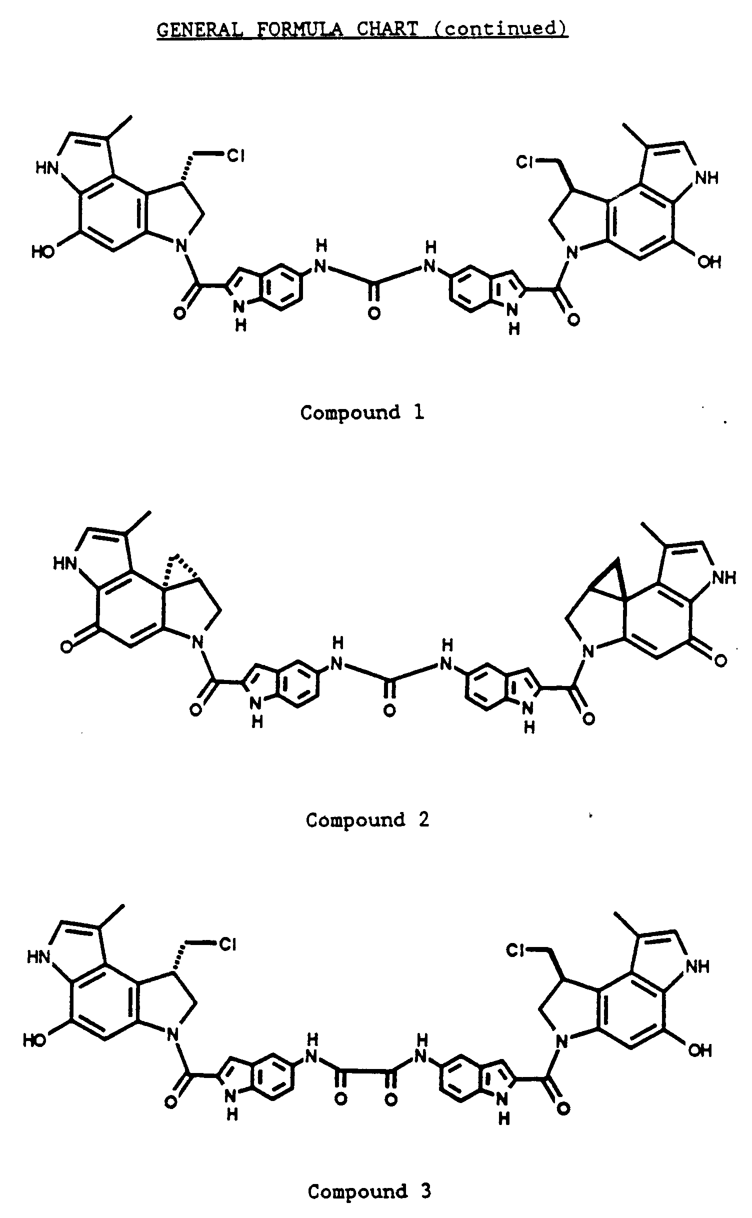

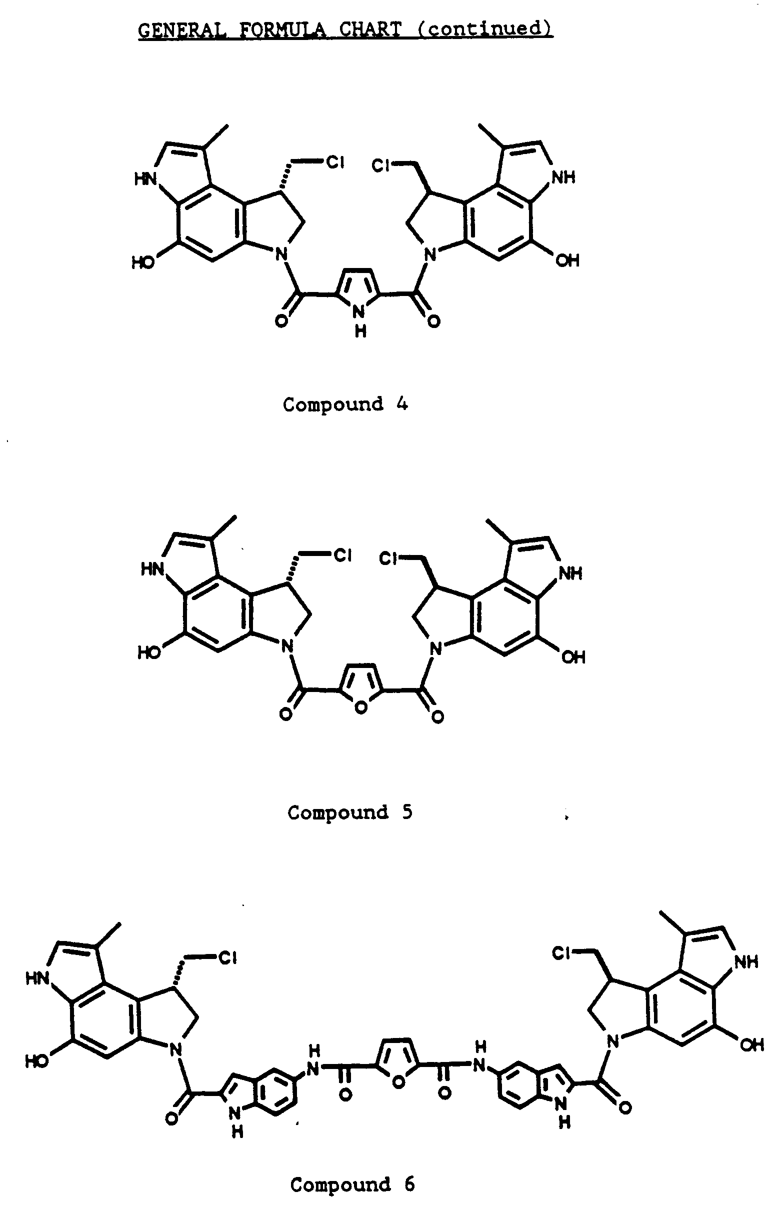

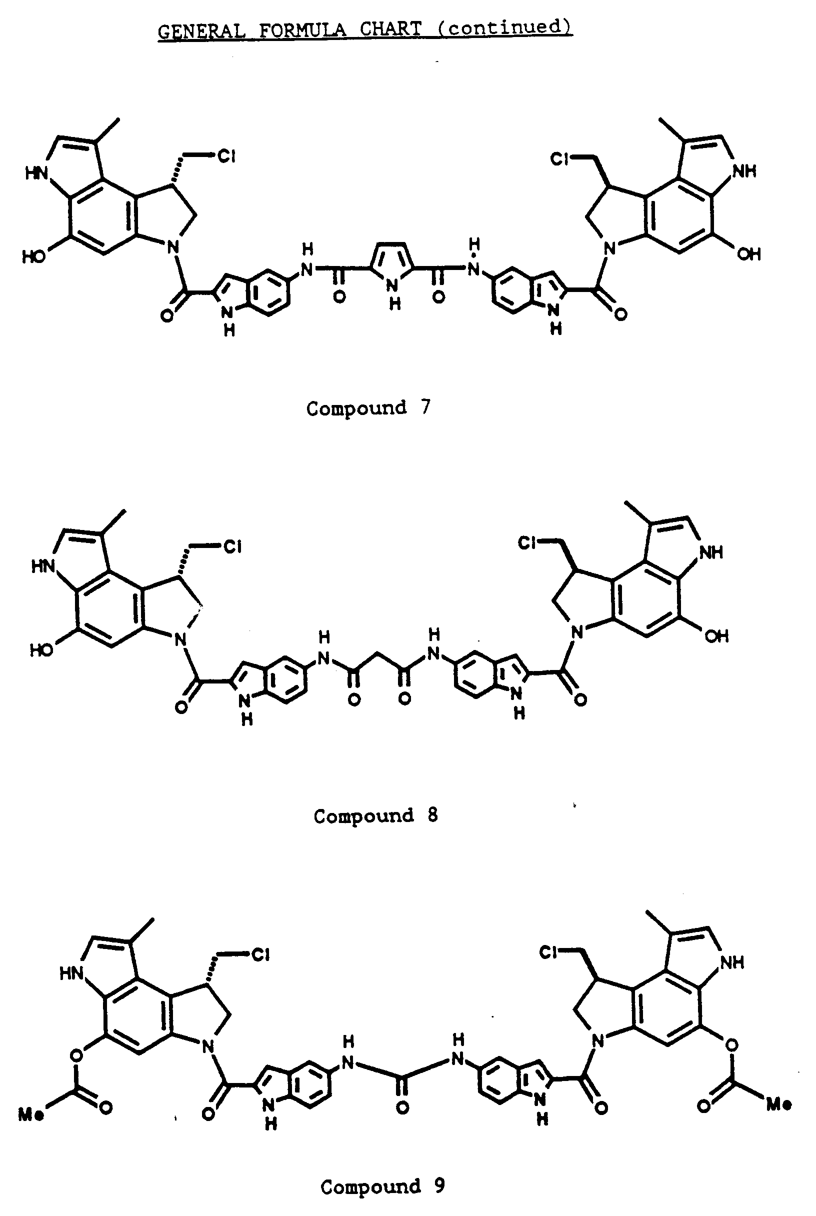

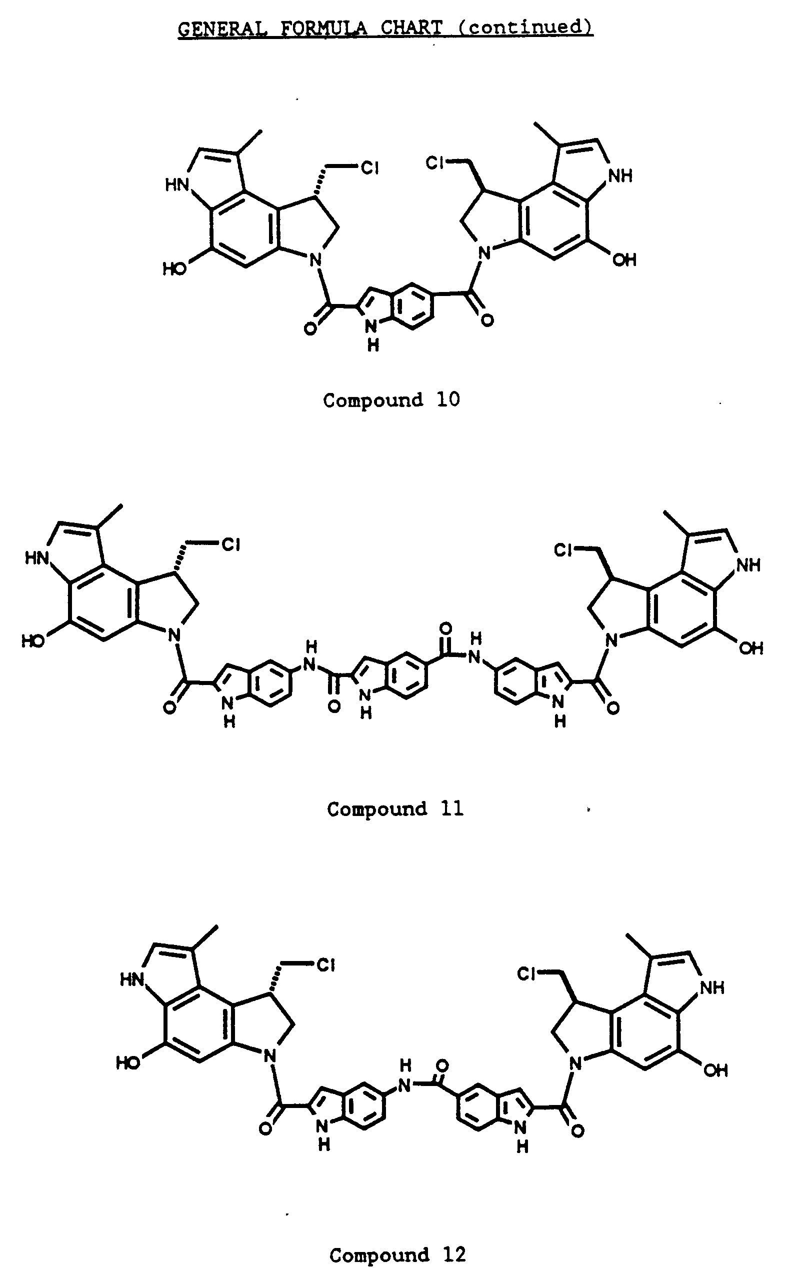

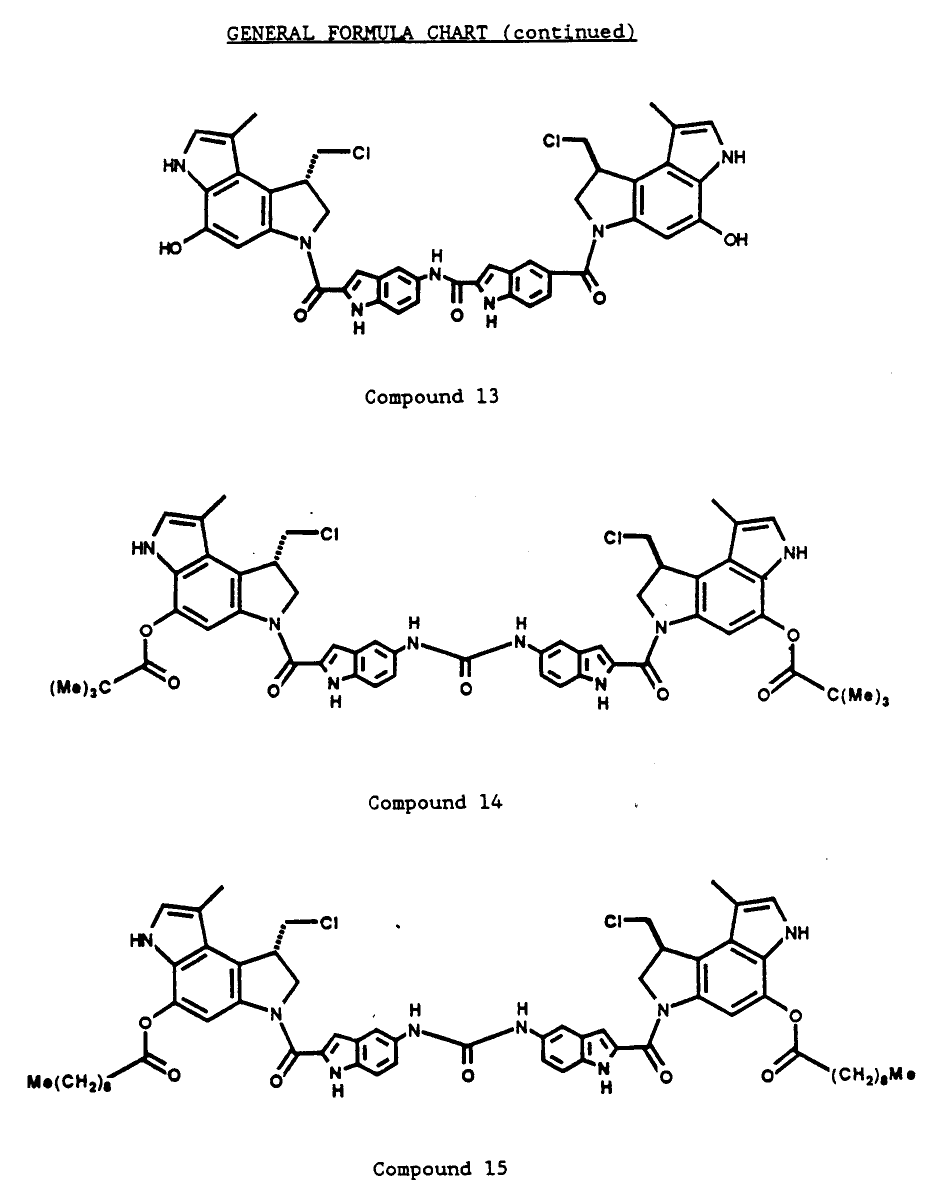

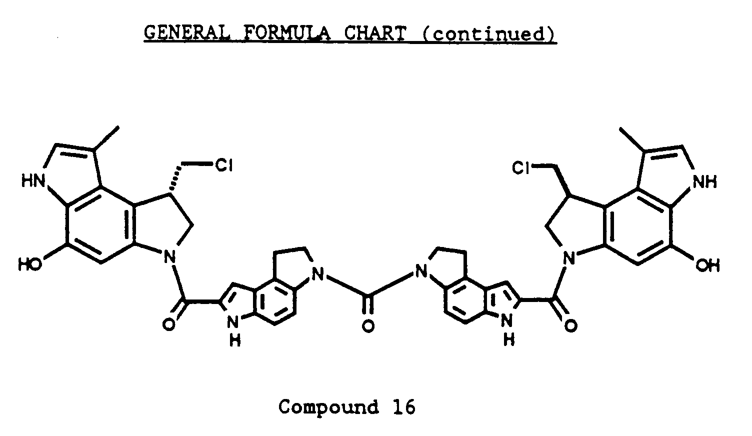

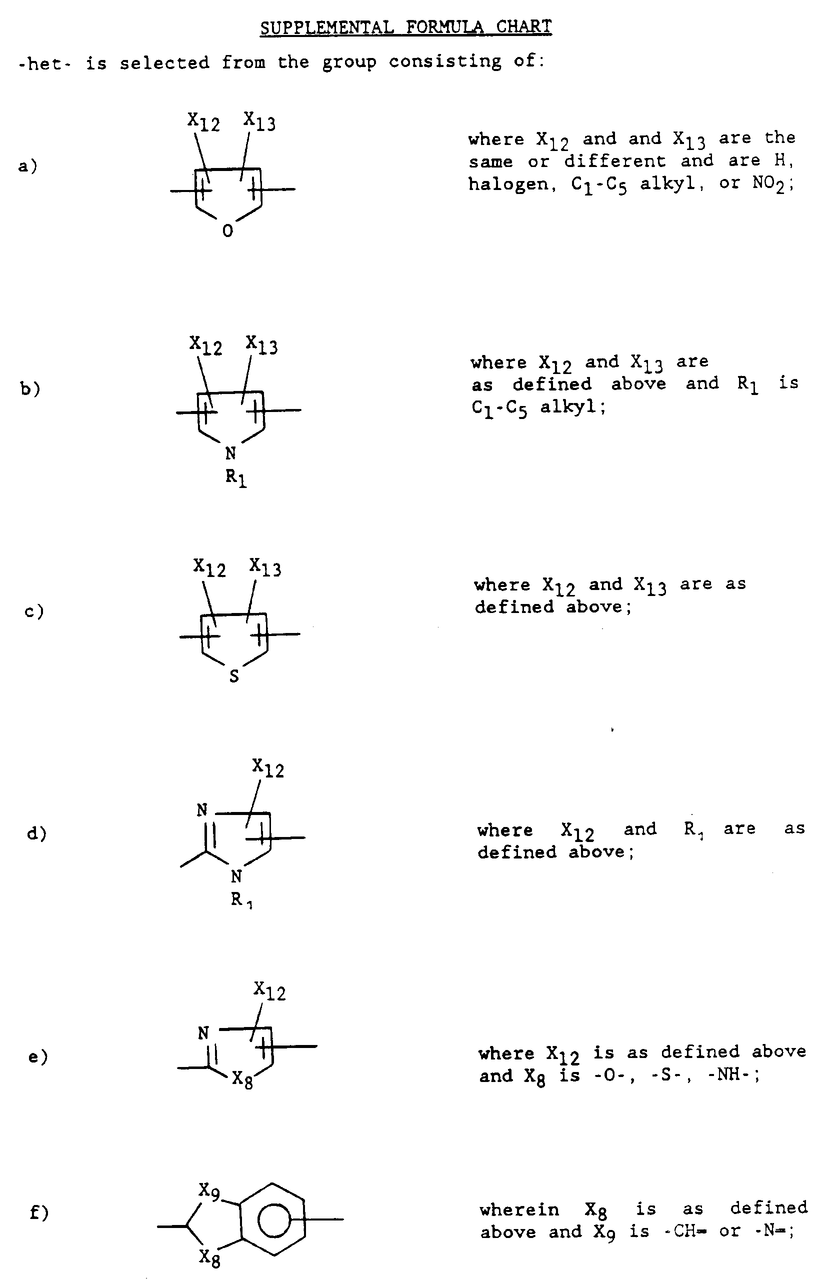

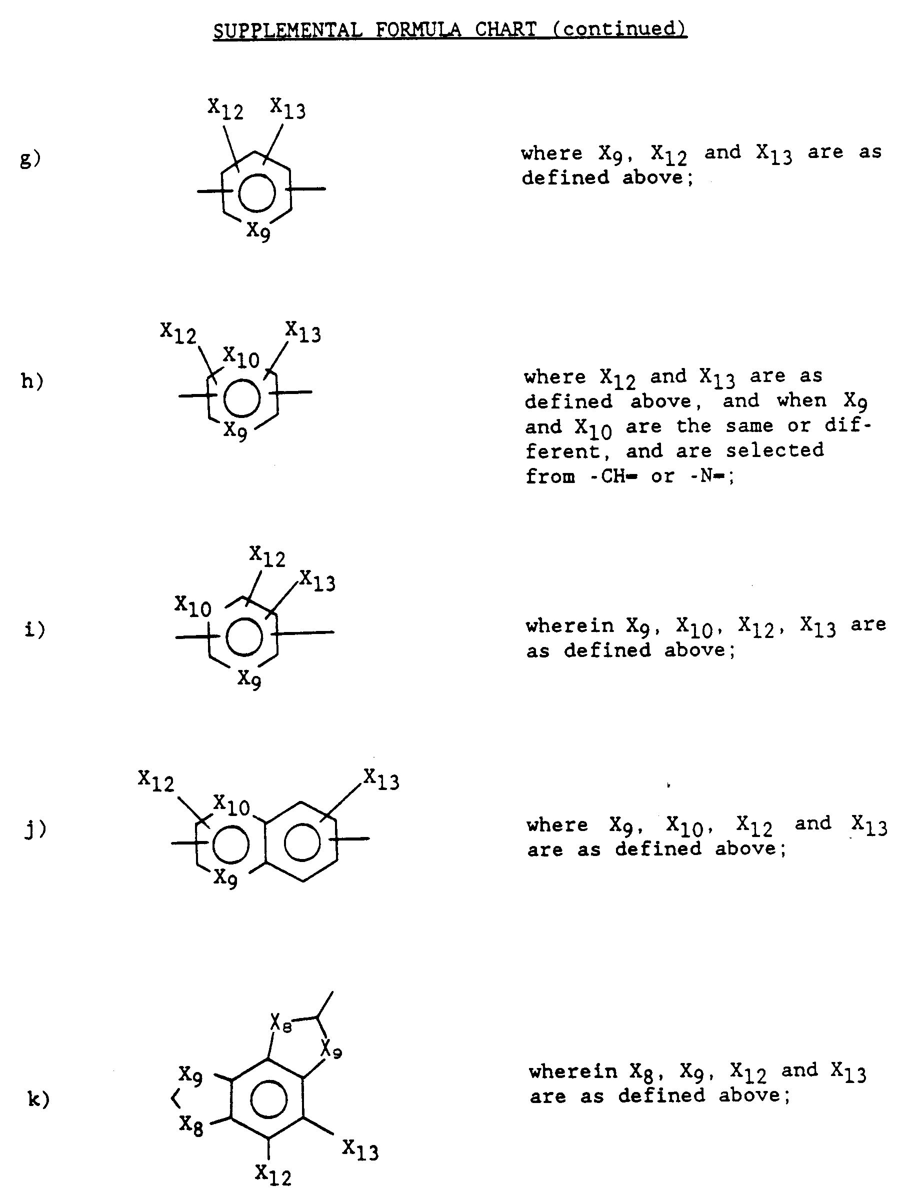

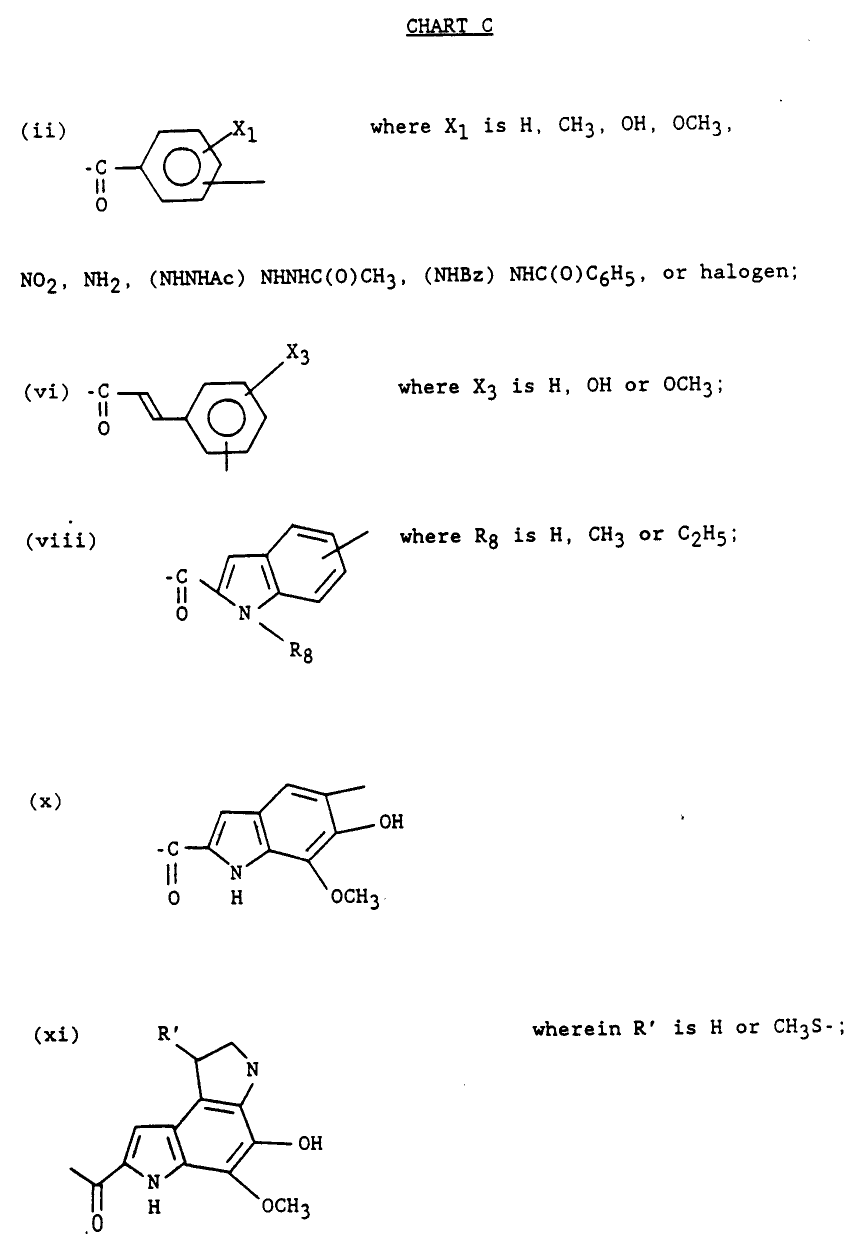

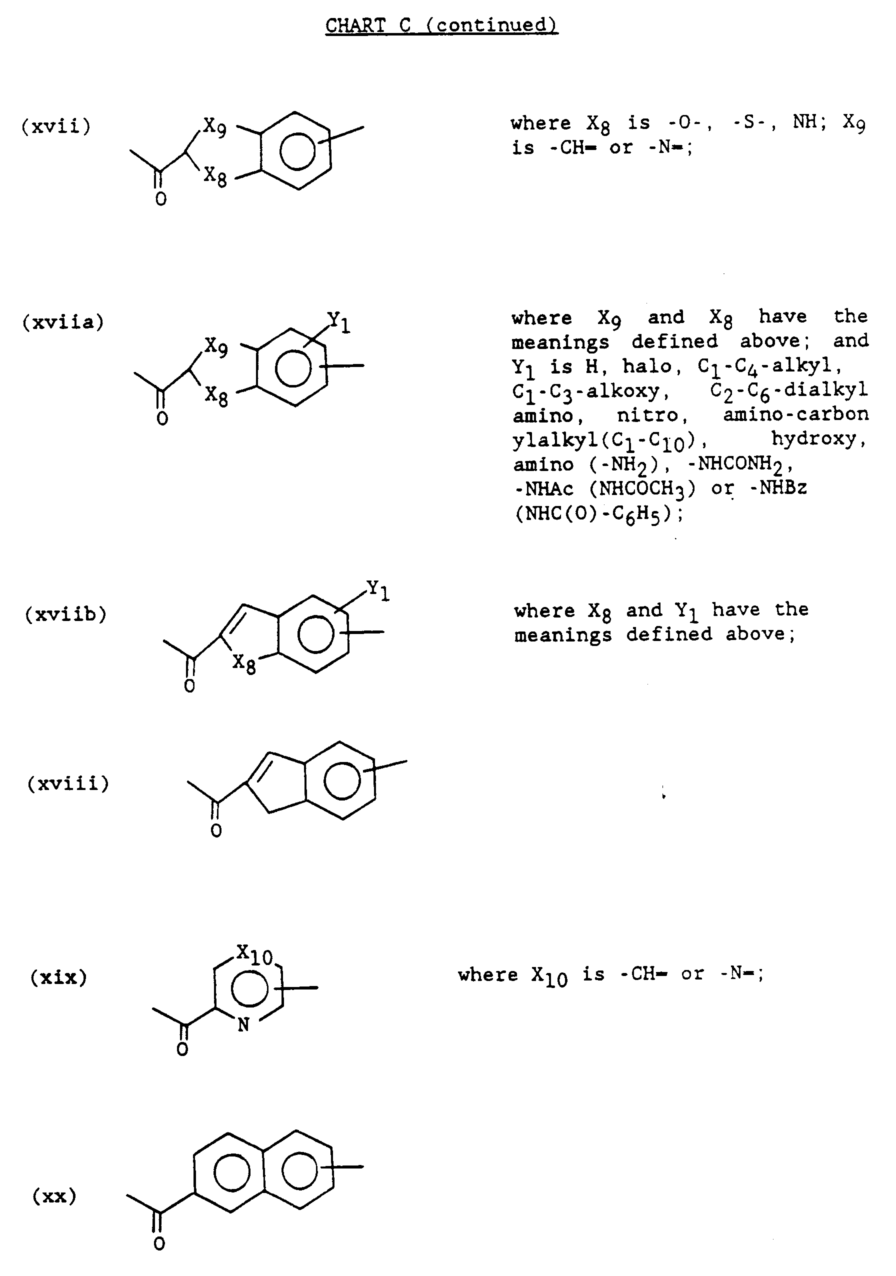

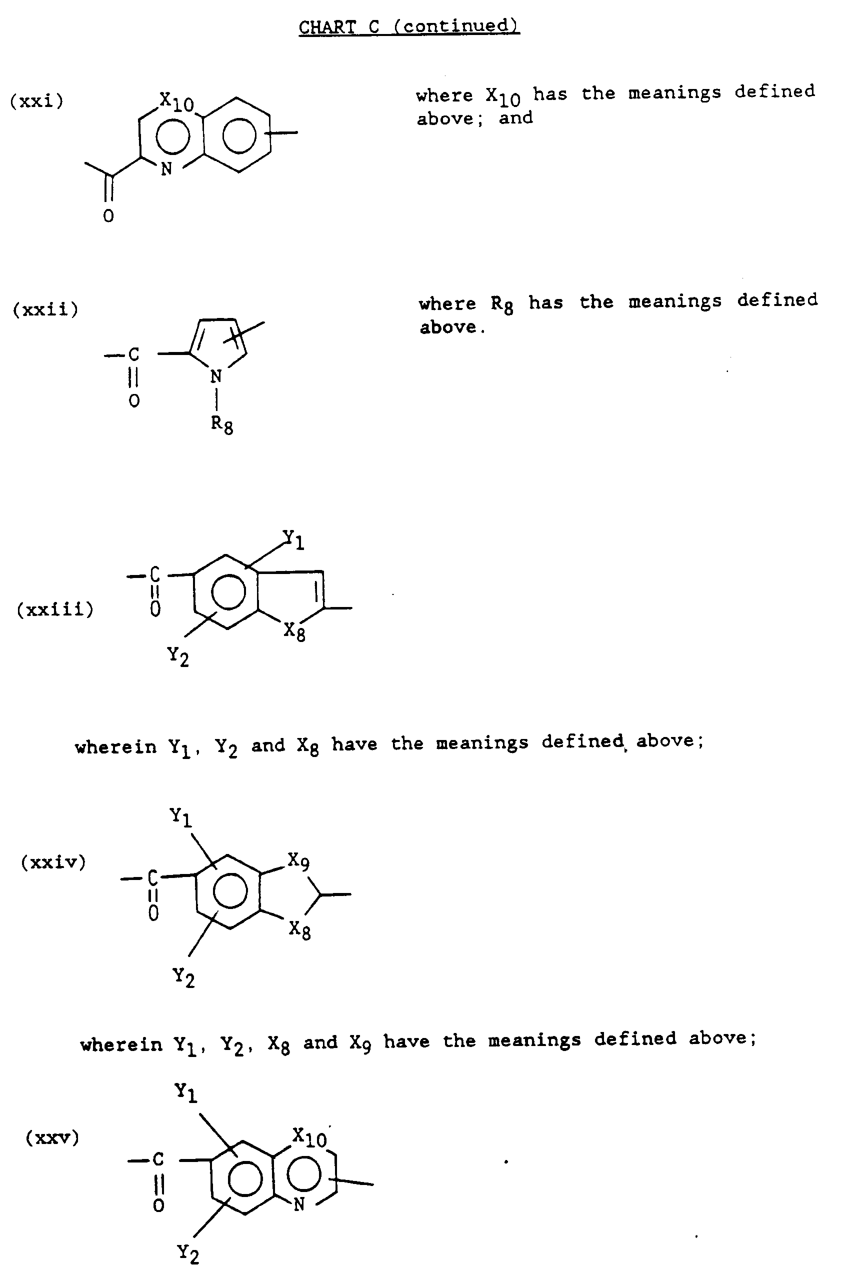

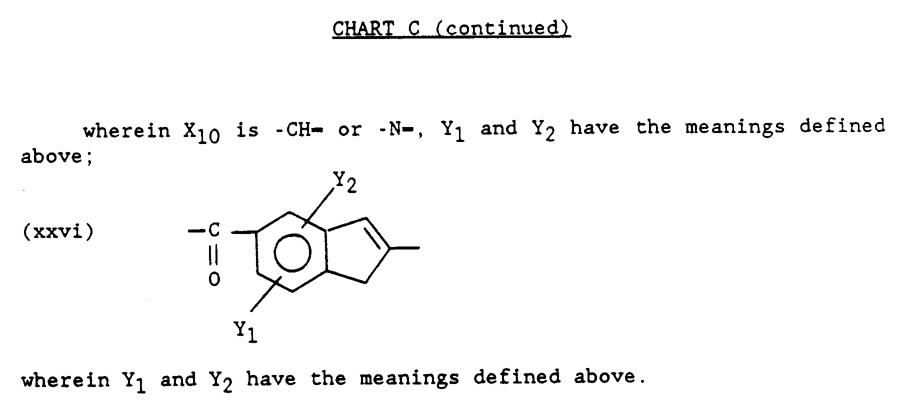

**Table 1**

| L-1210 Leukemia in vivo IV drug vs IP tumor dosed on day 1 L1210 In vivo | | |
|---|---|---|
| Compound # | Dose (µg/kg) | Best T/C^{b} |
| 14 | 20 | 156 |
| 9 | 50 | 165 |
| 10 | 6 | 213 (1 cure) |
| 11 | 13 | 188 |
| 12 | 2 | 181 |
| 13 | 13 | 188 |
| U-73,975 | 100 | 213 |
| 1 | 15 | 4/6 cures |
| 3 | 6 | 106 |
| 8 | 50 | 194 |
| 2 | 10 | 169 |
| 6 | 5 | 178 (1 cure) |
| 7 | 1 | 156 |
| 5 | 600 | 156 |
| 4 | 50 | 156 |
| 15 | 13 | 243 (1 cure) |
| 16 | 12 | 243 (1/6 cure) |

| | | |
|---|---|---|
| ^{a} Male BDF₁ mice | | |
| ^{b} T/C = Treated/Control x 100, where Treated is the median survival time of the treated group and control is the median survival time of the untreated control group. Animals surviving 30 days are considered cured. | | |

**Table 2**

| In vitro Biological Data | |
|---|---|
| Compound # | ID₅₀^{b} |
| 1 | 0.000001 |
| 3 | 0.000009 |
| 8 | 0.000006 |
| 2 | 0.000001 |
| 6 | 0.000012 |
| 7 | 0.000004 |
| 5 | 0.00001 |
| 4 | 0.000004 |
| 15 | 0.000001 |
| 16 | 0.000002 |

| | |
|---|---|
| ^{a} Drugs are tested against L1210 leukemia cells. | |
| ^{b} The concentration of drug in µg/ml which inhibits cell growth 50% is reported. | |

**Table 3**

| | | U-73-975 | Cpd 1 |
|---|---|---|---|
| L-1210 i.p.^{a} | OD^{b} µg/kg | 100 | 10-15 |
| i.v. dose on day 1 | best T/C (cures)^{c} | 213 | (4/6) |
| L-1210 i.p.^{a} | OD µg/kg | 20 | 4 |
| i.p. dose on days 1,5,9 | T/C (cures) | (4/6) | (3/6) |
| L-1210 sc | OD µg/kg | 100 | 10-25 |
| i.p. dose 1 on day 1 | best T/C (cures) | (4/6) | (6/6) |
| B-16 i.p. | OD µg/kg | 100 | 15 |
| i.v. dose on day 1 | T/C | 160 | 167 |
| Lewis Lung i.v. | OD µg/kg | 25 | 4 |
| i.v. dose on days 1,5,9 | T/C | 147 | 168 |
| NO TUMOR | | No Delayed Death | No Delayed Death |

| | | | |
|---|---|---|---|
| ^{a}. i.p. = intraperitoneally and i.v. = intravenously | | | |
| ^{b}. Od = optimum dose | | | |
| ^{c}. In these tests, animals which survive 30 days are considered cured | | | |

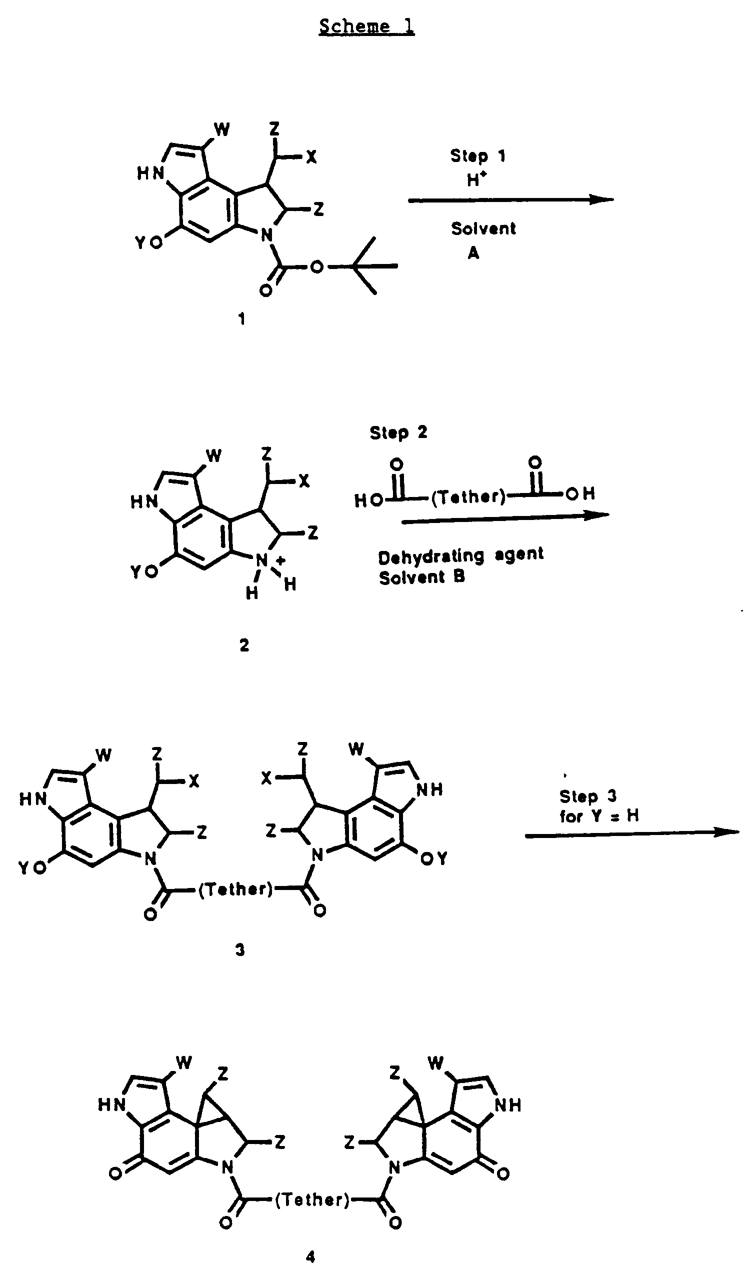

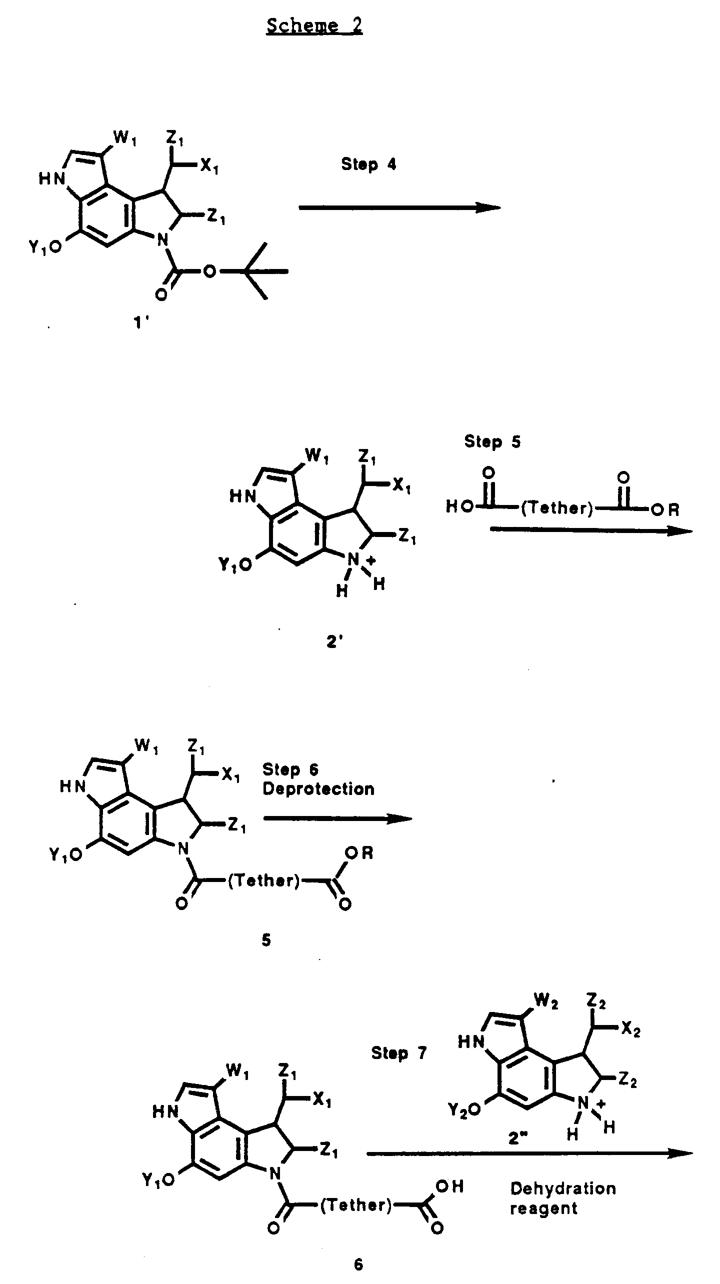

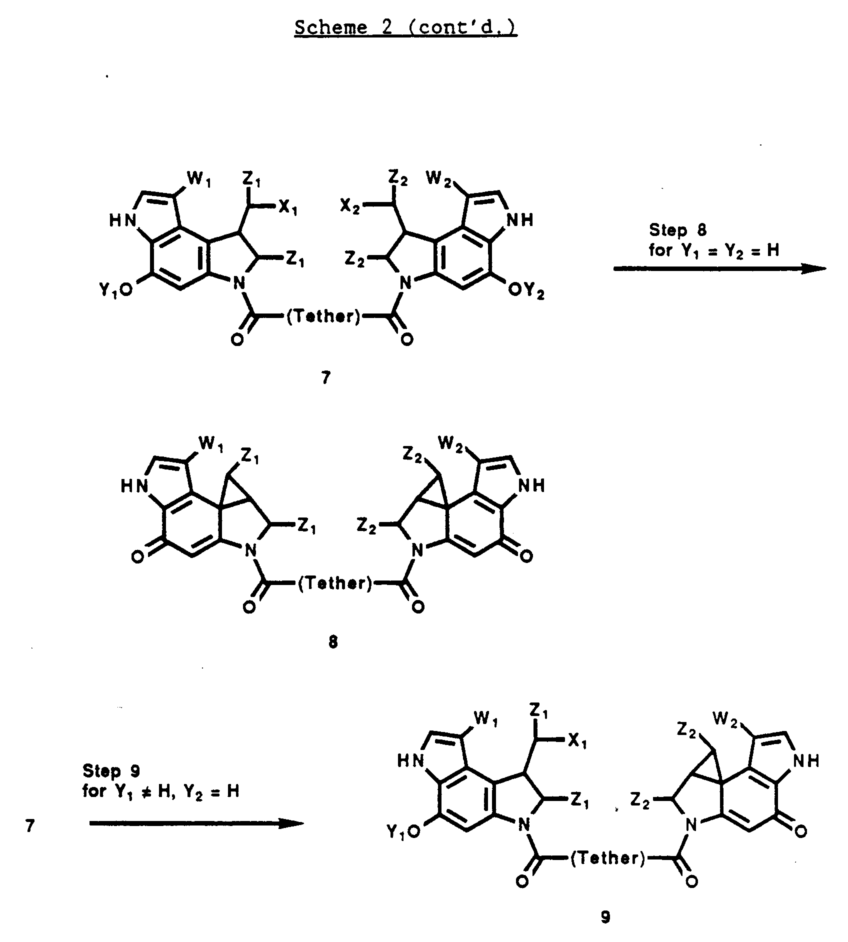

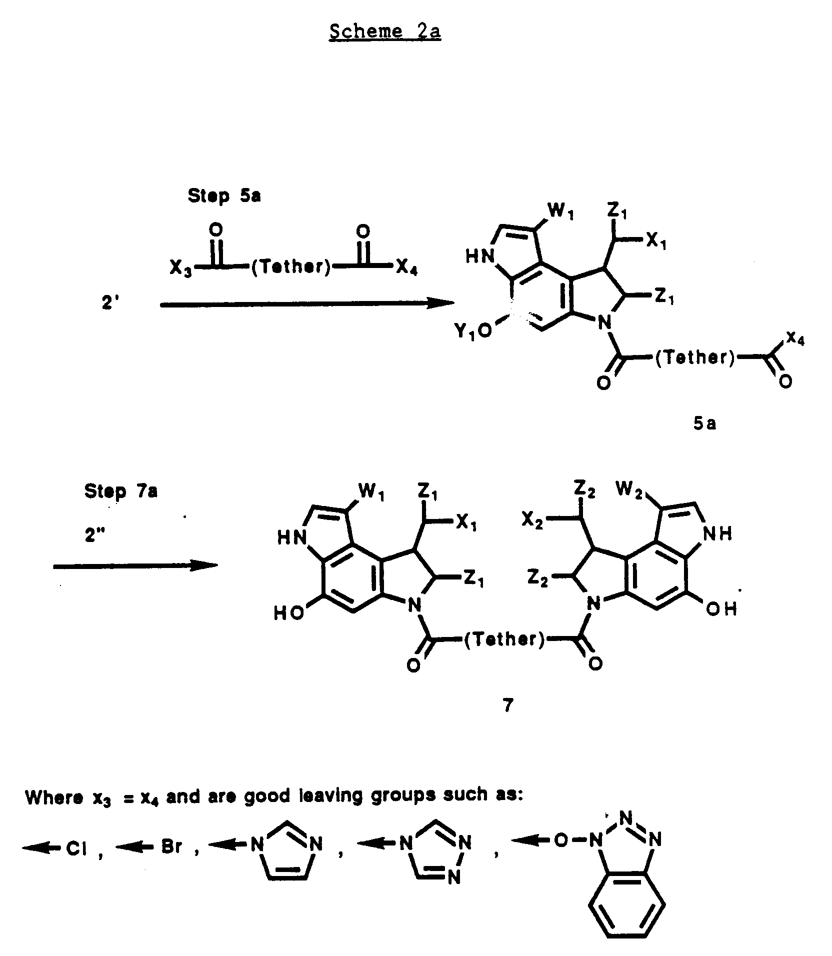

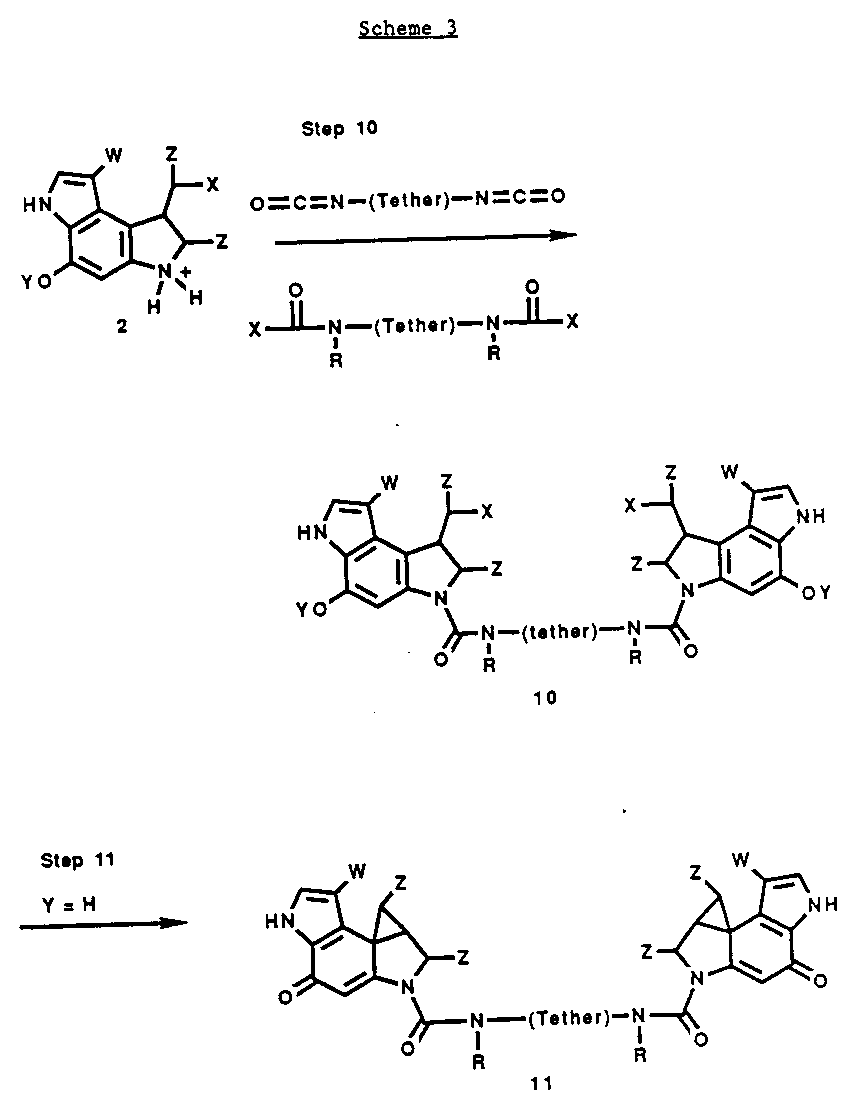

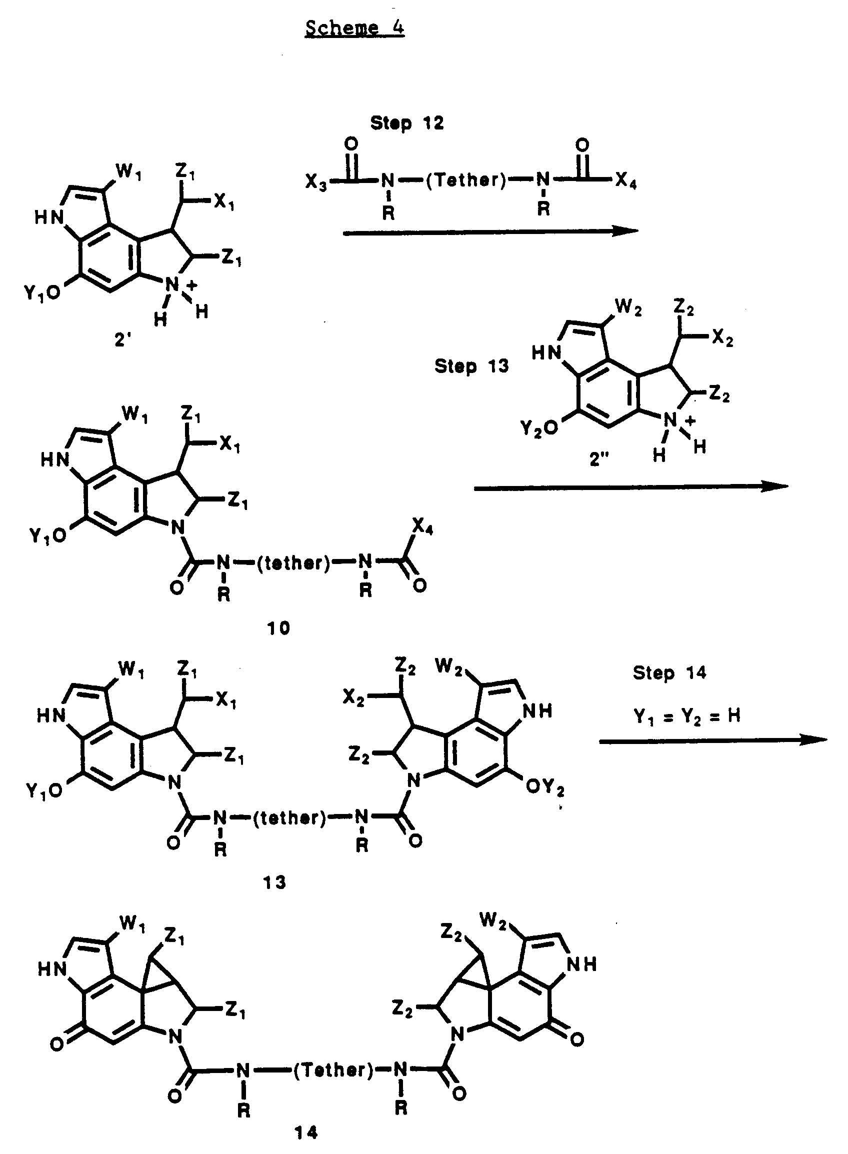

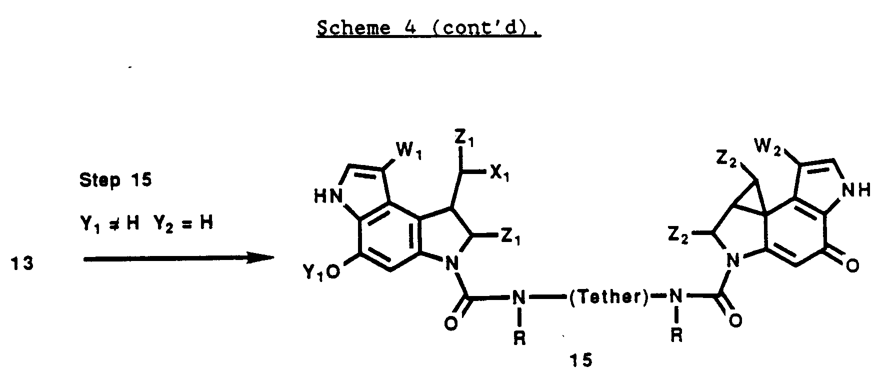

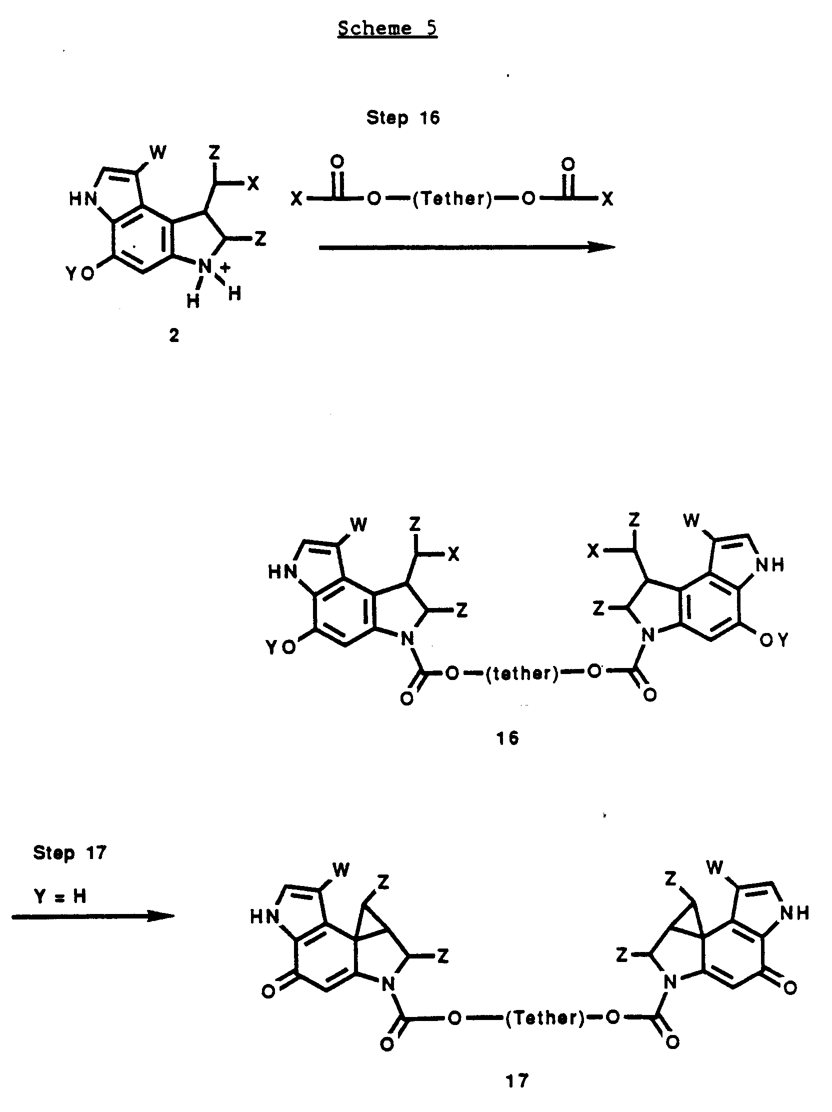

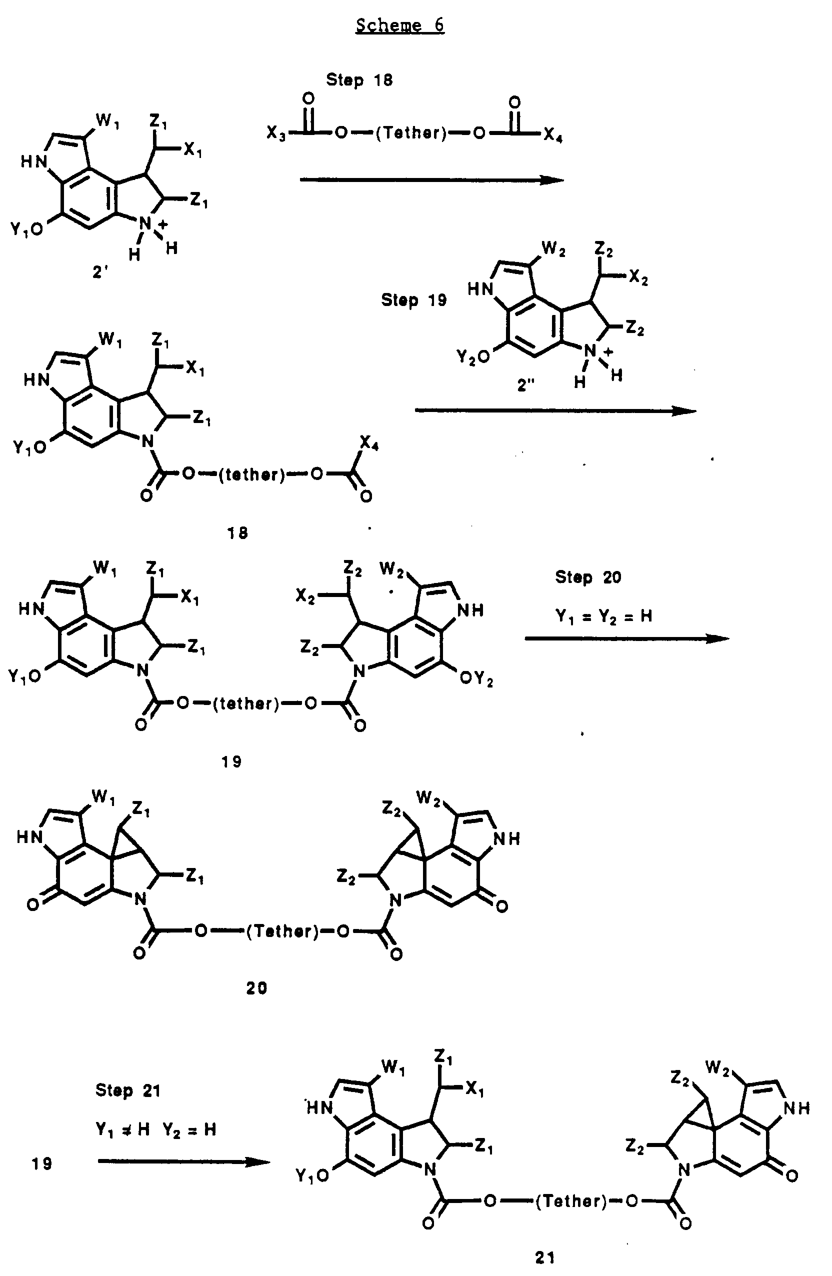

## Claims

1. A compound of formula I
CPI₁ - R₅ - T - R'₅ - CPI₂
wherein CPI₁ and CPI₂ are independently selected from formulae A and B 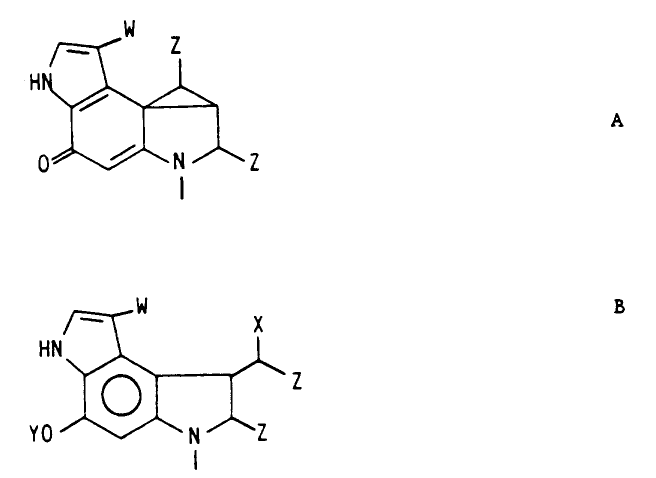
wherein W and Z are independently selected from hydrogen, C₁-C₅ alkyl and phenyl;
X is azido, a halogen atom, cyanate, thiocyanate, isocyanate, thioisocyanate, -PO(OR)₂, -O-PO₂R, -O-PSOR, -O-SOR or -O-SO₂R; and
Y is hydrogen, -C(O)R, -C(S)R, -C(O)OR₁, -S(O)₂R₁, -C(O)NR₂R₃, -C(S)NR₂R₃, or -C(O)NHSO₂R₃;
wherein R is C₁-C₂₀ alkyl; C₂-C₆ alkenyl; C₂-C₆ alkynyl; phenyl optionally substituted with one, 2 or 3 substituents selected from C₁-C₄ alkyl, C₁-C₃ alkoxy, halo, C₁-C₃ alkylthio, CF₃, C₂-C₆ dialkylamino and nitro; or naphthyl optionally substituted with one or 2 substituents as defined above;
R₁ is C₁-C₂₀ alkyl or optionally substituted phenyl as defined above;
R₂ is hydrogen or C₁-C₂₀ alkyl;
R₃ is hydrogen, C₁-C₂₀ alkyl or optionally-substituted phenyl as defined above; and
R₄ is C₁-C₁₀ alkyl, optionally-substituted phenyl as defined above or optionally-substituted naphthyl as defined above;
R₅ and R'₅ are independently selected from a direct bond and a divalent group of any of the formulae: 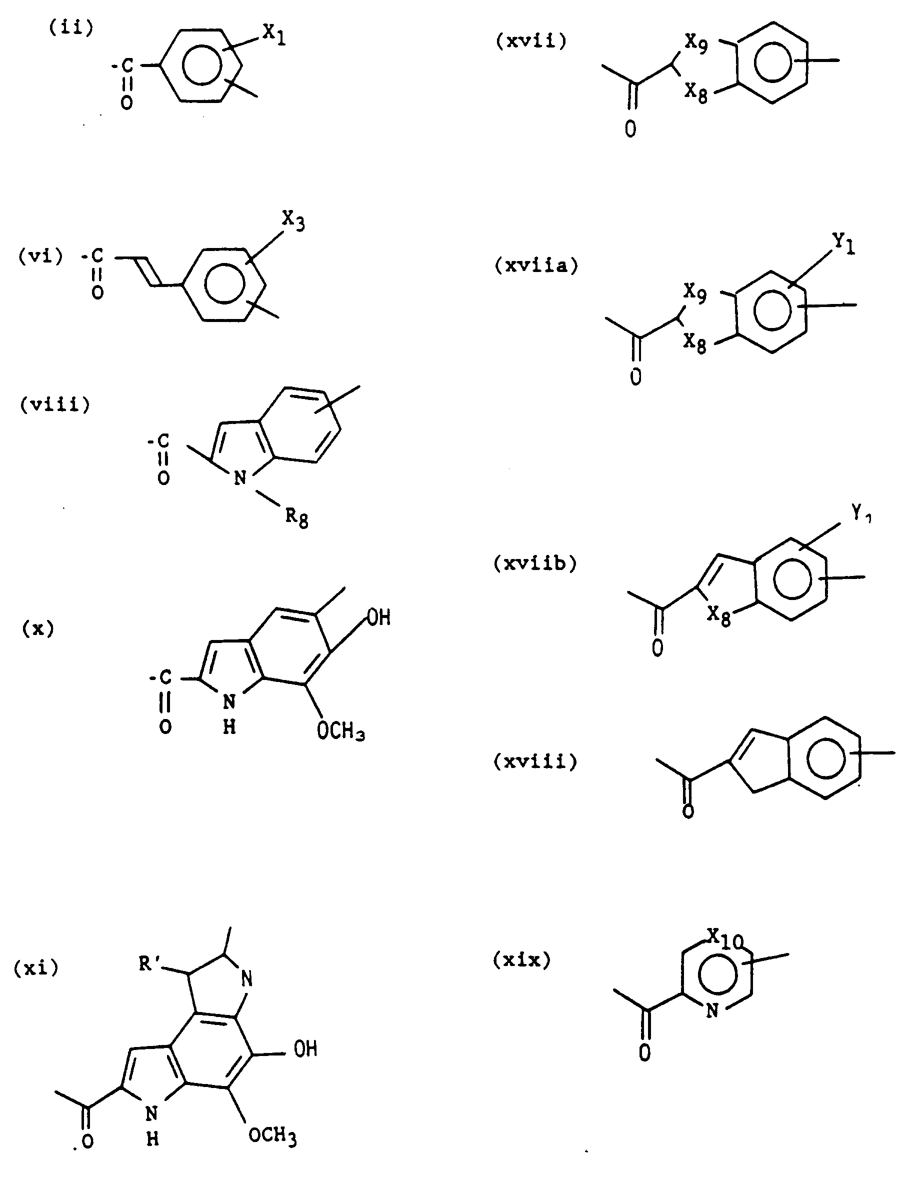 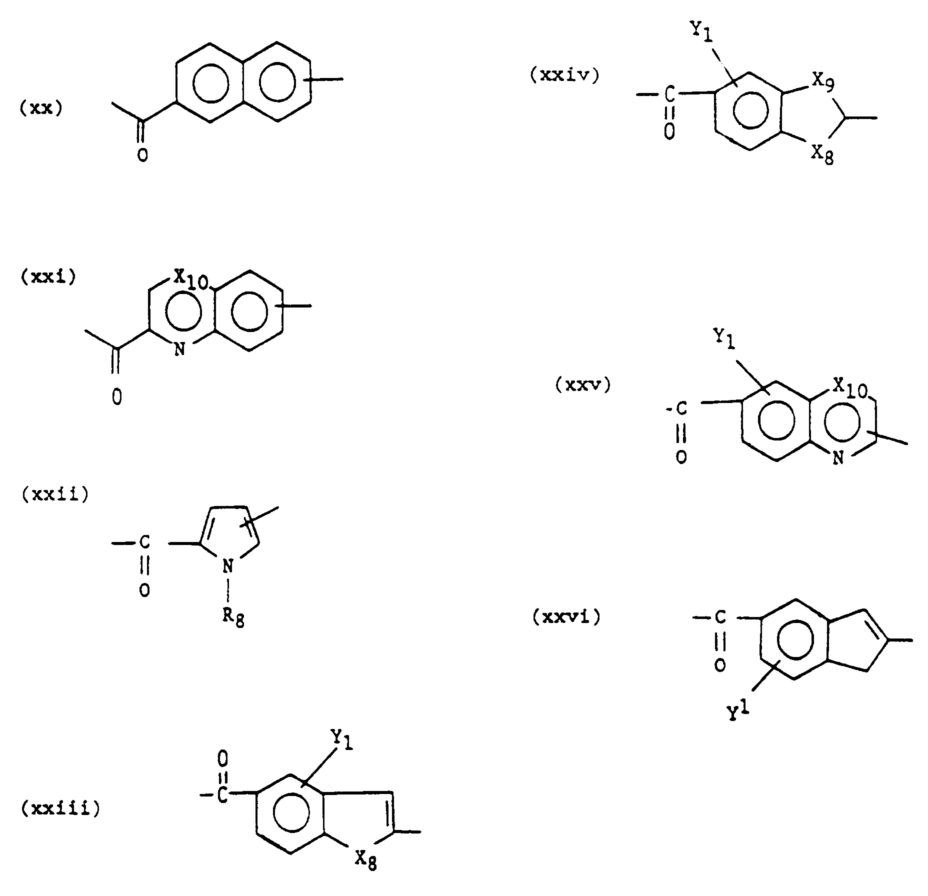
wherein X₁ is H, CH₃, OH, OCH₃, NO₂, NH₂, NHNHC(O)CH₃, NHC(O)C₆H₅, or halogen; X₃ is H, OH or OCH₃; R₈ is H, CH₃ or C₂H₅; R' is H or CH₃S-; X₈ is -O-, -S- or NH; X₉ and X₁₀ are each -CH= or -N=; and Y₁ is H, halo, C₁-C₄ alkyl, C₁-C₃ alkoxy, C₂-C₆ dialkylamino, nitro, aminocarbonylalkyl(C₁-C₁₀), hydroxy, -NH₂, -NHCONH₂, -NHCOCH₃ or -NHC(O)-C₆H₅; and
wherein T is a tether linkage selected from:
a) -NHC(O)-;
b) -C(O)NH-;
c) -C(O)O-;
d) -OC(O)-;
e) -NR₁₃-T'-NR₁₄- where either R₁₃ and R₁₄ are independently hydrogen or C₁-C₈ alkyl, or R₁₃ and R₁₄ are taken together and are -(CH₂)ₙ- where n is 2 or 3; and T' is -CO-, -COCO-, -CO(CH₂)₁₋₅CO-, -C(O)PhC(O)-where Ph is 1,3- or 1,4-phenylene, or -C(O)-het-C(O)-; and
f) -C(O)-het-C(O)-, when R₅ and R'₅ are both a direct bond;
wherein -het- is a fused mono-, di- or tricyclic heteroaryl of 5 to 12 members, containing one, 2 or 3 heteroatoms selected from O, N and S, optionally-substituted with one or 2 substituents as defined above.

2. A compound according to claim 1, wherein W is methyl.

3. A compound according to claim 1 or claim 2, wherein X is halogen.

4. A compound according to any preceding claim, wherein Z is hydrogen.

5. A compound according to claim 1, wherein CPI₁ and CPI₂ are the same or different and are each either 1-(chloromethyl)-1,6-dihydro-8-methyl-5-hydroxybenzo[1,2:4,3-6']dipyrrol-3(2H)-yl or 4,5,8,8a-tetrahydro-7-methyl-4-oxocyclopropa[c]pyrrolo(3,2-e)indol-2(1H)-yl.

6. A compound according to any preceding claim, wherein T is -NHCO-, -CONH-, -NH-CO-NH- or -NH-CO-het-CO-NH-.

7. A compound according to claim 6, wherein T is -NH-CO-NH- or -NH-CO-het-CO-NH- and -het- is selected from pyrrole-2,5-diyl, furan-2,5-diyl, indole-2,5-diyl, benzofuran-2,5-diyl and 3,6-dihydrobenzo[1,2-b:4,3-b']-dipyrrole-2,7-diyl.

8. A compound according to any preceding claim, wherein R₅ and R'₅ are selected from 2-carbonylindol-5-yl, 2-carbonyl-6-hydroxy-7-methoxyindol-5'yl, 2-carbonyl-1,2,3,6-tetrahydrobenzo[1,2-b:4,3-b']dipyrrol-7-yl and 2-carbonyl-4-hydroxy-5-methoxy-1,2,3,6-tetrahydrobenzo[1,2-b:4,3-b']dipyrrol-7-yl.

9. A compound according to claim 1, wherein Y is hydrogen or (C₁-C₁₀ alkyl)carbonyl.

10. A compound according to claim 1, selected from:
[S-(R*,R*)]-6,6'-[carbonylbis(imino-1H-indole-5,2-dicarbonyl)]bis[8-chloromethyl)-3,6,7,8-tetrahydro-1-methyl-benzo[1,2-b:4,3-b']dipyrrol-4-ol (Compound 1);
[7bR-[2(7'bR*,8'aS*),7bR*,8aS*]]-2,2'-[carbonylbis(imino-1H-indole-5,2-dicarbonyl)]bis[1,2,8,8a-tetrahydro-7-methyl-cyclopropa(c)pyrrolo[3,2-e]indol-4(5H)-one (Compound 2);
(R*,S*)-N,N'-bis[2-[[1-(chloromethyl)-1,6-dihydro-5-hydroxy-8-methylbenzo[1,2-b:4,3b']dipyrrol-3(2H)-yl]carbonyl)-1H-indol-5-yl]-ethanediamide (Compound 3);
[S-(R*,R*)]-6,6'-(1H-pyrrole-2,5-diyldicarbonyl)bis[8-(chloromethyl)-3,6,7,8-tetrahydro-1-methyl-benzo[1,2-b:4,3-b'-]dipyrrol-4-ol (Compound 4);
[S-(R*,R*)]-6,6'-(2,5-furandiyldicarbonyl)bis[8-(chloromethyl)-3,6,7,8-tetrahydro-1-methyl-benzo[1,2-b:4,3'b]dipyrrol-4-ol (Compound 5);
[S-(R*,R*]-N,N'-bis[2-[[1-(chloromethyl)-1,6-dihydro-5-hydroxy-8-methylbenzo[1,2-b:4,3-b']dipyrrol-3(2H)-yl]carbonyl]-1H-indol-5-yl]-2,5-furandicarboxamide (Compound 6);
[S-(R*,R*)]-N,N'-bis[2[[1-(chloromethyl)-1,6-dihydro-5-hydroxy-8-methylbenzo[1,2-b:4,3-b']dipyrrol-3(2H)-yl]carbonyl]-1H-indol-5-yl]-1H-pyrrole-2,5-dicarboxamide (Compound 7);
[R-(R*,S*)]-N,N'-bis[2-[[1-(chloromethyl)-1,6-dihydro-5-hydroxy-8-methylbenzo[1,2-b:4,3-b']dipyrrol-3(2H)-yl]carbonyl]-1H-indol-5-yl]-propanediamide (Compound 8);
[S-(R*,R*)]-6,6'-[carbonylbis(imino-1H-indole-5,2-diylcarbonyl)]bis[8-(chloromethyl)-3,6,7,8-tetrahydro-1-methyl-benzo[1,2-b:4,3-b']dipyrrol-4-ol diacetate (Compound 9);
[S-(R*,R*)]-6,6'-(1H-indole-2,5-diyldicarbonyl)bis[8-(chloromethyl)-3,6,7,8-tetrahydro-1-methyl-benzo[1,2-b:4,3-b']dipyrrol-4-ol (Compound 10);
[S-(R*,R*)]-N,N'-bis[2-[[1-(chloromethyl)-1,6-dihydro-5-hydroxy-8-methylbenzo [1,2-b:4,3-b']dipyrrol-3(2H)-yl]carbonyl]-1H-indol-5-yl]-1H-indole-2,5-dicarboxamide (Compound 11);
[S-(R*,R*)]-2-[[1-(chloromethyl)-1,6-dihydro-5-hydroxy-8-methylbenzo[1,2-b:4,3-b']dipyrrol-3(2H)-yl]carbonyl]-N-[3-[[1-(chloromethyl)-1,6-dihydro-5-hydroxy-8-methylbenzo[1,2-b:4,3-b']dipyrrol-3(2H)-yl]carbonyl]-1H-indol-5-yl]-1H-indole-5-carboxamide (Compound 12);
[S-(R*,R*)]-5-[[1-(chloromethyl)-1,6-dihydro-5-hydroxy-8-methylbenzo[1,2-b:4,3-b']dipyrrol-3(2H)-yl]-carbonyl)-N-[2-[[1-(chloro methyl)-1,6-dihydro-5-hydroxy-8-methylbenzo[1,2-b:3-b']dipyrrol-3(2H)-yl]carbonyl)-1H-indol-5-yl]-1H-indole-2-carboxamide (Compound 13);
[S-(R*R*)]-carbonylbis[imino-1H-indole-5,2-diylcarbonyl[1-(chloromethyl)-1,6-dihydro-8-methylbenzo[1,2-b:4,3-b']dipyrrole-3,5(2H)-diyl]]ester, 2,2-dimethylpropanoic acid (Compound 14); and
[S-(R*,R*)]-carbonylbis[imino-1H-indole-5,2-diylcarbonyl[1-(chloromethyl)-1,6-dihydro-8-methylbenzo[1,2-b:4,3-b']dipyrrole 3,5(2H)-diyl]]ester, decanoic acid (Compound 15).

11. [S-(R*,R*)]-6,6'-[carbonylbis[(7,8-dihydrobenzo[1,2-b:4,3-b']dipyrrole-6,2(3H)-diyl)carbonyl]]bis[8-(chloromethyl)-3,6,7,8-tetrahydro-1-methyl-benzo[1,2-b:4,3b']dipyrrol-4-ol (Compound 16).

12. A process for preparing a compound according to any preceding claim, which comprises reacting the appropriate analogue of formula B with a biscarboxylic acid, bisisocyanate, bisaminocarboxylate or oxycarboxylate reagent, and then with the other appropriate analogue of formula B.

## Patentansprüche

1. Verbindung der Formel I
CPI₁ - R₅ - T - R'₅ - CPI₂
worin CPI₁ und CPI₂ unabhängig voneinander aus den Formeln A und B 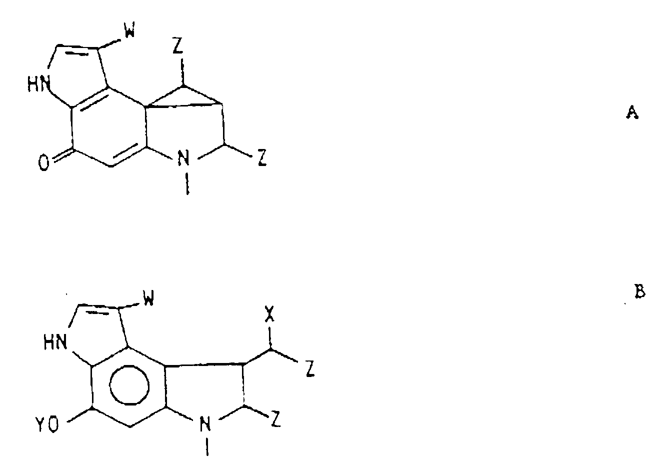 ausgewählt sind,
worin W und Z unabhängig voneinander aus Wasserstoff, C₁ -C₅-Alkyl und Phenyl ausgewählt sind, und
worin bedeuten:
X Azido, ein Halogenatom, Cyanat, Thiocyanat, Isocyanat, Thioisocyanat, -PO(OR)₂, -O-PO₂R, -O-PSOR, -O-SOR oder -O-SO₂R, und
Y Wasserstoff, -C(C)R, -C(S)R, -C(C)OR₁, -S(O)₂R₁, -C(O)NR₂R₃, -C(S)NR₂R₃ oder -C(O)NHSO₂R₃;
R C₁-C₂₀-Alkyl, C₂-C₆-Alkenyl, C₂-C₆-Alkinyl, Phenyl, gegebenenfalls 1-, 2- oder 3-fach substituiert durch C₁-C₄-Alkyl, C₁-C₃-Alkoxy, Halogen, C₁-C₃ -Alkylthio, CF₃, C₂-C₆-Dialkylamino und Nitro, oder Naphthyl, gegebenenfalls 1- oder 2-fach substituiert durch einen der zuvor genannten Substituenten;
R₁ C₁-C₂₀-Alkyl oder gegebenenfalls gemäß obiger Definition substituiertes Phenyl;
R₂ Wasserstoff oder C₁-C₂₀-Alkyl;
R₃ Wasserstoff, C₁-C₂₀-Alkyl oder gegebenenfalls gemäß obiger Definition substituiertes Phenyl;
R₄ C₁-C₁₀-Alkyl, gegebenenfalls gemäß obiger Definition substituiertes Phenyl oder gegebenenfalls gemäß obiger Definition substituiertes Naphthyl, und
R₅ und R'₅ unabhängig voneinander eine direkte Bindung oder eine zweiwertige Gruppe irgendeiner der folgenden Formeln: 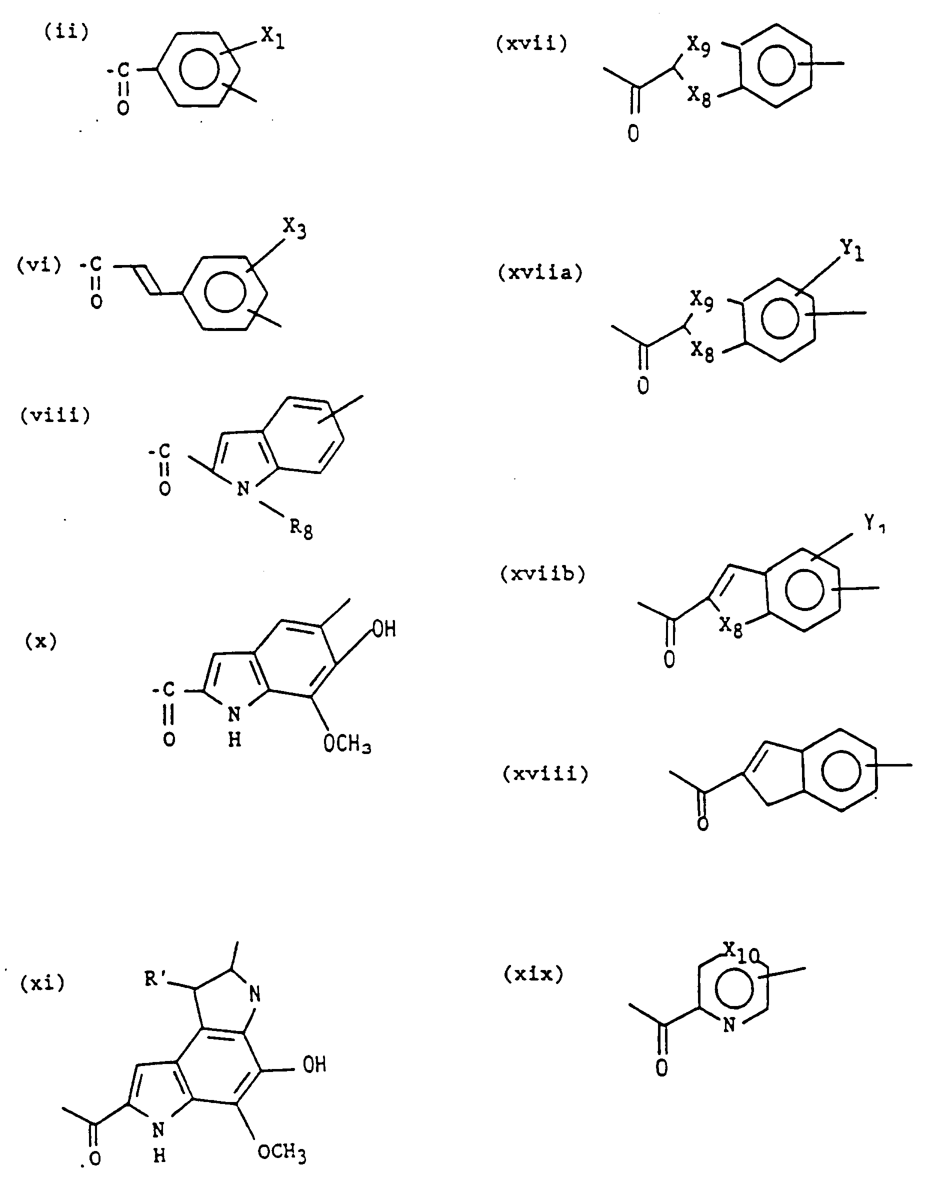 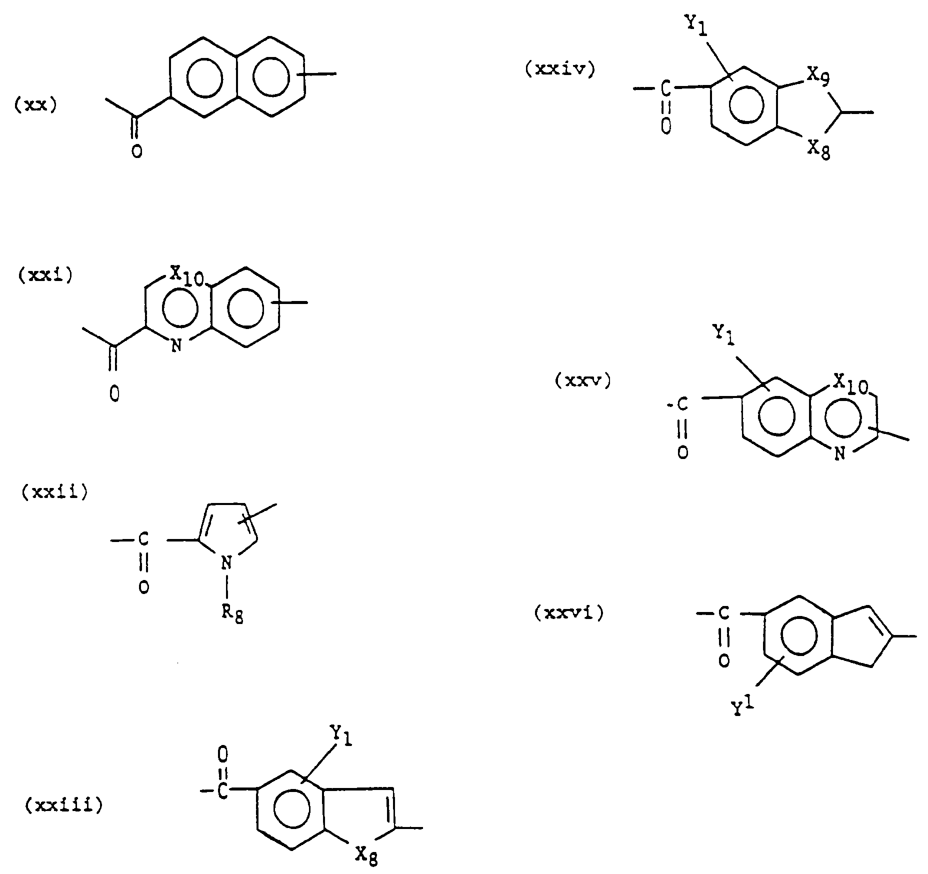
worin X₁ = H, CH₃, OH, OCH₃, NO₂, NH₂, NHNHC(O)CH₃, NHC(O)C₆H₅ oder Halogen; X₃ = H, OH oder OCH₃; R₈ = H, CH₃ oder C₂H₅; R' = H oder CH₃S-; X₈ = -O-, -S- oder NH; X₉ und X₁₀ jeweils = -CH= oder -N=, und Y₁ = H, Halogen, C₁-C₄-Alkyl, C₁-C₃-Alkoxy, C₂-C₆-Dialkylamino, Nitro, Aminocarbonylalkyl (C₁-C₁₀), Hydroxy, -NH₂, -NHCONH₂, -NHCOCH₃ oder -NHC(O)-C₆H₅, und
wobei T für eine Tether-Bindung, ausgewählt aus:
a) -NHC(O)-;
b) -C(O)NH-;
c) -C(O)O-;
d) -OC(O)-;
e) -NR₁₃-T'-NR₁₄- mit R₁₃ und R₁₄ jeweils unabhängig voneinander jeweils gleich Wasserstoff oder C₁-C₈-Alkyl oder R₁₃ und R₁₄ zusammen gleich -(CH₂)ₙ- (n = 2 oder 3); und T' gleich -CO-, -COCO-, -CO(CH₂)₁₋₅CO-, -C(O)PhC(O)-(Ph = 1,3- oder 1,4-Phenylen) oder -C(O)-het-C(O)-; und
f) -C(O)-het-C(O)- im Falle, daß R₅ und R'₅ beide einer direkten Bindung entsprechen,
mit -het- gleich einem kondensierten mono-, di- oder tricyclischen, 5- bis 12-gliedrigen Heteroaryl(rest)
mit 1, 2, oder 3 Heteroatom(en), ausgewählt aus O, N und S, gegebenenfalls 1- oder 2-fach gemäß obiger Definition substituiert,
steht.

2. Verbindung nach Anspruch 1, wobei W für Methyl steht.

3. Verbindung nach Anspruch 1 oder 2, wobei X für Halogen steht.

4. Verbindung nach einem der vorhergehenden Ansprüche, wobei Z für Wasserstoff steht.

5. Verbindung nach Anspruch 1, wobei CPI₁ und CPI₂, die gleich oder verschieden sind, jeweils 1-(Chlormethyl)-1,6-dihydro-8-methyl-5-hydroxybenzo[1,2:4,3-6']dipyrrol-3(2H)-yl oder 4,5,8,8a-Tetrahydro-7-methyl-4-oxocyclopropa[c]pyrrolo(3,2-e)indol-2-(1H)-yl bedeuten.

6. Verbindung nach einem der vorhergehenden Ansprüche, wobei T für -NHCO-, -CONH-, -NH-CO-NH- oder -NH-CO-het-CO-NH- steht.

7. Verbindung nach Anspruch 6, wobei T für -NH-CO-NH- oder -NH-CO-het-CO-NH- steht und -het- aus Pyrrol-2,5-diyl, Furan-2,5-diyl, Indol-2,5-diyl, Benzofuran-2,5-diyl und 3,6-Dihydrobenzo[1,2-b:4,3-b']-dipyrrol-2,7-diyl ausgewählt ist.

8. Verbindung nach einem der vorhergehenden Ansprüche, wobei R₅ und R'₅ aus 2-Carbonylindol-5-yl, 2-Carbonyl-6-hydroxy-7-methoxyindol-5-yl, 2-Carbonyl-1,2,3,6-tetrahydrobenzo[1,2-b:4,3-b']dipyrrol-7-yl und 2-Carbonyl-4-hydroxy-5-methoxy-1,2,3,6-tetrahydrobenzo[1,2-b:4,3-b']dipyrrol-7-yl ausgewählt sind.

9. Verbindung nach Anspruch 1, wobei Y für Wasserstoff oder (C₁-C₁₀-Alkyl)carbonyl steht.

10. Verbindung nach Anspruch 1, ausgewählt aus:
[S-(R*,R*)]-6,6'-[Carbonylbis(imino-1H-indol-5,2-dicarbonyl)]-bis[8-chlormethyl)-3,6,7,8-tetrahydro-1-methylbenzo[1,2-b:4,3-b']dipyrrol-4-ol (Verbindung 1);
[7bR-[2(7'bR*,8'aS*),7bR*,8aS*]]-2,2'-[Carbonylbis(imino-1H-indol-5,2-dicarbonyl)]bis[1,2,8,8a-tetrahydro-7-methyl-cyclopropa(c)pyrrolo[3,2-e]indol-4-(5H)-on (Verbindung 2);
(R*,S*)-N,N'-Bis[2-[[1-(chlormethyl)-1,6-dihydro-5-hydroxy-8-methylbenzo[1,2-b:4,3-b']dipyrrol-3(2H)-yl]carbonyl]-1H-indol-5-yl]ethandiamid (Verbindung 3);
[S-(R*,R*)]-6,6'-(1H-Pyrrol-2,5-diyldicarbonyl)bis[8-(chlormethyl)-3,6,7,8-tetrahydro-1-methyl-benzo[1,2-b:4,3-b'-]dipyrrol-4-ol (Verbindung 4);
[S-(R*,R*)]-6,6'-(2,5-Furandiyldicarbonyl)bis[8-(chlormethyl)-3,6,7,8-tetrahydro-1-methyl-benzo[1,2-b:4,3-b'-]dipyrrol-4-ol (Verbindung 5);
[S-(R*,R*)]-N,N'-Bis[2-[[1-(chlormethyl)-1,6-dihydro-5-hydroxy-8-methylbenzo[1,2-b:4,3-b'-]dipyrrol-3(2H)-yl]-carbonyl]-1H-indol-5-yl-]-2,5-furandicarboxamid (Verbindung 6);
[S-(R*,R*)]-N,N'-Bis[2-[[1-(chlormethyl)-1,6-dihydro-5-hydroxy-8-methylbenzo[1,2-b:4,3-b'-]dipyrrol-3(2H)-yl]-carbonyl]-1H-indol-5-yl]-1H-pyrrol-2,5-dicarboxamid (Verbindung 7);
[R-(R*,S*)]-N,N'-Bis[2-[[1-(chlormethyl)-1,6-dihydro-5-hydroxy-8-methylbenzo[1,2-b:4,3-b'-]dipyrrol-3(2H)-yl]carbonyl]-1H-indol-5-yl]-propandiamid (Verbindung 8);
S-(R*,R*)]-6,6'-[Carbonylbis(imino-1H-indol-5,2-diylcarbonyl)]bis[8-(chlormethyl)-3,6-7,8-tetrahydro-1-methyl-methyl-benzo[1,2-b:4,3-b'-]dipyrrol-4-ol-diacetat (Verbindung 9);
[S-(R*,R*)]-6,6'-(1H-Indol-2,5-diyldicarbonyl)bis[8-(chlormethyl)-3,6,7,8-tetrahydro-1-methyl-benzo[1,2-b:4,3-b'-]dipyrrol-4-ol (Verbindung 10);
[S-(R*,R*)]-N,N'-Bis[2-[[1-(clormethyl)-1,6-dihydro-5-hydroxy-8-methylbenzo[1,2-b:4,3-b'-]dipyrrol-3(2H)-yl]-carbonyl]-1H-indol-5-yl]-1H-indol-2,5-dicarboxamid (Verbindung 11);
[S-(R*,R*)]-2-[[1-(Chlormethyl)-1,6-dihydro-5-hydroxy-8-methyl-benzo[1,2-b:4,3-b'-]dipyrrol-3(2H)-yl]-carbonyl]-N-[3-[[1-(chlormethyl)-1,6-dihydro-5-hydroxy-8-methylbenzo[1,2-b:4,3-b'-]dipyrrol-3(2H)-yl]carbonyl]-1H-indol-5-yl]-1H-indol-5-carboxamid (Verbindung 12);
[S-(R*,R*)]-5-[[1-(Chlormethyl)-1,6-dihydro-5-hydroxy-8-methyl-benzo[1,2-b:4,3-b'-]dipyrrol-3(2H)-yl]carbonyl]-N-[2-[[1-(chlormethyl)-1,6-dihydro-5-hydroxy-8-methylbenzo[1,2-b:4,3-b'-]dipyrrol-3(2H)-yl]carbonyl]-1H-indol-5-yl]-1H-indol-2-carboxamid (Verbindung 13);
2,2-Dimethylpropansäure-[S-(R*,R*)]-carbonylbis[imino-1H-indol-5,2-diylcarbonyl[1-(chlormethyl)-1,6-dihydro-8-methylbenzo[1,2-b:4,3-b'-]dipyrrol-3,5(2H)-diyl]]ester (Verbindung 14);
2,2-Decansäure-[S-(R*,R*)]-carbonylbis[imino-1H-indol-5,2-diylcarbonyl[1-(chlormethyl)-1,6-dihydro-8-methylbenzo[1,2-b:4,3-b'-]dipyrrol-3,5(2H)-diyl]]ester (Verbindung 15);

11. [S-(R*,R*)]-6,6'-[Carbonylbis[(7,8-dihydrobenzo[1,2-b-4,3-b')dipyrrol-6,2(3H)-diyl)carbonyl]]bis[8-(chlormethyl)-3,6,7,8-tetrahydro-1-methyl-benzo[1,2-b:4,3b']dipyrrol-4-ol (Verbindung 16).

12. Verfahren zur Herstellung einer Verbindung nach einem der vorhergehenden Ansprüche durch Umsetzen eines geeigneten Analogen der Formel B mit einem Biscarbonsäure-, Bisisocyanat-, Bisaminocarboxylat- oder Oxycarboxylatreagens und danach mit dem anderen geeigneten Analogen der Formel B.

## Revendications

1. Composé de formule I C
PI₁ - R₅ - T - R'₅ - CPI₂
dans laquelle CPI₁ et CPI₂ sont choisis, indépendamment, entre les formules A et B 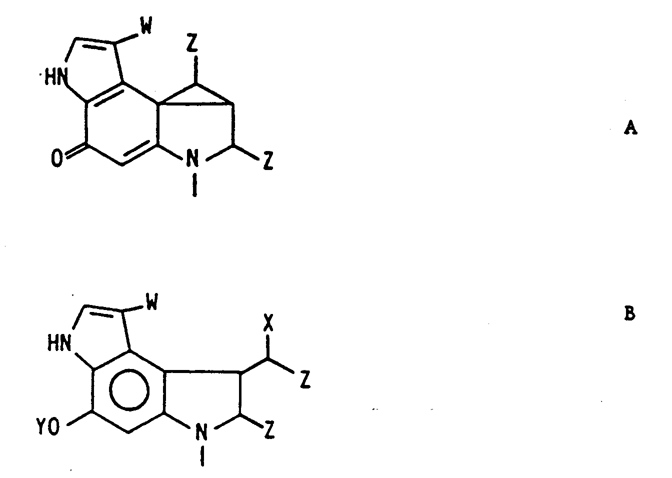
dans lesquelles W et Z sont choisis, indépendamment, entre l'hydrogène, des groupes alkyle en C₁ à C₅ et phényle ;
X représente un groupe azido, un atome d'halogène, un groupe cyanate, thiocyanate, isocyanate, thio-isocyanate, -PO(OR)₂, -O-PO₂R, -O-PSOR, -O-SOR ou -O-SO₂R ; et
Y représente l'hydrogène, un groupe -C(O)R, -C(S)R, -C(O)R₁, -S(O)₂R₁, -C(O)NR₂R₃, -C(S)NR₂R₃ ou -C(O)NHSO₂R₃ ;
dans lequel R représente un groupe alkyle en C₁ à C₂₀ ; alcényle en C₂ à C₆, alcynyle en C₂ à C₆ ; phényle facultativement substitué avec un, 2 ou 3 substituants choisis entre des substituants alkyle en C₁ à C₄, alkoxy en C₁ à C₃, halogéno, alkylthio en C₁ à C₃, CF₃, dialkylamino en C₂ à C₆ et nitro ; ou naphtyle facultativement substitué avec un ou 2 substituants répondant à la définition précitée ;
R₁ représente un groupe alkyle en C₁ à C₂₀ ou un groupe phényle facultativement substitué, répondant à la définition précitée ;
R₂ représente l'hydrogène ou un groupe alkyle en C₁ à C₂₀ ;
R₃ représente l'hydrogène, un groupe alkyle en C₁ à C₂₀ ou un groupe phényle facultativement substitué, répondant à la définition précitée ; et
R₄ représente un groupe alkyle en C₁ à C₁₀, phényle facultativement substitué répondant à la définition précitée ou naphtyle facultativement substitué répondant à la définition précitée ;
R₅ et R'₅ sont choisis, indépendamment, entre une liaison directe et un groupe divalent répondant à n'importe laquelle des formules : 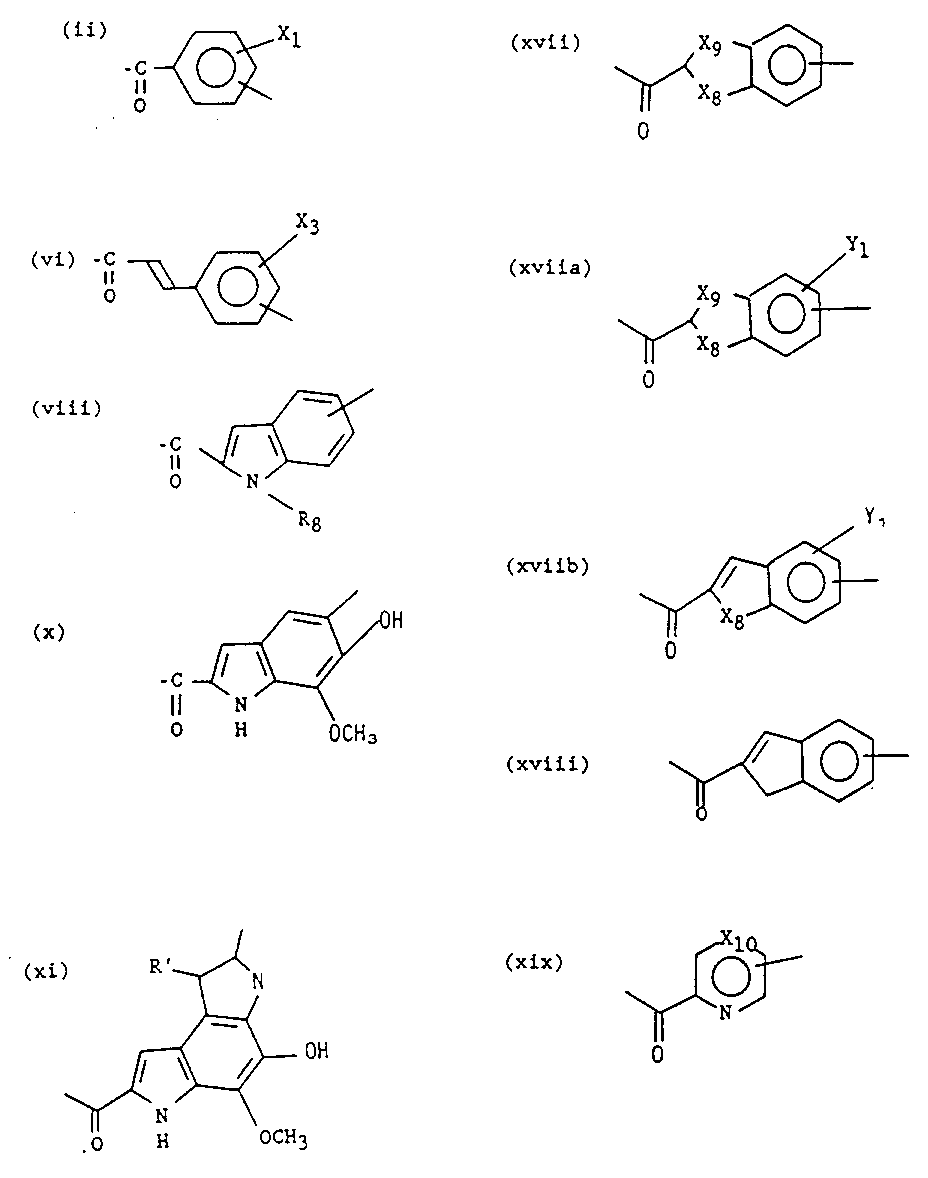 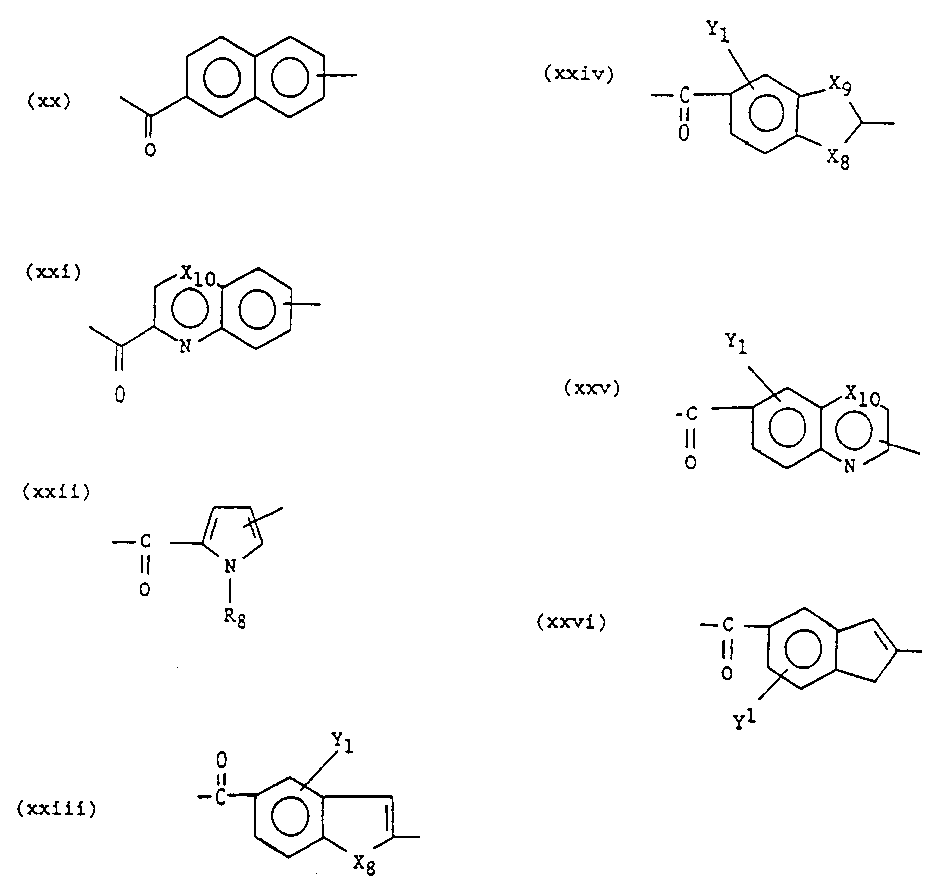
dans lesquelles X₁ représente H, un groupe CH₃, OH, OCH₃, NO₂, NH₂, NHNHC(O)CH₃, NHC(O)C₆H₅ ou halogéno ; X₃ représente H, un groupe OH ou OCH₃ ; R₈ représente H, un groupe CH₃ ou C₂H₅ ; R' réprésente H ou un groupe CH₃S- ; X₈ représente -O-, -S- ou un groupe NH ; X₉ et X₁₀ représentent chacun un groupe -CH= ou -N= ; et Y₁ représente H, un groupe halogéno, alkyle en C₁ à C₄, alkoxy en C₁ à C₃, dialkylamino en C₂ à C₆, nitro, aminocarbonyl-(alkyle en C₁ à C₁₀), hydroxy, -NH₂, -NHCONH₂, -NHCOCH₃ ou -NHC(O)-C₆H₅ ; et
T représente un groupe de liaison choisi entre :
a) -NHC(O)- ;
b) -C(O)NH- ;
c) -C(O)O- ;
d) -OC(O)- ;
e) -NR₁₃-T'-NR₁₄- dans lequel R₁₃ et R₁₄ représentent, indépendamment, l'hydrogène ou un groupe alkyle en C₁ à C₈, ou bien R₁₃ et R₁₄ sont pris conjointement et représentent un groupe -(CH₂)ₙ- dans lequel n est égal à 2 ou 3 ; et T' représente un groupe -CO-, -COCO-, -CO(CH₂)₁₋₅CO-, -C(O)PhC(O)- dans lequel Ph représente un groupe 1,3 ou 1,4-phénylène ou -C(O)-het-C(O)- ; et
f) -C(O)-het-C(O)-, dans lequel R₅ et R'₅ représentent l'un et l'autre une liaison directe ;
le terme -het- représentant un groupe hétéroaryle mono-, di- ou tricyclique condensé de 5 à 12 atomes, contenant un, 2 ou 3 hétéroatomes choisis entre O, N et S, facultativement substitué avec un ou 2 substituants répondant à la définition précitée.

2. Composé suivant la revendication 1, dans lequel W représente un groupe méthyle.

3. Composé suivant la revendication 1 ou la revendication 2, dans lequel X représente un halogène.

4. Composé suivant l'une quelconque des revendications précédentes, dans lequel Z représente l'hydrogène.

5. Composé suivant la revendication 1, dans lequel CPI₁ et CPI₂ sont identiques ou différents et représentent chacun un groupe 1-(chlorométhyl)-1,6-dihydro-8-méthyl-5-hydroxybenzo[1,2:4,3-6']dipyrrole-3-(2H)-yle ou 4,5,8,8a-tétrahydro-7-méthyl-4-oxocyclopropa[c]pyrrolo-(3,2-e)-indole-2(1H)-yle.

6. Composé suivant l'une quelconque des revendications précédentes, dans lequel T représente un groupe -NHCO-, -CONH-, -NH-CO-NH- ou -NH-CO-het-CO-NH-.

7. Composé suivant la revendication 6, dans lequel T représente un groupe -NH-CO-NH- ou -NH-CO-het-CO-NH- et -het- est choisi entre des groupes pyrrole-2,5-diyle, furanne-2,5-diyle, indole-2,5-diyle, benzofuranne-2,5-diyle et 3,6-dihydrobenzo[1,2-b:4,3-b']dipyrrole-2,7-diyle.

8. Composé suivant l'une quelconque des revendications précédentes, dans lequel R₅ et R'₅ sont choisis entre des groupes 2-carbonylindole-5-yle, 2-carbonyl-6-hydroxy-7-méthoxyindole-5-yle, 2-carbonyl-1,2,3,6-tétrahydrobenzo[1,2-b:4,3-b']dipyrrole-7-yle et 2-carbonyl-4-hydroxy-5-méthoxy-1,2,3,6-tétrahydrobenzo[1,2-b:4,3-b']dipyrrole-7-yle.

9. Composé suivant la revendication 1, dans lequel Y représente l'hydrogène ou un groupe (alkyle en C₁ à C₁₀)carbonyle.

10. Composé suivant la revendication 1, choisi entre les suivants :
[S-(R*,R*)]-6,6'-[carbonylbis(imino-1H-indole-5,2-dicarbonyl)]-bis-[8-chlorométhyl)-3,6,7,8-tétrahydro-1-méthyl-benzo[1,2-b:4,3-b']dipyrrole-4-ol (Composé 1) ;
[7bR-[2(7'bR*,8'aS*),7bR*,8aS*]]-2,2'-[carbonylbis(imino-1H-indole-5,2-dicarbonyl)]bis[1,2,8,8a-tétrahydro-7-méthyl-cyclopropa(c)pyrrolo[3,2-e]indole-4(5H)-one (Composé 2) ;
(R*,S*)-N,N'-bis[2-[[1-(chlorométhyl)-1,6-dihydro-5-hydroxy-8-méthylbenzo[1,2-b:4,3-b']dipyrrole-3(2H)-yl]-carbonyl]-1H-indole-5-yl]éthanediamide (Composé 3) ;
[S-(R*,R*)]-6,6'-(1H-pyrrole-2,5-diyldicarbonyl)bis[8-(chlorométhyl)-3,6,7,8-tétrahydro-1-méthyl-benzo[1,2-b:4,3-b']dipyrrole-4-ol (Composé 4) ;
[S-(R*,R*)]-6,6'-(2,5-furannediyldicarbonyl)bis[8-(chlorométhyl)-3,6,7,8-tétrahydro-1-méthyl-benzo[1,2-b:4,3'-b]dipyrrole-4-ol (Composé 5) ;
[S-(R*,R*)]-N,N'-bis[2-[[1-(chlorométhyl)-1,6-dihydro-5-hydroxy-8-méthylbenzo[1,2-b:4,3-b']dipyrrole-3(2H)-yl]-carbonyl]-1H-indole-5-yl]-2,5-furannedicarboxamide (Composé 6) ;
[S-(R*,R*)]-N,N'-bis[2-[[1-(chlorométhyl)-1,6-dihydro-5-hydroxy-8-méthylbenzo[1,2-b:4,3-b']dipyrrole-3(2H)-yl]-carbonyl]-1H-indol-5-yl]-1H-pyrrole-2,5-dicarboxamide (Compose 7) ;
[R-(R*,S*)]-N,N'-bis[2-[[1-(chlorométhyl)-1,6-dihydro-5-hydroxy-8-méthylbenzo[1,2-b:4,3-b']dipyrrole-3(2H)-yl]carbonyl]-1H-indole-5-yl]-propanediamide (Composé 8) ;
diacétate de [S-(R*,R*)]-6,6'-[carbonylbis(imino-1H-indole-5,2-diylcarbonyl)]bis[8-(chlorométhyl)-3,6,7,8-tétahydro-1-méthyl-benzo[1,2-b:4,3-b']dipyrrole-4-ol (Composé 9) ;
[S-(R*,R*)]-6,6'-(1H-indole-2,5-diyldicarbonyl)bis[8-(chlorométhyl)-3,6,7,8-tétahydro-1-méthyl-benzo[1,2-b:4,3-b']dipyrrole-4-ol (Composé 10) ;
[S-(R*,R*)]-N,N'-bis[2-[[1-(chlorométhyl)-1,6-dihydro-5-hydroxy-8-méthylbenzo[1,2-b:4,3-b']dipyrrole-3(2H)-yl]-carbonyl]-1H-indole-5-yl]-1H-indole-2,5-dicarboxamide (Composé 11) ;
[S-(R*,R*)]-2-[[1-(chlorométhyl)-1,6-dihydro-5-hydroxy-8-méthylbenzo[1,2-b:4,3-b']dipyrrole-3(2H)-yl]-carbonyl]-N-[3-[[1-(chlorométhyl)-1,6-dihydro-5-hydroxy-8-méthylbenzo[1,2-b:4,3-b']dipyrrole-3(2H)yl]carbonyl]-1H-indole-5-yl]-1H-indole-5-dicarboxamide (Composé 12) ;
[S-(R*,R*)]-5-[[1-(chlorométhyl)-1,6-dihydro-5-hydroxy-8-méthylbenzo[1,2-b:4,3-b']dipyrrole-3(2H)-yl]-carbonyl)-N-[2-[[1-(chlorométhyl)-1,6-dihydro-5-hydroxy-8-méthylbenzo[1,2-b:4,3-b']dipyrrole-3(2H)-yl]carbonyl)-1H- indole-5-yl]-1H-indole-2-carboxamide (Composé 13) ;
[S-(R*,R*)]-carbonylbis[imino-1H-indole-5,2-diylcarbonyl-[1-(chlorométhyl)-1,6-dihydro-8-méthylbenzo-[1,2-b:4,3-b']dipyrrole-3,5(2H)-diyl]]ester, acide 2,2-diméthylpropanoïque (Composé 14) ;
[S-(R*,R*)]-carbonylbis[imino-1H-indole-5,2-diylcarbonyl-[1-(chlorométhyl)-1,6-dihydro-8-méthylbenzo-[1,2-b:4,3-b']dipyrrole-3,5(2H)-diyl]]ester, acide décanoïque (Composé 15).

11. [S-(R*,R*)]-6,6'-carbonylbis[7,8-dihydrobenzo-[1,2-b:4,3-b']dipyrrole-6,2(3H)-diyl)carbonyl]]bis[8-(chlorométhyl)-3,6,7,8-tétrahydro-1-méthylbenzo[1,2-b:4,3b']dipyrrole-4-ol (Composé 16).

12. Procédé pour la préparation d'un composé suivant l'une quelconque des revendications précédentes, qui comprend la réaction de l'analogue approprié de formule B avec un réactif consistant en un acide bis-carboxylique, un bis-isocyanate, un bis-aminocarboxylate ou un oxycarboxylate, et ensuite avec l'autre analogue approprié de formule B.
